# EUROPEAN PATENT SPECIFICATION

(11) **EP 3 965 795 B1**
(45) Date of publication and mention of the grant of the patent: **26.11.2025**
(21) Application number: 19927905.0
(22) Date of filing: 07.05.2019
(51) Int. Cl.: A61K 38/16, A61K 9/19, A61P 31/18, A61K 9/08, A61K 47/02, A61K 47/12, A61K 47/10

(54) **STABLE ALBUVIRTIDE COMPOSITIONS**
STABILE ALBUVIRTIDZUSAMMENSETZUNGEN
COMPOSITIONS STABLES D'ALBUVIRTIDE

(43) Date of publication of application: 16.03.2022
(73) Proprietor: Frontier Biotechnologies Inc., Jiangning, Nanjing 211122 (CN)
(72) Inventor: LU, Rongjian, Nanjing 211122 (CN); MIN, Wenjie, Nanjing 211122 (CN)
(74) Representative: Finnegan Europe LLP
(86) International application number: PCT/CN2019/085892
(87) International publication number: WO 2020/223906

(56) References cited:
- EP-A1- 1 506 786
- EP-A1- 3 225 235
- CN-A- 101 810 583
- US-B2- 8 470 527
- CHONG HUIHUI ET AL: "Biophysical Property and Broad Anti-HIV Activity of Albuvirtide, a 3-Maleimimidopropionic Acid-Modified Peptide Fusion Inhibitor", PLOS ONE, vol. 7, no. 3, 5 March 2012 (2012-03-05), pages e32599, XP055913616, DOI: 10.1371/journal.pone.0032599
- ZBACNIK TEDDY J ET AL: "Role of Buffers in Protein Formulations", JOURNAL OF PHARMACEUTICAL SCIENCES, ELSEVIER INC, US, vol. 106, no. 3, 1 March 2017 (2017-03-01), pages 713 - 733, XP009194471, ISSN: 1520-6017, DOI: 10.1016/J.XPHS.2016.11.014
- KAROLINA L. ZAPADKA ET AL: "Factors affecting the physical stability (aggregation) of peptide therapeutics", INTERFACE FOCUS, vol. 7, no. 6, 20 October 2017 (2017-10-20), GB, pages 20170030, XP055598856, ISSN: 2042-8898, DOI: 10.1098/rsfs.2017.0030

## Description

### FIELD

The present invention relates to compositions comprising albuvirtide, an HIV-1 fusion inhibitor, the manufacturing process thereof and the use thereof in the prevention, treatment or prophylaxis of diseases caused by HIV infection.

### BACKGROUND

Human Immunodeficiency Virus (HIV), the virus that causes Acquired Immune Deficiency Syndrome (AIDS), has become one of the world's most serious health concern. HIV belongs to a class of viruses called retroviruses. HIV-1 envelope glycoprotein (Env) complex, which is composed of three receptor-binding gp120 subunits and three fusion protein gp41 subunits, mediates virus entry by fusing viral and cellular membranes and offers an attractive target for developing antiviral agents. During the fusion reaction, N- and C-terminal heptad repeat regions (NHR and CHR) of the gp41 ectodomain refold into a thermostable six-helix bundle structure (6-HB), representing a fusion-active conformation that can bridge the viral and cellular membranes for the merger. A number of peptides derived from the gp41 CHR (C-peptides) can specifically inhibit viral entry at low nanomolar concentration, including albuvirtide, which is a 3-maleimimidopropionic acid (MPA)-modified peptide HIV fusion inhibitor. Albuvirtide can irreversibly conjugate to serum albumin. Previous studies demonstrated its in vivo long half-life and potent anti-HIV activity.

Drug substances are usually preserved in combination with one or more other agents that serve varied and specialized functions. The properties that are commonly considered when preparing compositions of an active drug substance include ease of manufacture, ease of administration, and stability of the compositions. For example, US 8 470 527 B2 discloses pharmaceutical composition comprising maleimide modified HIV gp41 peptides, such as FB006M (albuvirtide), and the corresponding in vivo stability. However, due to the varying properties of active drug substances, compositions typically require pharmaceutical excipients that are uniquely tailored to the active drug substance in order to achieve advantageous physical and pharmaceutical properties. A need exists as to compositions of albuvirtide with improved stability and methods of manufacture of such pharmaceutical compositions. The present invention satisfies this need and provides related benefits.

### SUMMARY

Provided herein are liquid compositions comprising albuvirtide and a buffer system, wherein the composition has a buffer excipients at defined concentrations and ratios of excipients and albuvirtide with certain ratios of organic solvent and water. The compositions of albuvirtide enable the ease of manufacture, ease of administration, with improved process stability and storage stability. Also provided herein are liquid compositions comprising albuvirtide and an acidic buffer, wherein the composition has a pH between 1.0 and 3.5.

The embodiments of the present invention have been reflected in independent claims 1, 6, and 7. The preferred embodiments of the present invention have been reflected in dependent claims 2-5.

In some embodiments, the liquid compositions provided herein further comprises an organic solvent. In some embodiments, the organic solvent is acetonitrile, ethanol, methanol, isopropanol, or any combination thereof. In some embodiments, the liquid composition comprises the organic solvent and water at a ratio between 5:95 and 95:5. In some embodiments, the liquid composition comprises the organic solvent and water at a ratio between 20:80 and 45:55. In some embodiments, the liquid composition comprises the organic solvent and water at a ratio between 30:70 and 40:60. In some embodiments, the liquid composition comprises the organic solvent and water at a ratio of about 35:65.

The liquid compositions have a pH between 1.0 and 3.5. In some embodiments, the liquid compositions have a pH between 1.5 and 3.0.

The albuvirtide is present at a concentration between 5 mg/ml and 200 mg/ml in the liquid compositions provided herein. In some embodiments, the albuvirtide is present at a concentration between 10 mg/ml and 50 mg/ml. In some embodiments, the albuvirtide is present at a concentration between 15 mg/ml and 35 mg/ml. In some embodiments, the albuvirtide is present at a concentration between 15 mg/mg and 25 mg/ml.

The acidic buffer in the liquid compositions provided herein has a concentration between 5 and 200 mM in the liquid compositions provided herein. In some embodiments, the acidic buffer has a concentration between 10 and 50 mM. In some embodiments, the acidic buffer has a concentration between 15 and 35 mM. In some embodiments, the acidic buffer has a concentration of about 20 mM. In some embodiments, the acidic buffer in the liquid compositions provided herein comprises an acid selected from the group consisting of phosphoric acid, hydrochloric acid, acetic acid, citric acid, formic acid, trifluoroacetic acid (TFA), sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid; and a salt form of the acid.

In some embodiments, the salt form of the acid in the liquid compositions provided herein comprises a sodium salt of the acid. In some embodiments, the salt form of the acid comprises a potassium salt of the acid. In some embodiments, the salt form of the acid comprises an ammonium salt of the acid. In some embodiments, the salt form of the acid comprises any combination of a sodium salt , a potassium salt, and an ammonium salt of the acid.

In some embodiments, the acidic buffer in the liquid compositions provided herein comprises the acid and the salt form of the acid at a ratio between 100:0 to 5:95. In some embodiments, the acidic buffer comprises the acid and the salt form of the acid at a ratio between 80:20 to 20:80. In some embodiments, the acidic buffer comprises the acid and the salt form of the acid at a ratio of about 60:40.

In some embodiments, the acidic buffer in the liquid compositions provided herein comprises acetic acid. In some embodiments, the acidic buffer comprises hydrochloric acid. In some embodiments, the acidic buffer comprises phosphoric acid.

In some embodiments, the acidic buffer in the liquid compositions provided herein comprises phosphoric acid and a salt form of the acid. In some embodiments, the salt form of phosphoric acid is sodium phosphate. In some embodiments, the salt form of phosphoric acid is sodium dihydrogen phosphate. In some embodiments, the salt form of phosphoric acid is potassium phosphate. In some embodiments, the salt form of phosphoric acid is potassium dihydrogen phosphate. In some embodiments, the salt form of phosphoric acid is ammonium phosphate. In some embodiments, the salt form of phosphoric acid is ammonium dihydrogen phosphate. In some embodiments, the acidic buffer comprises the phosphate acid and the salt form of the phosphate acid at a ratio between 100:0 to 5:95. In some embodiments, the acidic buffer comprises the phosphate acid and the salt form of the phosphate acid at a ratio between 80:20 to 20:80. In some embodiments, acidic buffer comprises the phosphate acid and the salt form of the phosphate acid at a ratio of about 60:40.

In some embodiments, provided herein are stable lyophilized compositions of albuvirtide prepared by lyophilizing the liquid compositions disclosed herein.

In some embodiments, provided herein are stable lyophilized compositions comprising albuvirtide and an acidic buffer.

In some embodiments, the acidic buffer in the stable lyophilized compositions provided herein comprises an acid selected from the group consisting of phosphoric acid, hydrochloric acid, acetic acid, citric acid, sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid; and a salt form of the acidic acid. In some embodiments, the acid is acetic acid. In some embodiments, the acid is hydrochloric acid. In some embodiments, the acid is phosphoric acid. In some embodiments, the salt form of the acid comprises a sodium salt of the acid. In some embodiments, the salt form of the acid comprises a potassium salt of the acid. In some embodiments, the salt form of the acid comprises an ammonium salt of the acid. In some embodiments, the salt form of the acid comprises any combination of a sodium salt , a potassium salt, and an ammonium salt of the acid. In some embodiments, the acidic buffer comprises phosphoric acid and sodium dihydrogen phosphate.

In some embodiments, the purity of albuvirtide in the stable lyophilized compositions provided herein is decreased by at most 5%, at most 3%, at most 2%, at most 1%, or at most 0.5% during lyophilization. In some embodiments, less than 2% of the albuvirtide forms dimer during lyophilization. In some embodiments, the purity of albuvirtide in the stable lyophilized compositions provided herein is decreased by at most 7%, at most 5%, at most 3%, at most 2%, at most 1%, at most 0.5%, or at most 0.3% after 5-day storage at 25 °C. In some embodiments, the purity of albuvirtide in the stable lyophilized compositions provided herein is decreased by at most 7%, at most 5%, at most 3%, at most 2%, at most 1%, at most 0.5%, or at most 0.3% after 30-day storage at 6 °C. In some embodiments, the purity of albuvirtide in the stable lyophilized compositions provided herein is decreased by at most 18%, at most 12%, at most 7%, or at most 5% after 30-day storage at 25 °C. In some embodiments, the purity of albuvirtide in the stable lyophilized compositions provided herein is decreased by at most 5%, at most 3%, at most 2%, at most 1%, or at most 0.5% after 3-month storage at -20 °C.

In some embodiments, provided herein are pharmaceutical compositions of albuvirtide prepared by dissolving the stable lyophilized compositions described herein in water-for-injection and sterilizing the dissolved compositions. In some embodiments, provided herein are also lyophilized pharmaceutical compositions of albuvirtide prepared by lyophilizing the pharmaceutical compositions described herein.

In some embodiments, provided herein are also pharmaceutical formulations of albuvirtide prepared by reconstituting the lyophilized pharmaceutical compositions described herein in a pharmaceutically-acceptable carrier. In some embodiments, albuvirtide is present at a concentration between 1.0 mg/ml and 80.0 mg/ml in the pharmaceutical compositions, and at a concentration between 5.0 mg/ml and 100.0 mg/ml in the liquid compositions provided herein. In some embodiments, albuvirtide is present at a concentration between 18.0 mg/ml and 22.0 mg/ml in the pharmaceutical compositions provided herein. In some embodiments, albuvirtide is present at a concentration between 3.0 mg/ml and 320.0 mg/ml in the pharmaceutical formulations provided herein. In some embodiments, albuvirtide is present at a concentration between 3.0 mg/ml and 200.0 mg/ml in the pharmaceutical formulations provided herein. In some embodiments, albuvirtide is present at a concentration between 3.0 mg/ml and 50.0 mg/ml in the pharmaceutical formulations provided herein.

In some embodiments, provided herein are devices comprising the pharmaceutical formulations of albuvirtide.

In some embodiments, provided herein are articles of manufacture comprising the stable lyophilized compositions of albuvirtide. In some embodiments, provided herein are articles of manufacture comprising the pharmaceutical compositions of albuvirtide disclosed herein. In some embodiments, provided herein are articles of manufacture comprising the lyophilized pharmaceutical compositions of albuvirtide disclosed herein.

In some embodiments, provided herein are methods of preparing a stable lyophilized composition of albuvirtide from a first composition comprising (a) albuvirtide and (b) TFA or formic acid, comprising: (i) replacing the TFA or formic acid in the first composition with an acidic buffer to make a second composition comprising albuvirtide and the acidic buffer, wherein the acidic buffer comprises an acid selected from the group consisting of phosphoric acid, hydrochloric acid, acetic acid, citric acid, sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid, and (ii) lyophilizing the second composition to make the stable lyophilized albuvirtide composition. In some embodiments, methods provided herein further comprise adjusting the albuvirtide concentration in the second composition to be between 5 mg/ml and 200 mg/ml before lyophilization.

In some embodiments, the first composition is a chromatographic eluent.

In some embodiments, the first composition further comprises an organic solvent. In some embodiments, the organic solvent is acetonitrile, methanol, ethanol, isopropanol, or any combination thereof. In some embodiments, the organic solvent is acetonitrile.

In some embodiments, the methods provided herein comprise replacing the TFA or formic acid in the first composition by the acidic buffer using gradient elution.

In some embodiments, the second composition further comprises an organic solvent. In some embodiments, the method further comprises removing the organic solvent from the second composition before step (ii). In some embodiments, the organic solvent is removed by reduced-pressure evaporation. In some embodiments, the organic solvent is removed using rotary evaporator. In some embodiments, the second composition comprises organic solvent and water at a ratio between 5:95 and 95:5. In some embodiments, the second composition comprises organic solvent and water at a ratio between 20:80 and 45:55. In some embodiments, the second composition comprises organic solvent and water at a ratio between 30:70 and 40:60. In some embodiments, the second composition comprises organic solvent and water at a ratio of about 35:65. In some embodiments, the organic solvent is acetonitrile, methanol, ethanol, isopropanol, any combination thereof. In some embodiments, the organic solvent is acetonitrile.

The second composition has a pH between 1.0 and 3.5. In some embodiments, the second composition has a pH between 1.5 and 3.0.

The methods provided herein comprise adjusting the second composition to have a pH between 1.0 and 3.5. In some embodiments, the methods provided herein comprise adjusting the second composition to have a pH between 1.5 and 3.0.

In some embodiments, the acidic buffer used in the methods provided herein has a concentration between 5 and 200 mM. In some embodiments, the acidic buffer has a concentration between 10 and 50 mM. In some embodiments, the acidic buffer has a concentration between 15 and 35 mM. In some embodiments, the acidic buffer has a concentration of about 20 mM.

In some embodiments, the acidic buffer used in the methods provided herein further comprises a salt form of the acid. In some embodiments, the salt form of the acid comprises a sodium salt of the acid. In some embodiments, the salt form of the acid comprises a potassium salt of the acid. In some embodiments, the salt form of the acid comprises an ammonium salt of the acid. In some embodiments, the salt form of the acid comprises any combination of a sodium salt , a potassium salt, and an ammonium salt of the acid.

In some embodiments, the acidic buffer used in the methods provided herein comprises the acid and the salt form of the acid at a ratio between 100:0 to 5:95. In some embodiments, the acidic buffer comprises the acid and the salt form of the acid at a ratio between 80:20 to 20:80. In some embodiments, the acidic buffer comprises the acid and the salt form of the acid at a ratio between at a ratio of about 60:40.

In some embodiments, the acidic buffer used in the methods provided herein comprises hydrochloric acid. In some embodiments, the acidic buffer comprises acetic acid. In some embodiments, the acidic buffer comprises phosphoric acid. In some embodiments, the acidic buffer comprises sodium dihydrogen phosphate and phosphoric acid.

In some embodiments, the acidic buffer used in the methods provided herein comprises phosphoric acid and sodium dihydrogen phosphate at a ratio between 100:0 to 5:95. In some embodiments, the acidic buffer comprises phosphoric acid and sodium dihydrogen phosphate at a ratio between 80:20 to 20:80. In some embodiments, the acidic buffer comprises phosphoric acid and sodium dihydrogen phosphate at a ratio of about 60:40.

In some embodiments, the albuvirtide concentration in the second composition is adjusted to be between 5 mg/ml and 200 mg/ml in the methods disclosed herein. In some embodiments, the albuvirtide concentration in the second composition is adjusted to be between 10 mg/ml and 50 mg/ml. In some embodiments, the albuvirtide concentration in the second composition is adjusted to be between 15 mg/ml and 35 mg/ml. In some embodiments, the albuvirtide concentration in the second composition is adjusted to be about 20 mg/ml.

In some embodiments, provided herein are stable lyophilized compositions of albuvirtide prepared by the method disclosed herein.

In some embodiments, methods provided herein further comprise (a) dissolving the stable lyophilized compositions albuvirtide disclosed herein in water-for-rejection; and (b) sterilizing the dissolved compositions to make pharmaceutical compositions of albuvirtide. In some embodiments, sterilization comprises septic filtration.

In some embodiments, methods provided herein comprise lyophilizing the pharmaceutical compositions of albuvirtide disclosed herein to make lyophilized pharmaceutical compositions of albuvirtide.

In some embodiments, methods provided herein further comprise reconstituting the lyophilized pharmaceutical compositions of albuvirtide in a pharmaceutically acceptable carrier to make pharmaceutical formulations of albuvirtide. In some embodiments, the pharmaceutically acceptable carrier comprises (a) water-for-injection, (b) saline, (c) saline and sodium bicarbonate, (d) Na₂HPO₄, (e) Na₃PO₄ , or (f) saline and Na₂HPO₄; or its potassium form.

In some embodiments, provided herein are lyophilized pharmaceutical compositions, comprising a mixture of: (a) a therapeutically effective amount of albuvirtide, and (b) an effective amount of a stabilizer, which comprises phosphate group, wherein when said lyophilized pharmaceutical composition is dissolved in water to form an aqueous solution having albuvirtide at a concentration of 10.0 mg/ml, the aqueous solution has a pH between 1.0 and 3.5. In some embodiments, the aqueous solution has a pH between 1.5 and 3.0.

In some embodiments, provided herein are lyophilized pharmaceutical compositions, comprising a mixture of (a) a therapeutically effective amount of albuvirtide, and (b) an effective amount of a stabilizer, which comprises citric acid.

In some embodiments, provided herein are lyophilized pharmaceutical compositions, comprising a mixture of (a) a therapeutically effective amount of albuvirtide, and (b) an effective amount of a stabilizer, which comprises hydrochloride acid.

In some embodiments, the lyophilized pharmaceutical composition comprises said stabilizer and albuvirtide at a molar ratio between 1:10 and 200:1. In some embodiments, the lyophilized pharmaceutical composition comprises said stabilizer and albuvirtide at a molar ratio between 1:5 and 100:1. In some embodiments, the lyophilized pharmaceutical composition comprises said stabilizer and albuvirtide at a molar ratio between 1:2 and 50:1. In some embodiments, the lyophilized pharmaceutical composition comprises said stabilizer and albuvirtide at a molar ratio between 2:1 and 25:1. In some embodiments, the lyophilized pharmaceutical composition comprises said stabilizer and albuvirtide at a molar ratio between 3:1 and 10:1.

In some embodiments, the lyophilized pharmaceutical composition comprises a stabilizer that comprises phosphoric acid and/or a salt form of phosphoric acid. In some embodiments, the salt form of phosphoric acid comprises a sodium salt of phosphoric acid. In some embodiments, salt form of phosphoric acid comprises a potassium salt of phosphoric acid. In some embodiment, the salt form of phosphoric acid comprises an ammonium salt of phosphoric acid. In some embodiments, the salt form of phosphoric acid comprises a sodium salt of phosphoric acid and a potassium salt of phosphoric acid.

In some embodiments, the lyophilized pharmaceutical composition comprises a sodium salt of phosphoric acid at a molar ratio of sodium and phosphorus between 0.02:1 and 0.8:1. In some embodiments, the lyophilized pharmaceutical composition comprises a sodium salt of phosphoric acid at a molar ratio of sodium and phosphorus between 0.2:1 and 0.6:1. In some embodiments, the lyophilized pharmaceutical composition comprises a potassium salt of phosphoric acid at a molar ratio of potassium and phosphorus between 0.02:1 and 0.8:1. In some embodiments, the lyophilized pharmaceutical composition comprises a potassium salt of phosphoric acid at a molar ratio of potassium and phosphorus between 0.2:1 and 0.6:1. In some embodiments, the lyophilized pharmaceutical composition comprises an ammonium salt of phosphoric acid at a molar ratio of ammonium and phosphorus between 0.02:1 and 0.8:1. In some embodiments, the lyophilized pharmaceutical composition comprises an ammonium salt of phosphoric acid at a molar ratio of ammonium and phosphorus between 0.2:1 and 0.6:1. In some embodiments, the lyophilized pharmaceutical composition comprises a sodium salt and a potassium salt of phosphoric acid at a molar ratio of the sum of sodium and potassium with phosphorus between 0.02:1 and 0.8:1. In some embodiments, the lyophilized pharmaceutical composition comprises a sodium salt and a potassium salt of phosphoric acid at a molar ratio of the sum of sodium and potassium with phosphorus between 0.2:1 and 0.6:1.

In some embodiments, the lyophilized pharmaceutical composition comprises a stabilizer comprising sodium dihydrogen phosphate and phosphoric acid, and the ratio of phosphoric acid and sodium dihydrogen phosphate is between 80:20 and 20:80. In some embodiments, the lyophilized pharmaceutical composition comprises a stabilizer comprising sodium dihydrogen phosphate and phosphoric acid, and the ratio of phosphoric acid and sodium dihydrogen phosphate is between 60:40 and 40:60.

In some embodiments, provided herein are methods of preparing a stable lyophilized composition of albuvirtide from a liquid composition comprising (a) albuvirtide and (b) an acidic buffer, comprising: (i) adjusting the amount of albuvirtide and the acidic buffer so that the albuvirtide has a concentration between 5 mg/ml and 200 mg/ml, and the acidic buffer has a concentration between 5 mM and 200 mM; (ii) lyophilizing the liquid composition to make the stable lyophilized albuvirtide composition; wherein the acidic buffer comprises an acid selected from the group consisting of phosphoric acid, hydrochloric acid, acetic acid, citric acid, sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid.

In some embodiments, the liquid composition further comprises organic solvent selected from acetonitrile, methanol, ethanol, isopropanol, any combination thereof. In some embodiments, the liquid composition comprises organic solvent and water at a ratio between 5:95 and 95:5. In some embodiments, the liquid composition comprises organic solvent and water at a ratio between 20:80 and 45:55. In some embodiments, the liquid composition comprises organic solvent and water at a ratio between 30:70 and 40:60. In some embodiments, the liquid composition comprises organic solvent and water at a ratio of about 35:65.

In some embodiments, the amount of albuvirtide is adjusted to a concentration between 10 mg/ml and 50 mg/ml. In some embodiments, the amount of albuvirtide is adjusted to a concentration between 15 mg/ml and 35 mg/ml. In some embodiments, the amount of albuvirtide is adjusted to a concentration between 15 mg/ml and 25 mg/ml. In some embodiments, the amount of acidic buffer is adjusted to a concentration between 10 mM and 50 mM. In some embodiments, the amount of acidic buffer is adjusted to a concentration between 15 mM and 35 mM. In some embodiments, the amount of acidic buffer is adjusted to a concentration of about 20 mM.

The method further comprises adjusting the pH of the liquid composition to be between 1.0 and 3.5. In some embodiments, the method further comprises adjusting the pH of the liquid composition to be between 1.5 and 3.0.

In some embodiments, the acidic buffer further comprises a salt form of the acid. In some embodiments, the salt form of the acid comprises a sodium salt of the acid. In some embodiments, the molar ratio of sodium and phosphorus is between 0.02:1 and 0.8:1. In some embodiments, the molar ratio of sodium and phosphorus is between 0.2:1 and 0.6:1. In some embodiments, the salt form of the acid comprises a potassium salt of the acid. In some embodiments, the molar ratio of potassium and phosphorus is between 0.02:1 and 0.8:1. In some embodiments, the molar ratio of potassium and phosphorus is between 0.2:1 and 0.6:1. In some embodiments, the salt form of the acid comprises an ammonium salt of the acid. In some embodiments, the molar ratio of ammonium and phosphorus is between 0.02:1 and 0.8:1. In some embodiments, the molar ratio of ammonium and phosphorus is between 0.2:1 and 0.6:1. In some embodiments, the salt form of the acid comprises any combination of a sodium salt , a potassium salt, and an ammonium salt of the acid. In some embodiments, the molar ratio of the sum of sodium, potassium and ammonium with phosphorus is between 0.02:1 and 0.8:1. In some embodiments, the molar ratio of the sum of sodium, potassium and ammonium with phosphorus is between 0.2:1 and 0.6:1.

In some embodiments, the acid buffer comprises sodium dihydrogen phosphate and phosphoric acid, and the ratio of phosphoric acid and sodium dihydrogen phosphate is between 80:20 and 20:80. In some embodiments, the acid buffer comprises sodium dihydrogen phosphate and phosphoric acid, and the ratio of phosphoric acid and sodium dihydrogen phosphate is between 60:40 and 40:60. In some embodiments, the method further comprises (a) dissolving the stable lyophilized composition in water-for-rejection; and (b) sterilizing the dissolved composition to make a pharmaceutical composition. In some embodiments, said sterilization comprises septic filtration.

In some embodiments, the method further comprises lyophilizing the pharmaceutical composition to make a lyophilized pharmaceutical composition.

In some embodiments, the method further comprises reconstituting the lyophilized pharmaceutical composition in a pharmaceutically acceptable carrier. In some embodiments, the pharmaceutically acceptable carrier comprises (a) water-for-injection, (b) saline, or (c) saline and sodium bicarbonate, (d) Na₂HPO₄, (e) Na₃PO₄ , or (f) saline and Na₂HPO₄; or its potassium form.

Disclosed herein are methods for inhibiting viral replication of human immunodeficiency virus (HIV) in a subject, comprising administering to said subject a therapeutically effective amount of the pharmaceutical formulations disclosed herein.

Disclosed herein are methods for treating or preventing HIV infection in a subject by administering to said subject a therapeutically effective amount of the pharmaceutical formulations disclosed herein.

The subject may have been infected with HIV, may have been exposed to HIV, or may be at a high risk of being exposed to HIV. The , subject may have acquired immune deficiency syndromes (AIDS).

Disclosed herein are methods for treating or preventing AIDS in a subject by administering to the subject a therapeutically effective amount of the pharmaceutical formulations provided herein.

The reconstituted pharmaceutical formulations provided herein may be administered intravenously, intramuscularly, or subcutaneously.

Methods disclosed herein comprise administering 100-2000 mg albuvirtide. Methods disclosed herein comprise administering 320 mg albuvirtide. Methods disclosed herein comprise weekly administration, biweekly administration, monthly administration, bimonthly administration, or quarterly administration.

The methods disclosed herein comprise administering the pharmaceutical formulations in combination with one or more additional HIV treatment(s). The one or more additional HIV treatment(s) may be selected from the group consisting of: (a) abacavir (ZIAGEN); (b) enteric coated didanosine (VIDEX EC); (c) didanosine (VIDEX); (d) emtricitabine (EMTRIVA); (e) lamivudine (EPIVIR); (f) stavudine (ZERIT); (g) tenofovir alafenamide (VEMLIDY); (h) tenofovir disoproxil fumarate (VIREAD); (i) zidovudine (RETROVIR); (j) efavirenz (SUSTIVA); (k) etravirine (INTELENCE); (l) nevirapine (VIRAMUNE); (m) rilpivirine (EDURANT); (n) atazanavir (REYATAZ); (o) ATV/c (EVOTAZ); (p) darunavir (PREZISTA); (q) DRV/c (PREZCOBIX); (r) fosamprenavir (LEXIVA); (s) indinavir (CRIXIVAN); (t) lopinavir/ ritonavir (KALETRA); (u) nelfinavir (VIRACEPT); (v) ritonavir (NORVIR); (w) saquinavir (INVIRASE); (x) tipranavir (APTIVUS); (y) dolutegravir (TIVICAY); (z) raltegravir (ISENTRESS); (aa) enfuvirtide (FUZEON); (ab) maraviroc (SETZENTRY); (ac) ibalizumab (TROGARZO); and any combination thereof.

The one or more additional HIV treatment(s) may be selected from the group consisting of: (a) ABC/ZDV/3TC (TRIZIVIR); (b) ABC/3TC (EPZICOM); (c) ABC/3TC/DTG (TRIUMEQ); (d) FTC/EFV/TDF (ATRIPLA); (e) FTC/RPV/TDF (COMPLERA); (f) FTC/TAF (DESCOVY); (g) FTC/EVG/c/TAF (GENVOYA); (h) FTC/RPV/TAF (ODEFSEY); (i) FTC/EVG/c/TDF (STRIBILD); (j) FTC/TDF (TRUVADA); (k) 3TC/AZT (COMBIVIR); (1) BIC/FTC/TAF (BIKTARVY); (m) DTG/RPV (JULUCA); and any combination thereof.

The subject may be a human.

### BRIEF DESCRIPTION OF THE DRAWINGS

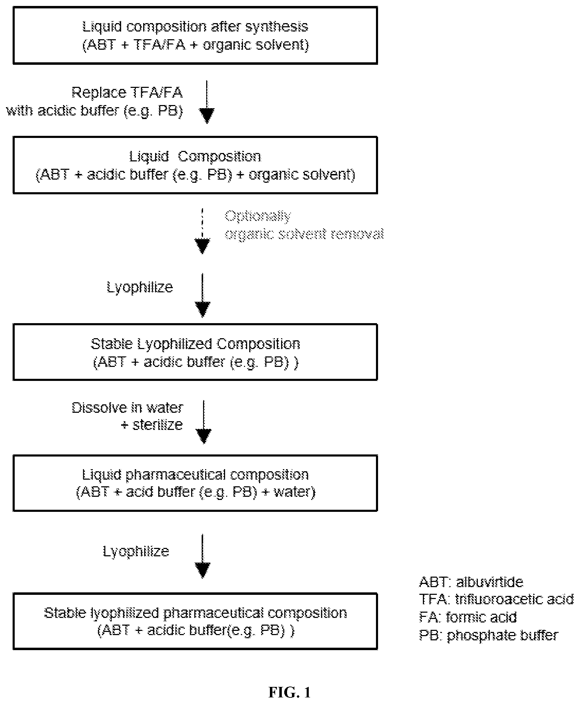

**FIG. 1****. Illustration of the manufacturing process of stable lyophilized composition having Albuvirtide and acidic buffer.**
**FIG. 2****. Stability of Albuvirtide compositions having different pH and acidic buffers:** Purity reduction of albuvirtide compositions during lyophilization varied depending on the pH of the compositions and the type of acid buffers.
**FIG. 3****. Stability of Albuvirtide-PB compositions having different pH and PB concentrations during lyophilization:** FIG. 3 shows the contour plots of mean reduction in purity of Albuvirtide-PB compositions of Table 1 having different pH and PB concentrations during lyophilization. Each data point on the slanted line (i.e. the contour line) shares the same mean reduction (%) in purity as indicated by the number on the line. PB: phosphate buffer.
**FIG. 4****. Stability of Albuvirtide-CB compositions having different pH and citrate buffer concentrations:** FIG. 4 shows the contour plots of mean reduction in purity of Albuvirtide-CB compositions having different pH and CB concentrations. Each data point on the slanted line (i.e. the contour line) shares the same mean reduction (%) in purity as indicated by the number on the line. CB: citrate buffer
**FIG. 5****. Stability of Lyophilized Albuvirtide compositions at 25 °C:** FIG. 5 shows the purity reduction of lyophilized albuvirtide compositions having different acidic buffers over a 30-day period at 25 °C.
**FIG. 6****. Stability of Lyophilized Albuvirtide-PB compositions at 25 °C:** FIG. 6 shows the purity reduction of lyophilized Albuvirtide-PB compositions having different pH and concentrations of phosphate buffers over a 30-day period at 25 °C.
**FIG. 7****. Stability of Lyophilized Albuvirtide-PB compositions:** FIG. 7 shows the contour plots of mean reduction in purity of lyophilized Albuvirtide-PB compositions of Table 8 having different concentrations of albuvirtide and PB at 25°C. Each data point on the slanted line (*i.e.* the contour line) shares the same mean reduction (%) in purity as indicated by the number on the line.

### DETAILED DESCRIPTION

It is to be understood that the disclosure is not limited to the particular embodiments set forth herein, and it is also to be understood that the terminology used herein is for the purpose of describing particular embodiments only, and is not intended to be limiting.

As used herein, the articles "a," "an," and "the" refer to one or to more than one of the grammatical object of the article. By way of example, an agent refers to one agent or two or more agents.

As used herein, the term "buffer" encompasses those agents which maintain the solution pH in an acceptable range prior to lyophilization. Typical buffers include, but are not limited to acidic buffers, basic buffers, or mixtures thereof. In some embodiments, acidic buffer can comprise a phosphoric acid, hydrochloric acid, acetic acid, citric acid, formic acid, trifluoroacetic acid (TFA), sulfuric acid, tartaric acid, lactic acid, succinic acid, ascorbic acid, aspartic acid, glutamic acid, propanoic acid, propanedioic acid, butyric acid, maleic acid, fumaric acid, malic acid, oxalic acid; and a salt thereof.

As used herein, terms "lyophilization," "lyophilized," and "freeze-dried" refer to a process by which the material to be dried is first frozen and then the ice or frozen solvent is removed by sublimation in a vacuum environment. One or more excipients can be included in pre-lyophilized compositions to enhance stability of the lyophilized product upon storage.

As used herein, the term "reconstitute," or "reconstitution" refers to the process of rehydrates a lyophilized composition with a solution.

As used herein, a "stable" composition is one in which the active ingredient therein (e.g. albuvirtide) essentially retains its physical stability and/or chemical stability and/or biological activity upon storage. Stability can be measured at a selected temperature for a selected time period. In some embodiments, the purity of albuvirtide of a stable albuvirtide composition is reduced by no more than 10% during storage. In some embodiments, the purity of albuvirtide of a stable albuvirtide composition is reduced by no more than 5% during storage. Impurities include for example, isoforms of albuvirtide, hydrolyzed form of albuvirtide and/or dimers of albuvirtide. Various analytical techniques for measuring protein stability are available in the art and are reviewed in Peptide and Protein Drug Delivery, 247-301, Vincent Lee Ed., Marcel Dekker, Inc., New York, N.Y., Pubs. (1991) and Jones, A. Adv. Drug Delivery Rev. 10:29-90 (1993). In some embodiments, the purity of albuvirtide is measured by high-performance liquid chromatography (HPLC).

As used herein, the term "pharmaceutical composition" refers to preparations which are in such form as to permit the active ingredients to be effective, and which is sterilized, contains no additional components toxic to the subjects to which the composition would be administered. "Pharmaceutically acceptable" or "physiologically acceptable" diluents, carriers or excipients (vehicles, additives) are those which can reasonably be administered to a subject mammal to provide an effective dose of the active ingredient employed. A "pharmaceutical composition" can be a liquid composition or a lyophilized composition. A "pharmaceutical composition" can also be a pharmaceutical formulation. As used herein, a "pharmaceutical formulation" refers to a liquid pharmaceutical composition that is ready for administration. A "pharmaceutical composition" can include one or more "pharmaceutically acceptable carriers," which are carriers that are nontoxic to the cell or mammal being exposed thereto at the dosages and concentrations employed. The term "carrier" refers to a diluent, excipient, or vehicle with which therapeutic is administered. Examples of suitable pharmaceutical carriers are described in Remington's Pharmaceutical Sciences (1990), Mack Publishing Co., Easton, PA.

As used herein, the term "subject" refers to a mammal. A subject can be a human or a non-human mammal such as a dog, cat, bovid, equine, mouse, rat, rabbit, or transgenic species thereof. A subject can be a human.

As used herein, the terms "treat," "treating," "treatment" and the like refer to a course of action (such as administering albuvirtide or a pharmaceutical composition comprising a albuvirtide) initiated after a disease, disorder or condition, or a symptom thereof, has been diagnosed, observed, and the like so as to eliminate, reduce, suppress, mitigate, or ameliorate, either temporarily or permanently, at least one of the underlying causes of a disease, disorder, or condition afflicting a subject, or at least one of the symptoms associated with a disease, disorder, condition afflicting a subject. Thus, treatment includes inhibiting (i.e., arresting the development or further development of the disease, disorder or condition or clinical symptoms association therewith) an active disease.

As used herein, the terms "prevent," "preventing," "prevention," and the like refer to a course of action (such as administering albuvirtide or a pharmaceutical composition comprising albuvirtide) initiated in a manner (e.g., prior to the onset of a disease, disorder, condition or symptom thereof) so as to prevent, suppress, inhibit or reduce, either temporarily or permanently, a subject's risk of developing a disease, disorder, condition or the like (as determined by, for example, the absence of clinical symptoms) or delaying the onset thereof, generally in the context of a subject predisposed to having a particular disease, disorder or condition. In certain instances, the terms also refer to slowing the progression of the disease, disorder or condition or inhibiting progression thereof to a harmful or otherwise undesired state.

As used herein, the term "administer," "administering," or "administration" refers to the act of delivering, or causing to be delivered, an active to the body of a subject by a method described herein or otherwise known in the art. Administering a compound or a pharmaceutical composition includes prescribing a compound or a pharmaceutical composition to be delivered into the body of a patient. Exemplary forms of administration include oral dosage forms, such as tablets, capsules, syrups, suspensions; injectable dosage forms, such as intravenous (IV), intramuscular (IM), or intraperitoneal (IP); transdermal dosage forms, including creams, jellies, powders, or patches; buccal dosage forms; inhalation powders, sprays, suspensions, and rectal suppositories.

As used herein, the term "therapeutically effective amount" of an active drug substance (e.g., albuvirtide) when used in connection with a disease or disorder refers to an amount sufficient to provide a therapeutic benefit in the treatment of the disease or disorder or to delay or minimize one or more symptoms associated with the disease or disorder. A therapeutically effective amount of an active drug substance (e.g., albuvirtide) means an amount of the drug substance that when used alone or in combination with other therapies, would provide a therapeutic benefit in the treatment or management of the disease or disorder. The term encompasses an amount that improves overall therapy, reduces or avoids symptoms, or enhances the therapeutic efficacy of another therapeutic agent. The term also refers to the amount of a compound that sufficiently elicits the biological or medical response of a biological molecule (e.g., a protein, enzyme, RNA, or DNA), cell, tissue, system, animal, or human, which is being sought by a researcher, veterinarian, medical doctor, or clinician.

As used herein, the term "stabilizer" refers to those substances which can be used to retain the physical stability and/or chemical stability and/or biological activity of the active ingredient therein (e.g. albuvirtide) during lyophilization or storage. Typical stabilizers include, but are not limited to osmolytic stabilizers, ionic salts, or mixtures thereof. In some embodiments, stabilizers can include ionic acids comprising phosphoric acid, hydrochloric acid, acetic acid, citric acid, formic acid, trifluoroacetic acid (TFA), sulfuric acid, tartaric acid, lactic acid, succinic acid, ascorbic acid, aspartic acid, glutamic acid, propanoic acid, propanedioic acid, butyric acid, maleic acid, fumaric acid, malic acid, oxalic acid; and/or any salt thereof.

As used herein, the term "effective amount" of a stabilizer when used in the lyophilized pharmaceutical composition with the active ingredient therein (e.g. albuvirtide) refers to an amount sufficient to provide physical stability and/or chemical stability and/or biological activity of the active ingredient during lyophilization or storage. Stability during lyophilization can be measured before and after the lyophilization process. In some embodiments, the term encompasses an amount that retain the purity of albuvirtide in the lyophilized pharmaceutical composition provided herein reduced by at most 5%, at most 3%, at most 2%, at most 1%, or at most 0.5% during lyophilization. Stability upon storage can be measured at a selected temperature for a selected time period. In some embodiments, the term encompasses an amount that retain the purity of albuvirtide in the lyophilized pharmaceutical composition provided herein reduced by no more than 10%, or no more than 5% during storage. Impurities include for example, isoforms of albuvirtide, hydrolyzed form of albuvirtide and/or dimers of albuvirtide. Various analytical techniques for measuring protein stability are available in the art and are reviewed in Peptide and Protein Drug Delivery, 247-301, Vincent Lee Ed., Marcel Dekker, Inc., New York, N.Y., Pubs. (1991) and Jones, A. Adv. Drug Delivery Rev. 10:29-90 (1993). In some embodiments, the purity of albuvirtide is measured by high-performance liquid chromatography (HPLC).

### A. Liquid Compositions

Provided herein are liquid compositions comprising albuvirtide at a concentration between 5 mg/ml and 200 mg/ml and an acidic buffer at a concentration between 5 and 200 mM, wherein the composition has a pH between 1.0 and 3.5 In some embodiments, the acidic buffer can comprise a phosphoric acid, hydrochloric acid, acetic acid, citric acid, formic acid, trifluoroacetic acid (TFA), or sulfuric acid; and a salt thereof.

In certain embodiments, the compositions comprise albuvirtide at a concentration of between 5 and 200 mg/mL, between 5 and 175 mg/mL, between 5 and 150 mg/mL, between 5 and 125 mg/mL, or between 5 and 100 mg/mL. In certain embodiments, the compositions comprise albuvirtide at a concentration of between 10 and 200 mg/mL, between 10 and 175 mg/mL, between 10 and 150 mg/mL, between 10 and 120 mg/mL, or between 10 and 100 mg/mL. In certain embodiments, the compositions comprise albuvirtide at a concentration of between 15 and 200 mg/mL. between 15 and 175 mg/mL, between 15 and 150 mg/mL, between 15 and 125 mg/mL, or between 15 and 100 mg/mL. In certain embodiments, the compositions comprise albuvirtide at a concentration of between 20 and 200 mg/mL, between 20 and 175 mg/mL, between 20 and 150 mg/mL, between 20 and 125 mg/mL, or between 20 and 100 mg/mL.

In certain embodiments, the compositions comprise albuvirtide at a concentration of between 5 and 75 mg/mL, between 10 and 75 mg/mL, between 15 and 75 mg/mL, between 20 and 75 mg/mL, between 5 and 50 mg/mL, between 10 and 50 mg/mL, between 15 and 50 mg/mL, between 20 and 50 mg/mL, between 5 and 45 mg/mL, between 10 and 45 mg/mL, between 15 and 45 mg/mL, between 20 and 45 mg/mL, between 5 and 40 mg/mL, between 10 and 40 mg/mL, between 15 and 40 mg/mL, between 20 and 40 mg/mL, between 5 and 35 mg/mL, between 10 and 35 mg/mL, between 15 and 35 mg/mL, between 20 and 35 mg/mL, between 5 and 30 mg/mL, between 10 and 30 mg/mL, between 15 and 30 mg/mL, or between 20 and 30 mg/mL.

In some embodiments, the compositions provided herein comprise albuvirtide at a concentration of between 5 and 200 mg/mL. In some embodiments, the compositions provided herein comprise albuvirtide at a concentration of between 5 and 100 mg/mL. In some embodiments, the compositions provided herein comprise albuvirtide at a concentration of between 5 and 75 mg/mL. In some embodiments, the compositions provided herein comprise albuvirtide at a concentration of between 5 and 50 mg/mL. In some embodiments, the compositions provided herein comprise albuvirtide at a concentration of between 5 and 45 mg/mL. In some embodiments, the compositions provided herein comprise albuvirtide at a concentration of between 5 and 40 mg/mL. In some embodiments, the compositions provided herein comprise albuvirtide at a concentration of between 5 and 35 mg/mL. In some embodiments, the compositions provided herein comprise albuvirtide at a concentration of between 5 and 30 mg/mL. In some embodiments, the compositions provided herein comprise albuvirtide at a concentration of between 5 and 25 mg/mL.

In some embodiments, the compositions provided herein comprise albuvirtide at a concentration of between 15 and 200 mg/mL. In some embodiments, the compositions provided herein comprise albuvirtide at a concentration of between 15 and 100 mg/mL. In some embodiments, the compositions provided herein comprise albuvirtide at a concentration of between 15 and 75 mg/mL. In some embodiments, the compositions provided herein comprise albuvirtide at a concentration of between 15 and 50 mg/mL. In some embodiments, the compositions provided herein comprise albuvirtide at a concentration of between 15 and 45 mg/mL. In some embodiments, the compositions provided herein comprise albuvirtide at a concentration of between 15 and 40 mg/mL. In some embodiments, the compositions provided herein comprise albuvirtide at a concentration of between 15 and 35 mg/mL. In some embodiments, the compositions provided herein comprise albuvirtide at a concentration of between 15 and 30 mg/mL. In some embodiments, the compositions provided herein comprise albuvirtide at a concentration of between 15 and 25 mg/mL.

In certain embodiments, the compositions provided herein comprise albuvirtide at a concentration of 10 mg/mL, 15 mg/mL, 20 mg/mL, 25 mg/mL, 30 mg/mL, 35 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, 100 mg/mL, 110 mg/mL, 120 mg/mL, 130 mg/mL, 140 mg/mL, 150 mg/mL, 160 mg/mL, 170 mg/mL, 180 mg/mL, 190 mg/mL.

In some embodiments, the compositions provided herein comprise albuvirtide at a concentration of about 10 mg/mL. In some embodiments, the compositions provided herein comprise albuvirtide at a concentration of about 15 mg/mL. In some embodiments, the compositions provided herein comprise albuvirtide at a concentration of about 20 mg/mL. In some embodiments, the compositions provided herein comprise albuvirtide at a concentration of about 25 mg/mL. In some embodiments, the compositions provided herein comprise albuvirtide at a concentration of about 30 mg/mL. In some embodiments, the compositions provided herein comprise albuvirtide at a concentration of about 35 mg/mL. In some embodiments, the compositions provided herein comprise albuvirtide at a concentration of about 40 mg/mL. In some embodiments, the compositions provided herein comprise albuvirtide at a concentration of about 45 mg/mL. In some embodiments, the compositions provided herein comprise albuvirtide at a concentration of about 50 mg/mL. In some embodiments, the compositions provided herein comprise albuvirtide at a concentration of about 75 mg/mL. In some embodiments, the compositions provided herein comprise albuvirtide at a concentration of about 100 mg/mL. In some embodiments, the compositions provided herein comprise albuvirtide at a concentration of about 125 mg/mL. In some embodiments, the compositions provided herein comprise albuvirtide at a concentration of about 150 mg/mL. In some embodiments, the compositions provided herein comprise albuvirtide at a concentration of about 175 mg/mL.

In some embodiments, the liquid compositions provided herein further comprise an organic solvent. The organic solvent can be acetonitrile, ethanol, methanol, isopropanol, or any combination thereof. In some embodiments, the organic solvent is acetonitrile. In some embodiments, the organic solvent is ethanol. In some embodiments, the organic solvent is methanol. In some embodiments, the organic solvent is isopropanol. In some embodiments, the liquid compositions provided herein comprise two organic solvents. In some embodiments, the liquid compositions provided herein comprise acetonitrile and ethanol. In some embodiments, the liquid compositions provided herein comprise acetonitrile and methanol. In some embodiments, the liquid compositions provided herein comprise acetonitrile and isopropanol. In some embodiments, the liquid compositions provided herein comprise ethanol and methanol. In some embodiments, the liquid compositions provided herein comprise ethanol and isopropanol. In some embodiments, the liquid compositions provided herein comprise methanol and isopropanol. The liquid compositions provided herein can comprise three organic solvents. In some embodiments, the liquid compositions provided herein comprise acetonitrile, ethanol, and methanol. In some embodiments, the liquid compositions provided herein comprise acetonitrile, ethanol, and isopropanol. In some embodiments, the liquid compositions provided herein comprise acetonitrile, methanol, and isopropanol. In some embodiments, the liquid compositions provided herein comprise ethanol, methanol, and isopropanol. In some embodiments, the liquid compositions provided herein comprise acetonitrile, ethanol, methanol, and isopropanol. In addition to those specifically exemplified above, the liquid compositions provided herein can also include other organic solvent known in the art, alone or in combination with those exemplified herein.

The liquid compositions provided herein can have an organic solvent and water at different ratios. In some embodiments, the liquid compositions provided herein can have an organic solvent and water at a ratio between 5:95 and 95:5 (v:v), between 10:90 and 90:10 (v:v), between 15:85 and 85:15 (v:v), between 20:80 and 80:20 (v:v), between 25:75 and 75:25 (v:v), between 30:70 and 70:30 (v:v), between 35:65 and 65:35 (v:v), between 40:60 and 60:40 (v:v), between 45:55 and 55:45 (v:v), or at about 50:50 (v:v). In some embodiments, the liquid compositions provided herein can have an organic solvent and water at a ratio between 5:95 and 95:5 (v:v). In some embodiments, the liquid compositions provided herein can have an organic solvent and water at a ratio between 10:90 and 90:10 (v:v). In some embodiments, the liquid compositions provided herein can have an organic solvent and water at a ratio between 15:85 and 85:15 (v:v). In some embodiments, the liquid compositions provided herein can have an organic solvent and water at a ratio between 20:80 and 80:20 (v:v). In some embodiments, the liquid compositions provided herein can have an organic solvent and water at a ratio between 25:75 and 75:25 (v:v). In some embodiments, the liquid compositions provided herein can have an organic solvent and water at a ratio between 30:70 and 70:30 (v:v). In some embodiments, the liquid compositions provided herein can have an organic solvent and water at a ratio between 35:65 and 65:35 (v:v). In some embodiments, the liquid compositions provided herein can have an organic solvent and water at a ratio between 40:60 and 60:40 (v:v). In some embodiments, the liquid compositions provided herein can have an organic solvent and water at a ratio between 45:55 and 55:45 (v:v). In some embodiments, the liquid compositions provided herein can have an organic solvent and water at a ratio between 20:80 and 45:55 (v:v). In some embodiments, the liquid compositions provided herein can have an organic solvent and water at a ratio between 30:70 and 40:60 (v:v). In some embodiments, the liquid compositions provided herein can have an organic solvent and water at a ratio about 50:50 (v:v). In some embodiments, the liquid compositions provided herein can have an organic solvent and water at a ratio about 35:65 (v:v).

The liquid compositions provided herein can have a pH between 1.0 and 3.5. In some embodiments, the liquid compositions provided herein can have a pH between 1.0 and 3.0. In some embodiments, the liquid compositions provided herein can have a pH between 1.0 and 2.5. In some embodiments, the liquid compositions provided herein can have a pH between 1.5 and 3.5. In some embodiments, the liquid compositions provided herein can have a pH between 1.5 and 3.0. In some embodiments, the liquid compositions provided herein can have a pH between 1.5 and 2.5 In some embodiments, the liquid compositions provided herein can have a pH between 2.0 and 3.5. In some embodiments, the liquid compositions provided herein can have a pH between 2.0 and 3.0. In some embodiments, the liquid compositions provided herein can have a pH between 2.0 and 2.5.

In some embodiments, the liquid compositions provided herein can have a pH of 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3 or 3.4. In some embodiments, the liquid compositions provided herein can have a pH of about 1.5. In some embodiments, the liquid compositions provided herein can have a pH of about 2.0. In some embodiments, the liquid compositions provided herein can have a pH of about 2.5. In some embodiments, the liquid compositions provided herein can have a pH of about 3.0.

The measurements of the pH can be taken at the temperature of 4 °C. The measurements of the pH can be taken at room temperature. The measurements of the pH can be taken at about 22 °C.

Provided herein are liquid compositions comprising albuvirtide at a concentration between 5 mg/ml and 200 mg/ml and an acidic buffer at a concentration between 5 and 200 mM, wherein the composition has a pH between 1.0 and 3.5 The acidic buffer can comprise an acid selected from the group consisting of a phosphoric acid, hydrochloric acid, acetic acid, citric acid, formic acid, trifluoroacetic acid (TFA), sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid; and any salt thereof. In some embodiments, the acidic buffer can comprise a phosphoric acid and a salt thereof. In some embodiments, the acidic buffer can comprise a hydrochloric acid and a salt thereof. In some embodiments, the acidic buffer can comprise an acetic acid and a salt thereof. In some embodiments, the acidic buffer can comprise a citric acid and a salt thereof. In some embodiments, the acidic buffer can comprise a formic acid and a salt thereof. In some embodiments, the acidic buffer can comprise a TFA and a salt thereof. In some embodiments, the acidic buffer can comprise a sulfuric acid and a salt thereof. In some embodiments, the acidic buffer can comprise a tartaric acid and a salt thereof. In some embodiments, the acidic buffer can comprise a lactic acid and a salt thereof. In some embodiments, the acidic buffer can comprise a succinic acid and a salt thereof. In some embodiments, the acidic buffer can comprise an ascorbic acid and a salt thereof. In some embodiments, the acidic buffer can comprise an aspartic acid and a salt thereof. In some embodiments, the acidic buffer can comprise a glutamic acid and a salt thereof. In some embodiments, the acidic buffer can comprise a propanoic acid and a salt thereof. In some embodiments, the acidic buffer can comprise a propanedioic acid and a salt thereof. In some embodiments, the acidic buffer can comprise a butyric acid and a salt thereof. In some embodiments, the acidic buffer can comprise a maleic acid and a salt thereof. In some embodiments, the acidic buffer can comprise a fumaric acid and a salt thereof. In some embodiments, the acidic buffer can comprise a malic acid and a salt thereof. In some embodiments, the acidic buffer can comprise an oxalic acid and a salt thereof.

In some embodiments, the salt form of the acid comprises a sodium salt of the acid. In some embodiments, the salt form of the acid comprises a potassium salt of the acid. In some embodiments, the salt form of the acid comprises an ammonium salt of the acid. In some embodiments, the salt form of the acid comprises any combination of a sodium salt , a potassium salt, and an ammonium salt of the acid.

In some embodiments, the acidic buffer can comprise a phosphoric acid and sodium phosphate. In some embodiments, the acidic buffer can comprise a hydrochloric acid and sodium chloride. In some embodiments, the acidic buffer can comprise an acetic acid and sodium acetate. In some embodiments, the acidic buffer can comprise a citric acid and sodium citrate. In some embodiments, the acidic buffer can comprise a formic acid and sodium formate. In some embodiments, the acidic buffer can comprise a TFA and sodium trifluoroacetate. In some embodiments, the acidic buffer can comprise a sulfuric acid and sodium sulfate. In some embodiments, the acidic buffer can comprise a tartaric acid and sodium tartrate. In some embodiments, the acidic buffer can comprise a lactic acid and sodium lactate. In some embodiments, the acidic buffer can comprise a succinic acid and sodium succinate. In some embodiments, the acidic buffer can comprise an ascorbic acid and sodium ascorbate. In some embodiments, the acidic buffer can comprise an aspartic acid and sodium aspartate. In some embodiments, the acidic buffer can comprise a glutamic acid and sodium glutamate. In some embodiments, the acidic buffer can comprise a propanoic acid and sodium propanoate. In some embodiments, the acidic buffer can comprise a propanedioic acid and sodium propanedioate. In some embodiments, the acidic buffer can comprise a butyric acid and sodium butyrate. In some embodiments, the acidic buffer can comprise a maleic acid and sodium maleate. In some embodiments, the acidic buffer can comprise a fumaric acid and sodium fumarate. In some embodiments, the acidic buffer can comprise a malic acid and sodium malate. In some embodiments, the acidic buffer can comprise an oxalic acid and sodium oxalate.

**In** some embodiments, the acidic buffer can comprise a phosphoric acid and potassium phosphate. **In** some embodiments, the acidic buffer can comprise a hydrochloric acid and potassium chloride. In some embodiments, the acidic buffer can comprise an acetic acid and potassium acetate. In some embodiments, the acidic buffer can comprise a citric acid and potassium citrate. In some embodiments, the acidic buffer can comprise a formic acid and potassium formate. In some embodiments, the acidic buffer can comprise a TFA and potassium trifluoroacetate. In some embodiments, the acidic buffer can comprise a sulfuric acid and potassium sulfate. In some embodiments, the acidic buffer can comprise a tartaric acid and potassium tartrate. In some embodiments, the acidic buffer can comprise a lactic acid and potassium lactate. In some embodiments, the acidic buffer can comprise a succinic acid and potassium succinate. In some embodiments, the acidic buffer can comprise an ascorbic acid and potassium ascorbate. In some embodiments, the acidic buffer can comprise an aspartic acid and potassium aspartate. In some embodiments, the acidic buffer can comprise a glutamic acid and potassium glutamate. In some embodiments, the acidic buffer can comprise a propanoic acid and potassium propanoate. In some embodiments, the acidic buffer can comprise a propanedioic acid and potassium propanedioate. In some embodiments, the acidic buffer can comprise a butyric acid and potassium butyrate. In some embodiments, the acidic buffer can comprise a maleic acid and potassium maleate. In some embodiments, the acidic buffer can comprise a fumaric acid and potassium fumarate. In some embodiments, the acidic buffer can comprise a malic acid and potassium malate. In some embodiments, the acidic buffer can comprise an oxalic acid and potassium oxalate.

In some embodiments, the acidic buffer can comprise a phosphoric acid and ammonium phosphate. In some embodiments, the acidic buffer can comprise a hydrochloric acid and ammonium chloride. In some embodiments, the acidic buffer can comprise an acetic acid and ammonium acetate. In some embodiments, the acidic buffer can comprise a citric acid and ammonium citrate. In some embodiments, the acidic buffer can comprise a formic acid and ammonium formate. In some embodiments, the acidic buffer can comprise a TFA and ammonium trifluoroacetate. In some embodiments, the acidic buffer can comprise a sulfuric acid and ammonium sulfate. In some embodiments, the acidic buffer can comprise a tartaric acid and ammonium tartrate. In some embodiments, the acidic buffer can comprise a lactic acid and ammonium lactate. In some embodiments, the acidic buffer can comprise a succinic acid and ammonium succinate. In some embodiments, the acidic buffer can comprise an ascorbic acid and ammonium ascorbate. In some embodiments, the acidic buffer can comprise an aspartic acid and ammonium aspartate. In some embodiments, the acidic buffer can comprise a glutamic acid and ammonium glutamate. In some embodiments, the acidic buffer can comprise a propanoic acid and ammonium propanoate. In some embodiments, the acidic buffer can comprise a propanedioic acid and ammonium propanedioate. In some embodiments, the acidic buffer can comprise a butyric acid and ammonium butyrate. In some embodiments, the acidic buffer can comprise a maleic acid and ammonium maleate. In some embodiments, the acidic buffer can comprise a fumaric acid and ammonium fumarate. In some embodiments, the acidic buffer can comprise a malic acid and ammonium malate. In some embodiments, the acidic buffer can comprise an oxalic acid and ammonium oxalate.

In some embodiments, the liquid compositions provided herein comprise albuvirtide and two acidic buffers, wherein the acidic buffers are selected form the group consisting of a phosphoric acid, hydrochloric acid, acetic acid, citric acid, formic acid, trifluoroacetic acid (TFA), sulfuric acid, tartaric acid, lactic acid, succinic acid, ascorbic acid, aspartic acid, glutamic acid, and salts thereof. In some embodiments, the liquid compositions provided herein comprise albuvirtide and three acidic buffers, wherein the acidic buffers are selected form the group consisting of a phosphoric acid, hydrochloric acid, acetic acid, citric acid, formic acid, trifluoroacetic acid (TFA), sulfuric acid, tartaric acid, lactic acid, succinic acid, ascorbic acid, aspartic acid, glutamic acid, propanoic acid, propanedioic acid, butyric acid, maleic acid, fumaric acid, malic acid, oxalic acid, and salts thereof. In some embodiments, the liquid compositions provided herein comprise albuvirtide and four acidic buffers, wherein the acidic buffers are selected form the group consisting of a phosphoric acid, hydrochloric acid, acetic acid, citric acid, formic acid, trifluoroacetic acid (TFA), sulfuric acid, tartaric acid, lactic acid, succinic acid, ascorbic acid, aspartic acid, glutamic acid, propanoic acid, propanedioic acid, butyric acid, maleic acid, fumaric acid, malic acid, oxalic acid, and salts thereof.

The liquid compositions provided herein comprise albuvirtide at a concentration between 5 mg/ml and 200 mg/ml and an acidic buffer at a concentration between 5 and 200 mM, wherein the composition has a pH between 1.0 and 3.5 The buffer concentration refers to the total concertation of the acid and the salt thereof. For example, if the phosphate buffer of a liquid composition consists of 5 mM phosphoric acid and 5 mM sodium phosphate, its phosphate buffer concentration is 10 mM.

In some embodiments, the liquid compositions provided herein can comprise an acidic buffer at a concentration between 5 and 175 mM. In some embodiments, the liquid compositions provided herein can comprise an acidic buffer at a concentration between 5 and 150 mM. In some embodiments, the liquid compositions provided herein can comprise an acidic buffer at a concentration between 5 and 125 mM. In some embodiments, the liquid compositions provided herein can comprise an acidic buffer at a concentration between 5 and 100 mM. In some embodiments, the liquid compositions provided herein can comprise an acidic buffer at a concentration between 10 and 200 mM. In some embodiments, the liquid compositions provided herein can comprise an acidic buffer at a concentration between 10 and 175 mM. In some embodiments, the liquid compositions provided herein can comprise an acidic buffer at a concentration between 10 and 150 mM. In some embodiments, the liquid compositions provided herein can comprise an acidic buffer at a concentration between 10 and 125 mM. In some embodiments, the liquid compositions provided herein can comprise an acidic buffer at a concentration between 10 and 100 mM.

In some embodiments, the liquid compositions provided herein can comprise an acidic buffer at a concentration between 5 and 75 mM. In some embodiments, the liquid compositions provided herein can comprise an acidic buffer at a concentration between 5 and 50 mM. In some embodiments, the liquid compositions provided herein can comprise an acidic buffer at a concentration between 5 and 45 mM. In some embodiments, the liquid compositions provided herein can comprise an acidic buffer at a concentration between 5 and 40 mM. some embodiments, the liquid compositions provided herein can comprise an acidic buffer at a concentration between 5 and 35 mM. In some embodiments, the liquid compositions provided herein can comprise an acidic buffer at a concentration between 5 and 30 mM. In some embodiments, the liquid compositions provided herein can comprise an acidic buffer at a concentration between 5 and 25 mM. In some embodiments, the liquid compositions provided herein can comprise an acidic buffer at a concentration between 5 and 20 mM.

In some embodiments, the liquid compositions provided herein can comprise an acidic buffer at a concentration between 10 and 75 mM. In some embodiments, the liquid compositions provided herein can comprise an acidic buffer at a concentration between 10 and 50 mM. In some embodiments, the liquid compositions provided herein can comprise an acidic buffer at a concentration between 10 and 45 mM. In some embodiments, the liquid compositions provided herein can comprise an acidic buffer at a concentration between 10 and 40 mM. some embodiments, the liquid compositions provided herein can comprise an acidic buffer at a concentration between 10 and 35 mM. In some embodiments, the liquid compositions provided herein can comprise an acidic buffer at a concentration between 10 and 30 mM. In some embodiments, the liquid compositions provided herein can comprise an acidic buffer at a concentration between 10 and 25 mM. In some embodiments, the liquid compositions provided herein can comprise an acidic buffer at a concentration between 10 and 20 mM.

In some embodiments, the liquid compositions provided herein can comprise an acidic buffer at a concentration between 15 and 75 mM. In some embodiments, the liquid compositions provided herein can comprise an acidic buffer at a concentration between 15 and 50 mM. In some embodiments, the liquid compositions provided herein can comprise an acidic buffer at a concentration between 15 and 45 mM. In some embodiments, the liquid compositions provided herein can comprise an acidic buffer at a concentration between 15 and 40 mM. some embodiments, the liquid compositions provided herein can comprise an acidic buffer at a concentration between 15 and 35 mM. In some embodiments, the liquid compositions provided herein can comprise an acidic buffer at a concentration between 15 and 30 mM. In some embodiments, the liquid compositions provided herein can comprise an acidic buffer at a concentration between 15 and 25 mM. In some embodiments, the liquid compositions provided herein can comprise an acidic buffer at a concentration between 15 and 20 mM.

In some embodiments, the liquid compositions provided herein can comprise an acidic buffer at a concentration of 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 100 mM, 110 mM, 120 mM, 130 mM, 140 mM, 150 mM, 160 mM, 170 mM, 180 mM, 190 mM, or 195 mM. In some embodiments, the liquid compositions provided herein can comprise an acidic buffer at a concentration of about 10 mM. In some embodiments, the liquid compositions provided herein can comprise an acidic buffer at a concentration of about 20 mM. In some embodiments, the liquid compositions provided herein can comprise an acidic buffer at a concentration of about 30 mM. In some embodiments, the liquid compositions provided herein can comprise an acidic buffer at a concentration of about 40 mM. In some embodiments, the liquid compositions provided herein can comprise an acidic buffer at a concentration of about 50 mM. In some embodiments, the liquid compositions provided herein can comprise an acidic buffer at a concentration of about 75 mM. In some embodiments, the liquid compositions provided herein can comprise an acidic buffer at a concentration of about 100 mM. In some embodiments, the liquid compositions provided herein can comprise an acidic buffer at a concentration of about 125 mM. In some embodiments, the liquid compositions provided herein can comprise an acidic buffer at a concentration of about 150 mM. In some embodiments, the liquid compositions provided herein can comprise an acidic buffer at a concentration of about 175 mM.

In some embodiments, the liquid compositions provided herein comprise an acidic buffer having the acid and the salt form of the acid at different ratios. In some embodiments, the acidic buffer can have the acid and salt form of the acid at a ratio between 100:0 and 5:95 (molar), namely, of 100 mM acidic buffer, there are 100 mM acid and 0 mM salt thereof, 5 mM acid and 95 mM salt thereof, or anything in between. In some embodiments, the acidic buffer can have the acid and salt form of the acid at a ratio between 95:5 (molar) and 5:95 (molar), between 90:10 (molar) and 10:90 (molar), between 85:15 (molar) and 15:85 (molar), between 80:20 (molar) and 20:80 (molar), between 75:25 (molar) and 25:75 (molar), between 70:30 (molar) and 30:70 (molar), between 65:35 (molar) and 35:65 (molar), between 60:40 (molar) and 40:60 (molar), between 55:45 (molar) and 45:55 (molar), or at about 50:50 (molar). In some embodiments, the liquid compositions provided herein can have acid and salt form of the acid at a ratio between 100:0 (molar) and 5:95 (molar). In some embodiments, the liquid compositions provided herein can have the acid and salt form of the acid at a ratio between 95:5 (molar) and 5:95 (molar). In some embodiments, the liquid compositions provided herein can have the acid and salt form of the acid at a ratio between 90:10 (molar) and 10:90 (molar). In some embodiments, the liquid compositions provided herein can have the acid and salt form of the acid at a ratio between 85:15 (molar) and 15:85 (molar). In some embodiments, the liquid compositions provided herein can have the acid and salt form of the acid at a ratio between 80:20 (molar) and 20:80 (molar). In some embodiments, the liquid compositions provided herein can have the acid and salt form of the acid at a ratio between 75:25 (molar) and 25:75 (molar). In some embodiments, the liquid compositions provided herein can have the acid and salt form of the acid at a ratio between 70:30 (molar) and 30:70 (molar). In some embodiments, the liquid compositions provided herein can have the acid and salt form of the acid at a ratio between 65:35 (molar) and 35:65 (molar). In some embodiments, the liquid compositions provided herein can have the acid and salt form of the acid at a ratio between 60:40 (molar) and 40:60 (molar). In some embodiments, the liquid compositions provided herein can have the acid and salt form of the acid at a ratio between 55:45 (molar) and 45:55 (molar).

In some embodiments, the liquid compositions provided herein can have the acid and salt form of the acid at a ratio of about 90:10 (molar). In some embodiments, the liquid compositions provided herein can have the acid and salt form of the acid at a ratio of about 80:20 (molar). In some embodiments, the liquid compositions provided herein can have the acid and salt form of the acid at a ratio of about 70:30 (molar). In some embodiments, the liquid compositions provided herein can have the acid and salt form of the acid at a ratio of about 60:40 (molar). In some embodiments, the liquid compositions provided herein can have the acid and salt form of the acid at a ratio of about 50:50 (molar).

Provided herein are liquid compositions comprising albuvirtide and a phosphate buffer, wherein the composition has a pH between 1.0 and 3.5, wherein the phosphate buffer comprises a phosphoric acid and a salt thereof. In some embodiments, the phosphate buffer can comprise a phosphoric acid and sodium phosphate. In some embodiments, the phosphate buffer can comprise a phosphoric acid and sodium dihydrogen phosphate. In some embodiments, the phosphate buffer can comprise a phosphoric acid and potassium phosphate. In some embodiments, the phosphate buffer can comprise a phosphoric acid and potassium dihydrogen phosphate.

In some embodiments, the liquid compositions provided herein can comprise albuvirtide and a phosphate buffer, wherein the phosphate buffer has a concentration between 5 and 200 mM. The buffer concentration refers to the total concertation of the phosphoric acid and the salt thereof. For example, if the phosphate buffer of a liquid composition consists of 5 mM phosphoric acid and 5 mM sodium dihydrogen phosphate, its phosphate buffer concentration is 10 mM.

In some embodiments, the liquid compositions provided herein can comprise a phosphate buffer at a concentration between 5 and 175 mM. In some embodiments, the liquid compositions provided herein can comprise a phosphate buffer at a concentration between 5 and 150 mM. In some embodiments, the liquid compositions provided herein can comprise a phosphate buffer at a concentration between 5 and 125 mM. In some embodiments, the liquid compositions provided herein can comprise a phosphate buffer at a concentration between 5 and 100 mM. In some embodiments, the liquid compositions provided herein can comprise a phosphate buffer at a concentration between 10 and 200 mM. In some embodiments, the liquid compositions provided herein can comprise a phosphate buffer at a concentration between 10 and 175 mM. In some embodiments, the liquid compositions provided herein can comprise a phosphate buffer at a concentration between 10 and 150 mM. In some embodiments, the liquid compositions provided herein can comprise a phosphate buffer at a concentration between 10 and 125 mM. In some embodiments, the liquid compositions provided herein can comprise a phosphate buffer at a concentration between 10 and 100 mM.

In some embodiments, the liquid compositions provided herein can comprise a phosphate buffer at a concentration between 5 and 75 mM. In some embodiments, the liquid compositions provided herein can comprise a phosphate buffer at a concentration between 5 and 50 mM. In some embodiments, the liquid compositions provided herein can comprise a phosphate buffer at a concentration between 5 and 45 mM. In some embodiments, the liquid compositions provided herein can comprise a phosphate buffer at a concentration between 5 and 40 mM. some embodiments, the liquid compositions provided herein can comprise a phosphate buffer at a concentration between 5 and 35 mM. In some embodiments, the liquid compositions provided herein can comprise a phosphate buffer at a concentration between 5 and 30 mM. In some embodiments, the liquid compositions provided herein can comprise a phosphate buffer at a concentration between 5 and 25 mM. In some embodiments, the liquid compositions provided herein can comprise a phosphate buffer at a concentration between 5 and 20 mM.

In some embodiments, the liquid compositions provided herein can comprise a phosphate buffer at a concentration between 10 and 75 mM. In some embodiments, the liquid compositions provided herein can comprise a phosphate buffer at a concentration between 10 and 50 mM. In some embodiments, the liquid compositions provided herein can comprise a phosphate buffer at a concentration between 10 and 45 mM. In some embodiments, the liquid compositions provided herein can comprise a phosphate buffer at a concentration between 10 and 40 mM. some embodiments, the liquid compositions provided herein can comprise a phosphate buffer at a concentration between 10 and 35 mM. In some embodiments, the liquid compositions provided herein can comprise a phosphate buffer at a concentration between 10 and 30 mM. In some embodiments, the liquid compositions provided herein can comprise a phosphate buffer at a concentration between 10 and 25 mM. In some embodiments, the liquid compositions provided herein can comprise a phosphate buffer at a concentration between 10 and 20 mM.

In some embodiments, the liquid compositions provided herein can comprise a phosphate buffer at a concentration between 15 and 75 mM. In some embodiments, the liquid compositions provided herein can comprise a phosphate buffer at a concentration between 15 and 50 mM. In some embodiments, the liquid compositions provided herein can comprise a phosphate buffer at a concentration between 15 and 45 mM. In some embodiments, the liquid compositions provided herein can comprise a phosphate buffer at a concentration between 15 and 40 mM. some embodiments, the liquid compositions provided herein can comprise a phosphate buffer at a concentration between 15 and 35 mM. In some embodiments, the liquid compositions provided herein can comprise a phosphate buffer at a concentration between 15 and 30 mM. In some embodiments, the liquid compositions provided herein can comprise a phosphate buffer at a concentration between 15 and 25 mM. In some embodiments, the liquid compositions provided herein can comprise a phosphate buffer at a concentration between 15 and 20 mM.

In some embodiments, the liquid compositions provided herein can comprise a phosphate buffer at a concentration of about 5 mM, 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 100 mM, 110 mM, 120 mM, 130 mM, 140 mM, 150 mM, 160 mM, 170 mM, 180 mM, 190 mM, or 195 mM. In some embodiments, the liquid compositions provided herein can comprise a phosphate buffer at a concentration of about 5 mM. In some embodiments, the liquid compositions provided herein can comprise a phosphate buffer at a concentration of about 10 mM. In some embodiments, the liquid compositions provided herein can comprise a phosphate buffer at a concentration of about 20 mM. In some embodiments, the liquid compositions provided herein can comprise a phosphate buffer at a concentration of about 30 mM. In some embodiments, the liquid compositions provided herein can comprise a phosphate buffer at a concentration of about 40 mM. In some embodiments, the liquid compositions provided herein can comprise a phosphate buffer at a concentration of about 50 mM. In some embodiments, the liquid compositions provided herein can comprise a phosphate buffer at a concentration of about 75 mM. In some embodiments, the liquid compositions provided herein can comprise a phosphate buffer at a concentration of about 100 mM. In some embodiments, the liquid compositions provided herein can comprise a phosphate buffer at a concentration of about 125 mM. In some embodiments, the liquid compositions provided herein can comprise a phosphate buffer at a concentration of about 150 mM. In some embodiments, the liquid compositions provided herein can comprise a phosphate buffer at a concentration of about 175 mM. In some embodiments, the liquid compositions provided herein can comprise a phosphate buffer at a concentration of about 200 mM.

In some embodiments, the liquid compositions provided herein comprise a phosphate buffer having the phosphoric acid and the salt form of the phosphoric acid at different ratios. In some embodiments, the salt form of the phosphoric acid is sodium phosphate. In some embodiments, the salt form of the phosphoric acid is sodium dihydrogen phosphate. In some embodiments, the salt form of the phosphoric acid is potassium phosphate. In some embodiments, the salt form of the phosphoric acid is potassium dihydrogen phosphate. In some embodiments, the salt form of the phosphoric acid is ammonium phosphate. In some embodiments, the salt form of the phosphoric acid is ammonium dihydrogen phosphate. In some embodiments, the salt form of the phosphoric acid comprises any combination of the above.

In some embodiments, the phosphoric acidic buffer can have the phosphoric acid and salt form of the phosphoric acid at a ratio between 100:0 (molar) and 5:95 (molar), between 95:5 (molar) and 5:95 (molar), between 90:10 (molar) and 10:90 (molar), between 85:15 (molar) and 15:85 (molar), between 80:20 (molar) and 20:80 (molar), between 75:25 (molar) and 25:75 (molar), between 70:30 (molar) and 30:70 (molar), between 65:35 (molar) and 35:65 (molar), between 60:40 (molar) and 40:60 (molar), between 55:45 (molar) and 45:55 (molar), or at about 50:50 (molar). In some embodiments, the liquid compositions provided herein can have the phosphoric acid and salt form of the phosphoric acid at a ratio between 100:0 (molar) and 5:95 (molar). In some embodiments, the liquid compositions provided herein can have the phosphoric acid and salt form of the phosphoric acid at a ratio between 95:5 (molar) and 5:95 (molar). In some embodiments, the liquid compositions provided herein can have the phosphoric acid and salt form of the phosphoric acid at a ratio between 90:10 (molar) and 10:90 (molar). In some embodiments, the liquid compositions provided herein can have the phosphoric acid and salt form of the phosphoric acid at a ratio between 85:15 (molar) and 15:85 (molar). In some embodiments, the liquid compositions provided herein can have the phosphoric acid and salt form of the phosphoric acid at a ratio between 80:20 (molar) and 20:80 (molar). In some embodiments, the liquid compositions provided herein can have the phosphoric acid and salt form of the phosphoric acid at a ratio between 75:25 (molar) and 25:75 (molar). In some embodiments, the liquid compositions provided herein can have the phosphoric acid and salt form of the phosphoric acid at a ratio between 70:30 (molar) and 30:70 (molar). In some embodiments, the liquid compositions provided herein can have the phosphoric acid and salt form of the phosphoric acid at a ratio between 65:35 (molar) and 35:65 (molar). In some embodiments, the liquid compositions provided herein can have the phosphoric acid and salt form of the phosphoric acid at a ratio between 60:40 (molar) and 40:60 (molar). In some embodiments, the liquid compositions provided herein can have the phosphoric acid and salt form of the phosphoric acid at a ratio between 55:45 (molar) and 45:55 (molar).

In some embodiments, the liquid compositions provided herein can have the phosphoric acid and salt form of the phosphoric acid at a ratio of about 90:10 (molar). In some embodiments, the liquid compositions provided herein can have the phosphoric acid and salt form of the phosphoric acid at a ratio of about 80:20 (molar). In some embodiments, the liquid compositions provided herein can have the phosphoric acid and salt form of the phosphoric acid at a ratio of about 70:30 (molar). In some embodiments, the liquid compositions provided herein can have the phosphoric acid and salt form of the phosphoric acid at a ratio of about 60:40 (molar). In some embodiments, the liquid compositions provided herein can have the phosphoric acid and salt form of the phosphoric acid at a ratio of about 50:50 (molar).

Provided herein are liquid compositions comprising albuvirtide and a phosphate buffer, wherein the composition has a pH between 1.0 and 3.5, wherein the phosphate buffer comprises a phosphoric acid and a salt thereof. In certain embodiments, the compositions comprise albuvirtide at a concentration of between 5 and 200 mg/mL, between 5 and 175 mg/mL, between 5 and 150 mg/mL, between 5 and 125 mg/mL, or between 5 and 100 mg/mL. In certain embodiments, the compositions comprise albuvirtide at a concentration of between 10 and 200 mg/mL, between 10 and 175 mg/mL, between 10 and 150 mg/mL, between 10 and 120 mg/mL, or between 10 and 100 mg/mL. In certain embodiments, the compositions comprise albuvirtide at a concentration of between 15 and 200 mg/mL, between 15 and 175 mg/mL, between 15 and 150 mg/mL, between 15 and 125 mg/mL, or between 15 and 100 mg/mL. In certain embodiments, the compositions comprise albuvirtide at a concentration of between 20 and 200 mg/mL, between 20 and 175 mg/mL, between 20 and 150 mg/mL, between 20 and 125 mg/mL, or between 20 and 100 mg/mL.

In certain embodiments, the compositions comprise albuvirtide at a concentration of between 5 and 75 mg/mL, between 10 and 75 mg/mL, between 15 and 75 mg/mL, between 20 and 75 mg/mL, between 5 and 50 mg/mL, between 10 and 50 mg/mL, between 15 and 50 mg/mL, between 20 and 50 mg/mL, between 5 and 45 mg/mL, between 10 and 45 mg/mL, between 15 and 45 mg/mL, between 20 and 45 mg/mL, between 5 and 40 mg/mL, between 10 and 40 mg/mL, between 15 and 40 mg/mL, between 20 and 40 mg/mL, between 5 and 35 mg/mL, between 10 and 35 mg/mL, between 15 and 35 mg/mL, between 20 and 35 mg/mL, between 5 and 30 mg/mL, between 10 and 30 mg/mL, between 15 and 30 mg/mL, or between 20 and 30 mg/mL.

The compositions provided herein comprise albuvirtide at a concentration of between 5 and 200 mg/mL. In some embodiments, the compositions provided herein comprise albuvirtide at a concentration of between 5 and 100 mg/mL. In some embodiments, the compositions provided herein comprise albuvirtide at a concentration of between 5 and 75 mg/mL. In some embodiments, the compositions provided herein comprise albuvirtide at a concentration of between 5 and 50 mg/mL. In some embodiments, the compositions provided herein comprise albuvirtide at a concentration of between 5 and 45 mg/mL. In some embodiments, the compositions provided herein comprise albuvirtide at a concentration of between 5 and 40 mg/mL. In some embodiments, the compositions provided herein comprise albuvirtide at a concentration of between 5 and 35 mg/mL. In some embodiments, the compositions provided herein comprise albuvirtide at a concentration of between 5 and 30 mg/mL. In some embodiments, the compositions provided herein comprise albuvirtide at a concentration of between 5 and 25 mg/mL.

In some embodiments, the compositions provided herein comprise albuvirtide at a concentration of between 15 and 200 mg/mL. In some embodiments, the compositions provided herein comprise albuvirtide at a concentration of between 15 and 100 mg/mL. In some embodiments, the compositions provided herein comprise albuvirtide at a concentration of between 15 and 75 mg/mL. In some embodiments, the compositions provided herein comprise albuvirtide at a concentration of between 15 and 50 mg/mL. In some embodiments, the compositions provided herein comprise albuvirtide at a concentration of between 15 and 45 mg/mL. In some embodiments, the compositions provided herein comprise albuvirtide at a concentration of between 15 and 40 mg/mL. In some embodiments, the compositions provided herein comprise albuvirtide at a concentration of between 15 and 35 mg/mL. In some embodiments, the compositions provided herein comprise albuvirtide at a concentration of between 15 and 30 mg/mL. In some embodiments, the compositions provided herein comprise albuvirtide at a concentration of between 15 and 25 mg/mL.

In certain embodiments, the compositions provided herein comprise albuvirtide at a concentration of 10 mg/mL, 15 mg/mL, 20 mg/mL, 25 mg/mL, 30 mg/mL, 35 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, 100 mg/mL, 110 mg/mL, 120 mg/mL, 130 mg/mL, 140 mg/mL, 150 mg/mL, 160 mg/mL, 170 mg/mL, 180 mg/mL, 190 mg/mL.

In some embodiments, the compositions provided herein comprise albuvirtide at a concentration of about 10 mg/mL. In some embodiments, the compositions provided herein comprise albuvirtide at a concentration of about 15 mg/mL. In some embodiments, the compositions provided herein comprise albuvirtide at a concentration of about 20 mg/mL. In some embodiments, the compositions provided herein comprise albuvirtide at a concentration of about 25 mg/mL. In some embodiments, the compositions provided herein comprise albuvirtide at a concentration of about 30 mg/mL. In some embodiments, the compositions provided herein comprise albuvirtide at a concentration of about 35 mg/mL. In some embodiments, the compositions provided herein comprise albuvirtide at a concentration of about 40 mg/mL. In some embodiments, the compositions provided herein comprise albuvirtide at a concentration of about 45 mg/mL. In some embodiments, the compositions provided herein comprise albuvirtide at a concentration of about 50 mg/mL. In some embodiments, the compositions provided herein comprise albuvirtide at a concentration of about 75 mg/mL. In some embodiments, the compositions provided herein comprise albuvirtide at a concentration of about 100 mg/mL. In some embodiments, the compositions provided herein comprise albuvirtide at a concentration of about 125 mg/mL. In some embodiments, the compositions provided herein comprise albuvirtide at a concentration of about 150 mg/mL. In some embodiments, the compositions provided herein comprise albuvirtide at a concentration of about 175 mg/mL.

In some embodiments, provided herein are liquid compositions comprising albuvirtide and a phosphate buffer, wherein the composition has a pH between 1.0 and 3.5, wherein the phosphate buffer comprises a phosphoric acid and a salt thereof. In some embodiments, the liquid compositions provided herein further comprise an organic solvent. The organic solvent can be acetonitrile, ethanol, methanol, isopropanol, or any combination thereof. In some embodiments, the organic solvent is acetonitrile. In some embodiments, the organic solvent is ethanol. In some embodiments, the organic solvent is methanol. In some embodiments, the organic solvent is isopropanol. In some embodiments, the liquid compositions provided herein comprise two organic solvents. In some embodiments, the liquid compositions provided herein comprise acetonitrile and ethanol. In some embodiments, the liquid compositions provided herein comprise acetonitrile and methanol. In some embodiments, the liquid compositions provided herein comprise acetonitrile and isopropanol. In some embodiments, the liquid compositions provided herein comprise ethanol and methanol. In some embodiments, the liquid compositions provided herein comprise ethanol and isopropanol. In some embodiments, the liquid compositions provided herein comprise methanol and isopropanol. The liquid compositions provided herein can comprise three organic solvents. In some embodiments, the liquid compositions provided herein comprise acetonitrile, ethanol, and methanol. In some embodiments, the liquid compositions provided herein comprise acetonitrile, ethanol, and isopropanol. In some embodiments, the liquid compositions provided herein comprise acetonitrile, methanol, and isopropanol. In some embodiments, the liquid compositions provided herein comprise ethanol, methanol, and isopropanol. In some embodiments, the liquid compositions provided herein comprise acetonitrile, ethanol, methanol, and isopropanol. In addition to those specifically exemplified above, the liquid compositions provided herein can also include other organic solvent known in the art, alone or in combination with those exemplified herein.

The liquid compositions provided herein can have an organic solvent and water at different ratios. In some embodiments, the liquid compositions provided herein can have an organic solvent and water at a ratio between 5:95 (v:v) and 95:5 (v:v), between 10:90 (v:v) and 90:10 (v:v), between 15:85 (v:v) and 85:15 (v:v), between 20:80 (v:v) and 80:20 (v:v), between 20:80 (v:v) and 45:55 (v:v), between 25:75 (v:v) and 75:25 (v:v), between 30:70 (v:v) and 70:30 (v:v), between 30:70 (v:v) and 40:60 (v:v), between 35:65 (v:v) and 65:35 (v:v), between 40:60 (v:v) and 60:40 (v:v), between 45:55 (v:v) and 55:45 (v:v), at about 35:65, or at about 50:50 (v:v). In some embodiments, the liquid compositions provided herein can have an organic solvent and water at a ratio between 5:95 (v:v) and 95:5 (v:v). In some embodiments, the liquid compositions provided herein can have an organic solvent and water at a ratio between 10:90 (v:v) and 90:10 (v:v). In some embodiments, the liquid compositions provided herein can have an organic solvent and water at a ratio between 15:85 (v:v) and 85:15 (v:v). In some embodiments, the liquid compositions provided herein can have an organic solvent and water at a ratio between 20:80 (v:v) and 80:20 (v:v). In some embodiments, the liquid compositions provided herein can have an organic solvent and water at a ratio between 20:80 (v:v) and 45:55 (v:v). In some embodiments, the liquid compositions provided herein can have an organic solvent and water at a ratio between 25:75 (v:v) and 75:25 (v:v). In some embodiments, the liquid compositions provided herein can have an organic solvent and water at a ratio between 30:70 (v:v) and 70:30 (v:v). In some embodiments, the liquid compositions provided herein can have an organic solvent and water at a ratio between 30:70 (v:v) and 40:60 (v:v). In some embodiments, the liquid compositions provided herein can have an organic solvent and water at a ratio between 35:65 (v:v) and 65:35 (v:v). In some embodiments, the liquid compositions provided herein can have an organic solvent and water at a ratio between 40:60 (v:v) and 60:40 (v:v). In some embodiments, the liquid compositions provided herein can have an organic solvent and water at a ratio between 45:55 (v:v) and 55:45 (v:v). In some embodiments, the liquid compositions provided herein can have an organic solvent and water at a ratio about 35:65 (v:v). In some embodiments, the liquid compositions provided herein can have an organic solvent and water at a ratio about 50:50 (v:v).

In some embodiments, provided herein are liquid compositions comprising albuvirtide and a phosphate buffer, wherein the composition has a pH between 1.0 and 3.5, wherein the phosphate buffer comprises a phosphoric acid and a salt thereof. In some embodiments, the liquid compositions provided herein can have a pH between 1.0 and 3.0. In some embodiments, the liquid compositions provided herein can have a pH between 1.0 and 2.5. In some embodiments, the liquid compositions provided herein can have a pH between 1.5 and 3.5. In some embodiments, the liquid compositions provided herein can have a pH between 1.5 and 3.0. In some embodiments, the liquid compositions provided herein can have a pH between 1.5 and 2.5. In some embodiments, the liquid compositions provided herein can have a pH between 2.0 and 3.5. In some embodiments, the liquid compositions provided herein can have a pH between 2.0 and 3.0. In some embodiments, the liquid compositions provided herein can have a pH between 2.0 and 2.5.

In some embodiments, the liquid compositions provided herein can have a pH of 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4. In some embodiments, the liquid compositions provided herein can have a pH of about 1.5. In some embodiments, the liquid compositions provided herein can have a pH of about 2.0. In some embodiments, the liquid compositions provided herein can have a pH of about 2.5. In some embodiments, the liquid compositions provided herein can have a pH of about 3.0.

The measurements of the pH can be taken at the temperature of 4 °C. The measurements of the pH can be taken at room temperature. The measurements of the pH can be taken at about 22 °C.

In some embodiments, provided herein are liquid compositions comprising albuvirtide at a concentration between 15 mg/ml and 35 mg/ml, a phosphate buffer at a concentration between 15 mM and 35 mM, and acetonitrile, wherein the phosphate buffer comprises a phosphoric acid and sodium dihydrogen phosphate at a ratio between 80:20 to 20:80, wherein the composition has a pH between 1.0 and 3.5.

In some embodiments, provided herein are liquid compositions comprising albuvirtide at about 20 mg/ml, a phosphate buffer at about 20 mM, and acetonitrile, wherein the phosphate buffer comprises a phosphoric acid and sodium dihydrogen phosphate at a ratio of about 60:40, wherein the composition has a pH between 1.5 and 3.0.

Different combinations and permutations of organic solvents, acids, and salt forms thereof, in addition to those expressly exemplified herein, are also contemplated herein.

In some embodiments, the liquid compositions provided herein can further include additional excipients, including pharmaceutically acceptable or physiologically acceptable diluents, carriers or excipients include, but are not limited to, antioxidants (e.g., ascorbic acid and sodium bisulfate), preservatives (e.g., benzyl alcohol, methyl parabens, ethyl or n-propyl, p-hydroxybenzoate), emulsifying agents, suspending agents, dispersing agents, solvents, fillers, bulking agents, vehicles, diluents, and/or adjuvants. For example, a suitable vehicle may be physiological saline solution or citrate buffered saline, possibly supplemented with other materials common in pharmaceutical compositions for parenteral administration.

### B. Lyophilized Compositions

The liquid compositions provided herein can by lyophilized to minimize the formation of aggregates and particulates and ensure that albuvirtide maintains its purity over time. Lyophilization is a freeze drying process that is often used in the preparation of pharmaceutical products to preserve their biological activity. The liquid composition is prepared, then lyophilized to form a dry cake-like product. The process generally involves drying a previously frozen sample in a vacuum to remove the ice, leaving the non-water components intact, in the form of a powdery or cake-like substance. The lyophilized product can be stored for prolonged periods of time, and at elevated temperatures, without loss of biological activity, and can be readily dissolved into a solution by the addition of an appropriate diluent. An appropriate diluent can be any liquid which is biologically acceptable and in which the lyophilized powder is completely soluble. Water-for-injection, which is sterile, pyrogen-free water, is a preferred diluent, since it does not include salts or other compounds which may affect the stability of the active drug substance. The advantage of lyophilization is that the water content is reduced to a level that greatly reduce the various molecular events which lead to instability of the product upon long-term storage. The lyophilized product is also more readily able to withstand the physical stresses of shipping.

The pre-lyophilized liquid compositions described herein can be lyophilized using appropriate freezing and drying parameters. For example, parameters can include a pre-freeze to holding at about 10 °C to about -10 °C for about 10-30 minutes. Freezing parameters can include freezing for -50 °C to -70 °C over a period of about 45 minutes to about 75 minutes. Parameters for the additional freeze step can include freezing at -40 °C to about -60 °C. Drying parameters can include a primary drying phase temperature of about -10 °C to -30 °C and pressure between about 40 mTorr to about 120 mTorr; and a secondary drying phase at about 10 °C to about 25 °C, using pressure between about 40 mTorr to 120 mTorr. In some embodiments, a total cycle time can be about 60 to 100 hours. In some embodiments, a cycle can include a pre-freeze step, a freeze step, a primary drying step, and secondary drying step. Considerations for a lyophilization cycle include freeze temperature, pressure, primary drying, secondary drying, and cycle time. The processes of lyophilization are well known in the art, and conditions can be optimized by persons of ordinary skill in the art.

In some embodiments, the purity of albuvirtide is decreased by at most 8%, at most 7%, at most 6%, at most 5%, at most 4%, at most 3%, at most 2%, at most 1%, or at most 0.5% during lyophilization. In some embodiments, the purity of albuvirtide is decreased by at most 8% during lyophilization. In some embodiments, the purity of albuvirtide is decreased by at most 7% during lyophilization. In some embodiments, the purity of albuvirtide is decreased by at most 6% during lyophilization. In some embodiments, the purity of albuvirtide is decreased by at most 5% during lyophilization. In some embodiments, the purity of albuvirtide is decreased by at most 4% during lyophilization. In some embodiments, the purity of albuvirtide is decreased by at most 3% during lyophilization. In some embodiments, the purity of albuvirtide is decreased by at most 2% during lyophilization. In some embodiments, the purity of albuvirtide is decreased by at most 1% during lyophilization. In some embodiments, the purity of albuvirtide is decreased by at most 0.5% during lyophilization.

In some embodiments, less than 5%, less than 4%, less than 3%, less than 2%, less than 1%, or less than 0.5% of the albuvirtide forms dimer during lyophilization. In some embodiments, less than 5% of the albuvirtide forms dimer during lyophilization. In some embodiments, less than 4% of the albuvirtide forms dimer during lyophilization. In some embodiments, less than 3% of the albuvirtide forms dimer during lyophilization. In some embodiments, less than 2% of the albuvirtide forms dimer during lyophilization. In some embodiments, less than 1% of the albuvirtide forms dimer during lyophilization. In some embodiments, less than 0.5% of the albuvirtide forms dimer during lyophilization.

In some embodiments, less than 5%, less than 4%, less than 3%, less than 2%, less than 1%, or less than 0.5% of the albuvirtide forms isomer during lyophilization. In some embodiments, less than 5% of the albuvirtide forms isomer during lyophilization. In some embodiments, less than 4% of the albuvirtide forms isomer during lyophilization. In some embodiments, less than 3% of the albuvirtide forms isomer during lyophilization. In some embodiments, less than 2% of the albuvirtide forms isomer during lyophilization. In some embodiments, less than 1% of the albuvirtide forms isomer during lyophilization. In some embodiments, less than 0.5% of the albuvirtide forms isomer during lyophilization.

Provided herein are stable lyophilized compositions of albuvirtide prepared by lyophilizing the liquid composition described above. In some embodiments, provided herein are stable lyophilized composition comprising albuvirtide and an acidic buffer. The acidic buffer can comprise an acid selected from the group consisting of a phosphoric acid, hydrochloric acid, acetic acid, citric acid, formic acid, trifluoroacetic acid (TFA), sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid; and a salt thereof.

Provided herein are lyophilized compositions comprising albuvirtide and an acidic buffer, which comprises an acid selected from the group consisting of a phosphoric acid, hydrochloric acid, acetic acid, citric acid, formic acid, trifluoroacetic acid (TFA), sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid; and a salt thereof. In some embodiments, the acidic buffer can comprise a phosphoric acid and a salt thereof. In some embodiments, the acidic buffer can comprise a hydrochloric acid and a salt thereof. In some embodiments, the acidic buffer can comprise an acetic acid and a salt thereof. In some embodiments, the acidic buffer can comprise a citric acid and a salt thereof. In some embodiments, the acidic buffer can comprise a formic acid and a salt thereof. In some embodiments, the acidic buffer can comprise a TFA and a salt thereof. In some embodiments, the acidic buffer can comprise a sulfuric acid and a salt thereof. In some embodiments, the acidic buffer can comprise a tartaric acid and a salt thereof. In some embodiments, the acidic buffer can comprise a lactic acid and a salt thereof. In some embodiments, the acidic buffer can comprise a succinic acid and a salt thereof. In some embodiments, the acidic buffer can comprise an ascorbic acid and a salt thereof. In some embodiments, the acidic buffer can comprise an aspartic acid and a salt thereof. In some embodiments, the acidic buffer can comprise a glutamic acid and a salt thereof. In some embodiments, the acidic buffer can comprise a propanoic acid and a salt thereof. In some embodiments, the acidic buffer can comprise a propanedioic acid and a salt thereof. In some embodiments, the acidic buffer can comprise a butyric acid and a salt thereof. In some embodiments, the acidic buffer can comprise a maleic acid and a salt thereof. In some embodiments, the acidic buffer can comprise a fumaric acid and a salt thereof. In some embodiments, the acidic buffer can comprise a malic acid and a salt thereof. In some embodiments, the acidic buffer can comprise an oxalic acid and a salt thereof.

In some embodiments, the salt form of the acid comprises a sodium salt of the acid. In some embodiments, the salt form of the acid comprises a potassium salt of the acid. In some embodiments, the salt form of the acid comprises an ammonium salt of the acid. In some embodiments, the salt form of the acid comprises any combination of a sodium salt , a potassium salt, and an ammonium salt of the acid.

In some embodiments, the acidic buffer can comprise a phosphoric acid and sodium phosphate. In some embodiments, the acidic buffer can comprise a hydrochloric acid and sodium chloride. In some embodiments, the acidic buffer can comprise an acetic acid and sodium acetate. In some embodiments, the acidic buffer can comprise a citric acid and sodium citrate. In some embodiments, the acidic buffer can comprise a formic acid and sodium formate. In some embodiments, the acidic buffer can comprise a TFA and sodium trifluoroacetate. In some embodiments, the acidic buffer can comprise a sulfuric acid and sodium sulfate. In some embodiments, the acidic buffer can comprise a tartaric acid and sodium tartrate. In some embodiments, the acidic buffer can comprise a lactic acid and sodium lactate. In some embodiments, the acidic buffer can comprise a succinic acid and sodium succinate. In some embodiments, the acidic buffer can comprise an ascorbic acid and sodium ascorbate. In some embodiments, the acidic buffer can comprise an aspartic acid and sodium aspartate. In some embodiments, the acidic buffer can comprise a glutamic acid and sodium glutamate. In some embodiments, the acidic buffer can comprise a propanoic acid and sodium propanoate. In some embodiments, the acidic buffer can comprise a propanedioic acid and sodium propanedioate. In some embodiments, the acidic buffer can comprise a butyric acid and sodium butyrate. In some embodiments, the acidic buffer can comprise a maleic acid and sodium maleate. In some embodiments, the acidic buffer can comprise a fumaric acid and sodium fumarate. In some embodiments, the acidic buffer can comprise a malic acid and sodium malate. In some embodiments, the acidic buffer can comprise an oxalic acid and sodium oxalate.

In some embodiments, the acidic buffer can comprise a phosphoric acid and potassium phosphate. In some embodiments, the acidic buffer can comprise a hydrochloric acid and potassium chloride. In some embodiments, the acidic buffer can comprise an acetic acid and potassium acetate. In some embodiments, the acidic buffer can comprise a citric acid and potassium citrate. In some embodiments, the acidic buffer can comprise a formic acid and potassium formate. In some embodiments, the acidic buffer can comprise a TFA and potassium trifluoroacetate. In some embodiments, the acidic buffer can comprise a sulfuric acid and potassium sulfate. In some embodiments, the acidic buffer can comprise a tartaric acid and potassium tartrate. In some embodiments, the acidic buffer can comprise a lactic acid and potassium lactate. In some embodiments, the acidic buffer can comprise a succinic acid and potassium succinate. In some embodiments, the acidic buffer can comprise an ascorbic acid and potassium ascorbate. In some embodiments, the acidic buffer can comprise an aspartic acid and potassium aspartate. In some embodiments, the acidic buffer can comprise a glutamic acid and potassium glutamate. In some embodiments, the acidic buffer can comprise a propanoic acid and potassium propanoate. In some embodiments, the acidic buffer can comprise a propanedioic acid and potassium propanedioate. In some embodiments, the acidic buffer can comprise a butyric acid and potassium butyrate. In some embodiments, the acidic buffer can comprise a maleic acid and potassium maleate. In some embodiments, the acidic buffer can comprise a fumaric acid and potassium fumarate. In some embodiments, the acidic buffer can comprise a malic acid and potassium malate. In some embodiments, the acidic buffer can comprise an oxalic acid and potassium oxalate.

In some embodiments, the acidic buffer can comprise a phosphoric acid and ammonium phosphate. In some embodiments, the acidic buffer can comprise a hydrochloric acid and ammonium chloride. In some embodiments, the acidic buffer can comprise an acetic acid and ammonium acetate. In some embodiments, the acidic buffer can comprise a citric acid and ammonium citrate. In some embodiments, the acidic buffer can comprise a formic acid and ammonium formate. In some embodiments, the acidic buffer can comprise a TFA and ammonium trifluoroacetate. In some embodiments, the acidic buffer can comprise a sulfuric acid and ammonium sulfate. In some embodiments, the acidic buffer can comprise a tartaric acid and ammonium tartrate. In some embodiments, the acidic buffer can comprise a lactic acid and ammonium lactate. In some embodiments, the acidic buffer can comprise a succinic acid and ammonium succinate. In some embodiments, the acidic buffer can comprise an ascorbic acid and ammonium ascorbate. In some embodiments, the acidic buffer can comprise an aspartic acid and ammonium aspartate. In some embodiments, the acidic buffer can comprise a glutamic acid and ammonium glutamate. In some embodiments, the acidic buffer can comprise a propanoic acid and ammonium propanoate. In some embodiments, the acidic buffer can comprise a propanedioic acid and ammonium propanedioate. In some embodiments, the acidic buffer can comprise a butyric acid and ammonium butyrate. In some embodiments, the acidic buffer can comprise a maleic acid and ammonium maleate. In some embodiments, the acidic buffer can comprise a fumaric acid and ammonium fumarate. In some embodiments, the acidic buffer can comprise a malic acid and ammonium malate. In some embodiments, the acidic buffer can comprise an oxalic acid and ammonium oxalate.

In some embodiments, the lyophilized compositions provided herein comprise albuvirtide and two acidic buffers, wherein the acidic buffers are selected form the group consisting of a phosphoric acid, hydrochloric acid, acetic acid, citric acid, formic acid, trifluoroacetic acid (TFA), sulfuric acid, tartaric acid, lactic acid, succinic acid, ascorbic acid, aspartic acid, glutamic acid, and salts thereof. In some embodiments, the lyophilized compositions provided herein comprise albuvirtide and three acidic buffers, wherein the acidic buffers are selected form the group consisting of a phosphoric acid, hydrochloric acid, acetic acid, citric acid, formic acid, trifluoroacetic acid (TFA), sulfuric acid, tartaric acid, lactic acid, succinic acid, ascorbic acid, aspartic acid, glutamic acid, and salts thereof. In some embodiments, the lyophilized compositions provided herein comprise albuvirtide and four acidic buffers, wherein the acidic buffers are selected form the group consisting of a phosphoric acid, hydrochloric acid, acetic acid, citric acid, formic acid, trifluoroacetic acid (TFA), sulfuric acid, tartaric acid, lactic acid, succinic acid, ascorbic acid, aspartic acid, glutamic acid, propanoic acid, propanedioic acid, butyric acid, maleic acid, fumaric acid, malic acid, oxalic acid, and salts thereof.

In some embodiments, the stable lyophilized compositions provided herein comprise albuvirtide, phosphoric acid, and a salt thereof. In some embodiments, the salt form of the phosphoric acid is sodium phosphate. In some embodiments, the salt form of the phosphoric acid is sodium dihydrogen phosphate. In some embodiments, the salt form of the phosphoric acid is potassium phosphate. In some embodiments, the salt form of the phosphoric acid is potassium dihydrogen phosphate. In some embodiments, the salt form of the phosphoric acid is ammonium phosphate. In some embodiments, the salt form of the phosphoric acid is ammonium dihydrogen phosphate. In some embodiments, the salt form of the phosphoric acid comprises any combination of the salts above. In some embodiments, the lyophilized compositions provided herein further comprise an organic solvent. The organic solvent can be acetonitrile, ethanol, methanol, isopropanol, or any combination thereof.

In some embodiments, the lyophilized compositions provided herein comprise albuvirtide, phosphoric acid and sodium dihydrogen phosphate. In some embodiments, the lyophilized compositions provided herein comprise albuvirtide, phosphoric acid, sodium dihydrogen phosphate, and acetonitrile.

A bulking agent that provides good lyophilized cake properties, such as serine, glycine, and mannitol, can be optionally added to the present liquid compositions. These agents also contribute to the tonicity of the compositions and may provide protection to the freeze-thaw process and improve long-term stability. In addition, tonicity modifiers can be added to the composition to control osmotic pressure. The composition can further comprise one or more preservatives.

This lyophilized product retains the stability of the albuvirtide, and prevents the albuvirtide intended for administration to human subjects from physical and chemical degradation in the final product.

In some embodiments, the purity of albuvirtide in the stable lyophilized compositions provided herein is decreased by at most 10%, at most 9%, at most 8%, at most 7%, at most 6%, at most 5%, at most 4%, at most 3%, at most 2%, at most 1%, at most 0.5%, or at most 0.3% after 5-day storage at room temperature. In some embodiments, the purity of albuvirtide in the stable lyophilized compositions provided herein is decreased by at most 10% after 5-day storage at room temperature. In some embodiments, the purity of albuvirtide in the stable lyophilized compositions provided herein is decreased by at most 9% after 5-day storage at room temperature. In some embodiments, the purity of albuvirtide in the stable lyophilized compositions provided herein is decreased by at most 8% after 5-day storage at room temperature. In some embodiments, the purity of albuvirtide in the stable lyophilized compositions provided herein is decreased by at most 7% after 5-day storage at room temperature. In some embodiments, the purity of albuvirtide in the stable lyophilized compositions provided herein is decreased by at most 6% after 5-day storage at room temperature. In some embodiments, the purity of albuvirtide in the stable lyophilized compositions provided herein is decreased by at most 5% after 5-day storage at room temperature. In some embodiments, the purity of albuvirtide in the stable lyophilized compositions provided herein is decreased by at most 4% after 5-day storage at room temperature. In some embodiments, the purity of albuvirtide in the stable lyophilized compositions provided herein is decreased by at most 3% after 5-day storage at room temperature. In some embodiments, the purity of albuvirtide in the stable lyophilized compositions provided herein is decreased by at most 2% after 5-day storage at room temperature. In some embodiments, the purity of albuvirtide in the stable lyophilized compositions provided herein is decreased by at most 1% after 5-day storage at room temperature. In some embodiments, the purity of albuvirtide in the stable lyophilized compositions provided herein is decreased by at most 0.5% after 5-day storage at room temperature. In some embodiments, the purity of albuvirtide in the stable lyophilized compositions provided herein is decreased by at most 0.3% after 5-day storage at room temperature. In some embodiments, room temperature is between 22-26 °C. In some embodiments, room temperature is 25 °C.

In some embodiments, the purity of albuvirtide in the stable lyophilized compositions provided herein is decreased by at most 10%, at most 9%, at most 8%, at most 7%, at most 6%, at most 5%, at most 4%, at most 3%, at most 2%, at most 1%, at most 0.5%, or at most 0.3% after 30-day storage at refrigerated condition. In some embodiments, the purity of albuvirtide in the stable lyophilized compositions provided herein is decreased by at most 10% after 30-day storage at refrigerated condition. In some embodiments, the purity of albuvirtide in the stable lyophilized compositions provided herein is decreased by at most 9% after 30-day storage at refrigerated condition. In some embodiments, the purity of albuvirtide in the stable lyophilized compositions provided herein is decreased by at most 8% after 30-day storage at refrigerated condition. In some embodiments, the purity of albuvirtide in the stable lyophilized compositions provided herein is decreased by at most 7% after 30-day storage at refrigerated condition. In some embodiments, the purity of albuvirtide in the stable lyophilized compositions provided herein is decreased by at most 6% after 30-day storage at refrigerated condition. In some embodiments, the purity of albuvirtide in the stable lyophilized compositions provided herein is decreased by at most 5% after 30-day storage at refrigerated condition. In some embodiments, the purity of albuvirtide in the stable lyophilized compositions provided herein is decreased by at most 4% after 30-day storage at refrigerated condition. In some embodiments, the purity of albuvirtide in the stable lyophilized compositions provided herein is decreased by at most 3% after 30-day storage at refrigerated condition. In some embodiments, the purity of albuvirtide in the stable lyophilized compositions provided herein is decreased by at most 2% after 30-day storage at refrigerated condition. **In** some embodiments, the purity of albuvirtide in the stable lyophilized compositions provided herein is decreased by at most 1% after 30-day storage at refrigerated condition. In some embodiments, the purity of albuvirtide in the stable lyophilized compositions provided herein is decreased by at most 0.5% after 30-day storage at refrigerated condition. In some embodiments, the purity of albuvirtide in the stable lyophilized compositions provided herein is decreased by at most 0.3% after 30-day storage at refrigerated condition. In some embodiments, the stable lyophilized compositions stored at refrigerated condition is stored at 0-8 °C. In some embodiments, the stable lyophilized compositions stored at refrigerated condition is stored at 4 °C. In some embodiments, the stable lyophilized compositions stored at refrigerated condition is stored at 6 °C.

**In** some embodiments, the purity of albuvirtide in the stable lyophilized compositions provided herein is decreased by at most 20%, at most 19%, at most 18%, at most 17%, at most 16%, at most 15%, at most 14%, at most 13%, at most 12%, at most 11%, at most 10%, at most 9%, at most 8%, at most 7%, at most 6%, or at most 5% after 30-day storage at room temperature. In some embodiments, the purity of albuvirtide in the stable lyophilized compositions provided herein is decreased by at most 20% after 30-day storage at room temperature. In some embodiments, the purity of albuvirtide in the stable lyophilized compositions provided herein is decreased by at most 19% after 30-day storage at room temperature. In some embodiments, the purity of albuvirtide in the stable lyophilized compositions provided herein is decreased by at most 18% after 30-day storage at room temperature. In some embodiments, the purity of albuvirtide in the stable lyophilized compositions provided herein is decreased by at most 17% after 30-day storage at room temperature. In some embodiments, the purity of albuvirtide in the stable lyophilized compositions provided herein is decreased by at most 16% after 30-day storage at room temperature. In some embodiments, the purity of albuvirtide in the stable lyophilized compositions provided herein is decreased by at most 15% after 30-day storage at room temperature. In some embodiments, the purity of albuvirtide in the stable lyophilized compositions provided herein is decreased by at most 14% after 30-day storage at room temperature. In some embodiments, the purity of albuvirtide in the stable lyophilized compositions provided herein is decreased by at most 13% after 30-day storage at room temperature. In some embodiments, the purity of albuvirtide in the stable lyophilized compositions provided herein is decreased by at most 12% after 30-day storage at room temperature. In some embodiments, the purity of albuvirtide in the stable lyophilized compositions provided herein is decreased by at most 11% after 30-day storage at room temperature. In some embodiments, the purity of albuvirtide in the stable lyophilized compositions provided herein is decreased by at most 10% after 30-day storage at room temperature. In some embodiments, the purity of albuvirtide in the stable lyophilized compositions provided herein is decreased by at most 9% after 30-day storage at room temperature. In some embodiments, the purity of albuvirtide in the stable lyophilized compositions provided herein is decreased by at most 8% after 30-day storage at room temperature. In some embodiments, the purity of albuvirtide in the stable lyophilized compositions provided herein is decreased by at most 7% after 30-day storage at room temperature. In some embodiments, the purity of albuvirtide in the stable lyophilized compositions provided herein is decreased by at most 6% after 30-day storage at room temperature. In some embodiments, the purity of albuvirtide in the stable lyophilized compositions provided herein is decreased by at most 5% after 30-day storage at room temperature. In some embodiments, room temperature is between 22-26 °C. In some embodiments, room temperature is 25 °C.

In some embodiments, the purity of albuvirtide in the stable lyophilized compositions provided herein is decreased by at most 5%, at most 4%, at most 3%, at most 2%, at most 1%, at most 0.8%, at most 0.5%, at most 0.2%, or at most 0.1% after 3-month storage at frozen condition. In some embodiments, the purity of albuvirtide in the stable lyophilized compositions provided herein is decreased by at most 5% after 3-month storage at frozen condition. In some embodiments, the purity of albuvirtide in the stable lyophilized compositions provided herein is decreased by at most 4% after 3-month storage at frozen condition. In some embodiments, the purity of albuvirtide in the stable lyophilized compositions provided herein is decreased by at most 3% after 3-month storage at frozen condition. In some embodiments, the purity of albuvirtide in the stable lyophilized compositions provided herein is decreased by at most 2% after 3-month storage at frozen condition. In some embodiments, the purity of albuvirtide in the stable lyophilized compositions provided herein is decreased by at most 1% after 3-month storage at frozen condition. In some embodiments, the purity of albuvirtide in the stable lyophilized compositions provided herein is decreased by at most 0.8% after 3-month storage at frozen condition. In some embodiments, the purity of albuvirtide in the stable lyophilized compositions provided herein is decreased by at most 0.5% after 3-month storage at frozen condition. In some embodiments, the purity of albuvirtide in the stable lyophilized compositions provided herein is decreased by at most 0.2% after 3-month storage at frozen condition. In some embodiments, the purity of albuvirtide in the stable lyophilized compositions provided herein is decreased by at most 0.1% after 3-month storage at frozen condition. In some embodiments, frozen condition is between -25 °C and -15 °C. In some embodiments, frozen condition is about -20 °C.

Analytical methods for evaluating the product stability include HPLC, size exclusion chromatography (SEC), dynamic light scattering test (DLS), differential scanning calorimetery (DSC), iso-asp quantification, potency, UV/Vis spectroscopy, and Fourier-transform infrared spectroscopy (FTIR). SEC (J. Pharm. Scien., 83: 1645-1650, (1994); Pharm. Res., 1 1 :485 (1994); J. Pharm. Bio. Anal., 15: 1928 (1997); J. Pharm. Bio. Anal., 14:1 133-1 140 (1986)), measures percent monomer in the product and gives information of the amount of soluble aggregates. There methods are merely exemplary of methods for evaluating product stability well known to one of skill in the art. By way of example, UV/Vis spectroscopy measures scattered light intensity and gives information about the amounts of soluble and insoluble aggregates. UV spectroscopy measures absorbance and gives information of protein concentration. FTIR (Eur. J. Pharm. Biopharm., 45:231 (1998); Pharm. Res., 12:1250 (1995); J. Pharm. Scien., 85:1290 (1996); J. Pharm. Scien., 87:1069 (1998)) measures IR spectrum in the amide one region, and gives information of protein secondary structure.

Disclosed herein is a concentrated pre-lyophilized liquid composition comprising albuvirtide at a concentration between 5 mg/ml and 200 mg/ml and an acidic buffer at a concentration between 5 and 200 mM, wherein the composition has a pH between 1.0 and 3.5, which is lyophilized efficiently and effectively to a dry composition that retains the biological, physical and chemical stability of the albuvirtide. The lyophilized composition is stable for storage for prolonged periods of time at room temperature, refrigerated conditions, and/or frozen conditions.

The stable lyophilized compositions of albuvirtide provided herein can be further dissolved in water-for-injection and sterilized to make pharmaceutical compositions of albuvirtide. In some embodiments, sterilization comprises septic filtration. The liquid pharmaceutical compositions can be aliquoted. In some embodiments, each aliquot comprises 20 mg albuvirtide. In some embodiments, each aliquot comprises 50 mg albuvirtide. In some embodiments, each aliquot comprises 100 mg albuvirtide. In some embodiments, each aliquot comprises 150 mg albuvirtide. In some embodiments, each aliquot comprises 200 mg albuvirtide. In some embodiments, each aliquot comprises 300 mg albuvirtide. In some embodiments, each aliquot comprises 400 mg albuvirtide. In some embodiments, each aliquot comprises 500 mg albuvirtide. In some embodiments, each aliquot contains a single dose of albuvirtide.

In some embodiments, provided herein are pharmaceutical compositions having albuvirtide at a concentration between 10.0 mg/ml and 60.0 mg/ml. In some embodiments, provided herein are pharmaceutical compositions having albuvirtide at a concentration between 10.0 mg/ml and 40.0 mg/ml. In some embodiments, provided herein are pharmaceutical compositions having albuvirtide at a concentration between 10.0 mg/ml and 30.0 mg/ml. In some embodiments, provided herein are pharmaceutical compositions having albuvirtide at a concentration between 18.0 mg/ml and 22.0 mg/ml. In some embodiments, provided herein are pharmaceutical compositions having albuvirtide at a concentration of 6.0 mg/ml, 7.0 mg/ml, 8.0 mg/ml, 9.0 mg/ml, 10.0 mg/ml, 12.0 mg/ml, 14.0 mg/ml, 16.0 mg/ml, 18.0 mg/ml, 20.0 mg/ml, 22.0 mg/ml, 24.0 mg/ml, 26.0 mg/ml, 28.0 mg/ml, 30.0 mg/ml, 35.0 mg/ml, 40.0 mg/ml, 45.0 mg/ml, 50.0 mg/ml, 55.0 mg/ml, 60.0 mg/ml, 65.0 mg/ml, 70.0 mg/ml, 75.0 mg/ml, or 80.0 mg/ml.

In some embodiments, the liquid pharmaceutical compositions of albuvirtide can be lyophilized to prepare lyophilized pharmaceutical compositions. In some embodiments, the liquid pharmaceutical compositions of albuvirtide in aliquots can be lyophilized to prepare lyophilized pharmaceutical compositions in aliquots. In some embodiments, each aliquot comprises 20 mg albuvirtide. In some embodiments, each aliquot comprises 50 mg albuvirtide. In some embodiments, each aliquot comprises 100 mg albuvirtide. In some embodiments, each aliquot comprises 150 mg albuvirtide. In some embodiments, each aliquot comprises 200 mg albuvirtide. In some embodiments, each aliquot comprises 300 mg albuvirtide. In some embodiments, each aliquot comprises 400 mg albuvirtide. In some embodiments, each aliquot comprises 500 mg albuvirtide. In some embodiments, each aliquot contains a single dose of albuvirtide.

In lyophilized pharmaceutical compositions, the desired dosage can be obtained by lyophilizing the liquid pharmaceutical composition at the target albuvirtide concentration and reconstituting the product with the same volume as that of the starting fill volume. The desired dosage can also be obtained by lyophilizing a larger volume of a diluted composition, and reconstituting it with a less volume. For example, if a desired product dosage is 100 mg of albuvirtide in 1 mL of the composition, the compositions can be lyophilized with the following liquid configurations: 1 mL of 100 mg/mL, 2 mL of 50 mg/ml, or 4 mL of 25 mg/mL albuvirtide compositions. In all cases, the final product can be reconstituted with 1 mL diluent to obtain the target protein concentration of 100 mg/mL. However, as the albuvirtide concentration in the pre-lyophilized composition is reduced, the fill volume increases proportionately.

The lyophilized pharmaceutical composition can be reconstituted within a short time (e.g. less than ten minutes) to a solution containing about 10 mg/ml to about 80 mg/ml albuvirtide. The lyophilized pharmaceutical compositions can be reconstituted in a pharmaceutically acceptable carrier to make pharmaceutical formulations of albuvirtide that can be administered to a subject. In some embodiments, the subject is a human. In some embodiments, the pharmaceutically acceptable carrier comprises water-for-injection. In some embodiments, the pharmaceutically acceptable carrier comprises saline. In some embodiments, the pharmaceutically acceptable carrier comprises saline and sodium bicarbonate.

In some embodiments, the pharmaceutical formulations of albuvirtide are ready for parenteral administration. In some embodiments, the pharmaceutical formulations of albuvirtide are ready for intravenous administration. In some embodiments, the pharmaceutical formulations of albuvirtide are ready for intramuscular administration. In some embodiments, the pharmaceutical formulations of albuvirtide are ready for intraperitoneal administration. In some embodiments, the pharmaceutical formulations of albuvirtide are ready for subcutaneous injection.

In some embodiments, provided herein are also devices that can be used for delivering the pharmaceutical formulations having albuvirtide disclosed herein. Examples of such devices include, but are not limited to, a syringe, a pen, an implant, a needle-free injection device, an inhalation device, and a patch.

In some embodiments, provided herein are also articles of manufacture comprising the stable lyophilized compositions of albuvirtide disclosed herein. In some embodiments, provided herein are also articles of manufacture comprising the pharmaceutical compositions having albuvirtide disclosed herein. In some embodiments, provided herein are also articles of manufacture comprising the lyophilized pharmaceutical compositions having albuvirtide disclosed herein. In some embodiments, an article of manufacture is provided which contains the stable lyophilized compositions described herein and instructions for its use. The article of manufacture can include a container. Suitable containers include, for example, bottles, vials (e.g., dual chamber vials), syringes (such as dual chamber syringes), autoinjector pen containing a syringe, and test tubes. The container can be formed from a variety of materials such as glass, plastic or polycarbonate. The container holds the compositions described herein and the label on, or associated with, the container can indicate directions for use. For example, the label can indicate that the composition is useful or intended for subcutaneous administration. The container holding the composition can be a multi-use vial, which allows for repeat administrations (e.g., from 2-6 administrations) of the aqueous composition. The article of manufacture can further comprise a second container. The article of manufacture can further include other materials desirable from a commercial and user standpoint, including other buffers, diluents, filters, needles, syringes, and package inserts with instructions for use.

Provided herein are lyophilized pharmaceutical compositions comprising a mixture of albuvirtide and a stabilizer, which comprises an acid selected from the group consisting of a phosphoric acid, hydrochloric acid, acetic acid, citric acid, formic acid, trifluoroacetic acid (TFA), sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid; and a salt form thereof. In some embodiments, said stabilizer can comprise a phosphoric acid and a salt thereof. In some embodiments, said stabilizer can comprise a hydrochloric acid and a salt thereof. In some embodiments, said stabilizer can comprise an acetic acid and a salt thereof. In some embodiments, said stabilizer can comprise a citric acid and a salt thereof. In some embodiments, said stabilizer can comprise a formic acid and a salt thereof. In some embodiments, said stabilizer can comprise a TFA and a salt thereof. In some embodiments, said stabilizer can comprise a sulfuric acid and a salt thereof. In some embodiments, said stabilizer can comprise a tartaric acid and a salt thereof. In some embodiments, said stabilizer can comprise a lactic acid and a salt thereof. In some embodiments, said stabilizer can comprise a succinic acid and a salt thereof. In some embodiments, said stabilizer can comprise an ascorbic acid and a salt thereof. In some embodiments, said stabilizer can comprise an aspartic acid and a salt thereof. In some embodiments, said stabilizer can comprise a glutamic acid and a salt thereof. In some embodiments, said stabilizer can comprise a propanoic acid and a salt thereof. In some embodiments, said stabilizer can comprise a propanedioic acid and a salt thereof. In some embodiments, said stabilizer can comprise a butyric acid and a salt thereof. In some embodiments, said stabilizer can comprise a maleic acid and a salt thereof. In some embodiments, said stabilizer can comprise a fumaric acid and a salt thereof. In some embodiments, said stabilizer can comprise a malic acid and a salt thereof. In some embodiments, said stabilizer can comprise an oxalic acid and a salt thereof.

In some embodiments, the stabilizer provided herein can comprise an acid and salt form of the acid at a ratio of 100:0 (molar). In some embodiments, the stabilizer provided herein can comprise an acid and salt form of the acid at a ratio of about 90:10 (molar). In some embodiments, the liquid compositions provided herein can have the acid and salt form of the acid at a ratio of about 80:20 (molar). In some embodiments, the liquid compositions provided herein can have the acid and salt form of the acid at a ratio of about 70:30 (molar). In some embodiments, the liquid compositions provided herein can have the acid and salt form of the acid at a ratio of about 60:40 (molar). In some embodiments, the liquid compositions provided herein can have the acid and salt form of the acid at a ratio of about 50:50 (molar).

In some embodiments, the lyophilized pharmaceutical composition comprises said stabilizer and albuvirtide at a molar ratio between 1:10 and 200:1, between 1:5 and 150:1, between 1:5 and 100:1, between 1:2 and 50:1, between 1:1 and 25:1, between 2:1 and 25:1, between 3:1 and 10:1.

In some embodiments, the salt form of the acid comprises a sodium salt of the acid. In some embodiments, the salt form of the acid comprises a potassium salt of the acid. In some embodiments, the salt form of the acid comprises an ammonium salt of the acid. In some embodiments, the salt form of the acid comprises any combination of a sodium salt , a potassium salt, an ammonium salt of the acid.

In some embodiments, the lyophilized pharmaceutical composition provided herein comprise a stabilizer comprising a phosphate group and one or more cations selected from the group consist of sodium, potassium and ammonium. In some embodiments, the stabilizer is a salt of phosphoric acid that comprises one or more cations selected from the group consist of sodium, potassium and ammonium. In some embodiments, the lyophilized pharmaceutical composition comprises a salt of phosphoric acid at a molar ratio of the cation and phosphorus between 0:1 and 1:1, between 0.02:1 and 1:1, between 0.03:1 and 1:1, between 0.04:1 and 1:1, between 0.05:1 and 1:1, between 0.06:1 and 1:1, between 0.07:1 and 1:1, between 0.08:1 and 1:1, between 0.09:1 and 1:1, between 0.1:1 and 1:1, between 0:15 and 1:1, between 0.25:1 and 1:1, between 0.3:1 and 1:1, between 0.35:1 and 1:1, between 0.4:1 and 1:1, between 0.45:1 and 1:1, between 0.5:1 and 1:1, between 0.55:1 and 1:1, between 0.6:1 and 1:1, between 0.65:1 and 1:1, between 0.7:1 and 1:1, between 0.75:1 and 1:1, between 0.8:1 and 1:1, between 0.85:1 and 1:1, between 0.95:1 and 1:1. In some embodiments, the lyophilized pharmaceutical composition comprises a salt of phosphoric acid at a molar ratio of the cation and phosphorus between 0.02:1 and 0.95:1, between 0.03:1 and 0.95:1, between 0.04:1 and 0.95:1, between 0.05:1 and 0.95:1, between 0.06:1 and 0.95:1, between 0.07:1 and 0.95:1, between 0.08:1 and 0.95:1, between 0.09:1 and 0.95:1, between 0.1:1 and 0.95:1, between 0:15 and 0.95:1, between 0.25:1 and 0.95:1, between 0.3:1 and 0.95:1, between 0.35:1 and 0.95:1, between 0.4:1 and 0.95:1, between 0.45:1 and 0.95:1, between 0.5:1 and 0.95:1, between 0.55:1 and 0.95:1, between 0.6:1 and 0.95:1, between 0.65:1 and 0.95:1, between 0.7:1 and 0.95:1, between 0.75:1 and 0.95:1, between 0.8:1 and 0.95:1, between 0.85:1 and 0.95:1. In some embodiments, the lyophilized pharmaceutical composition comprises a salt of phosphoric acid at a molar ratio of the cation and phosphorus between 0.02:1 and 0.9:1, between 0.03:1 and 0.9:1, between 0.04:1 and 0.9:1, between 0.05:1 and 0.9:1, between 0.06:1 and 0.9:1, between 0.07:1 and 0.9:1, between 0.08:1 and 0.9:1, between 0.09:1 and 0.9:1, between 0.1:1 and 0.9:1, between 0:15 and 0.9:1, between 0.25:1 and 0.9:1, between 0.3:1 and 0.9:1, between 0.35:1 and 0.9:1, between 0.4:1 and 0.9:1, between 0.45:1 and 0.9:1, between 0.5:1 and 0.9:1, between 0.55:1 and 0.9:1, between 0.6:1 and 0.9:1, between 0.65:1 and 0.9:1, between 0.7:1 and 0.9:1, between 0.75:1 and 0.9:1, between 0.8:1 and 0.9:1, between 0.85:1 and 0.9:1. In some embodiments, the lyophilized pharmaceutical composition comprises a salt of phosphoric acid at a molar ratio of the cation and phosphorus between 0.02:1 and 0.85:1, between 0.03:1 and 0.85:1, between 0.04:1 and 0.85:1, between 0.05:1 and 0.85:1, between 0.06:1 and 0.85:1, between 0.07:1 and 0.85:1, between 0.08:1 and 0.85:1, between 0.09:1 and 0.85:1, between 0.1:1 and 0.85:1, between 0:15 and 0.85:1, between 0.25:1 and 0.85:1, between 0.3:1 and 0.85:1, between 0.35:1 and 0.85:1, between 0.4:1 and 0.85:1, between 0.45:1 and 0.85:1, between 0.5:1 and 0.85:1, between 0.55:1 and 0.85:1, between 0.6:1 and 0.85:1, between 0.65:1 and 0.85:1, between 0.7:1 and 0.85:1, between 0.75:1 and 0.85:1, between 0.8:1 and 0.85:1. In some embodiments, the lyophilized pharmaceutical composition comprises a salt of phosphoric acid at a molar ratio of the cation and phosphorus between 0.02:1 and 0.8:1, between 0.03:1 and 0.8:1, between 0.04:1 and 0.8:1, between 0.05:1 and 0.8:1, between 0.06:1 and 0.8:1, between 0.07:1 and 0.8:1, between 0.08:1 and 0.8:1, between 0.09:1 and 0.8:1, between 0.1:1 and 0.8:1, between 0:15 and 0.8:1, between 0.25:1 and 0.8:1, between 0.3:1 and 0.8:1, between 0.35:1 and 0.8:1, between 0.4:1 and 0.8:1, between 0.45:1 and 0.8:1, between 0.5:1 and 0.8:1, between 0.55:1 and 0.8:1, between 0.6:1 and 0.8:1, between 0.65:1 and 0.8:1, between 0.7:1 and 0.8:1, between 0.75:1 and 0.8:1. In some embodiments, the lyophilized pharmaceutical composition comprises a salt of phosphoric acid at a molar ratio of the cation and phosphorus between 0.02:1 and 0.75:1, between 0.03:1 and 0.75:1, between 0.04: 1 and 0.75:1, between 0.05:1 and 0.75:1, between 0.06:1 and 0.75:1, between 0.07:1 and 0.75:1, between 0.08:1 and 0.75:1, between 0.09:1 and 0.75:1, between 0.1:1 and 0.75:1, between 0:15 and 0.75:1, between 0.25:1 and 0.75:1, between 0.3:1 and 0.75:1, between 0.35:1 and 0.75:1, between 0.4:1 and 0.75:1, between 0.45:1 and 0.75:1, between 0.5:1 and 0.75:1, between 0.55:1 and 0.75:1, between 0.6:1 and 0.75:1, between 0.65:1 and 0.75:1, between 0.7:1 and 0.75:1. In some embodiments, the lyophilized pharmaceutical composition comprises a salt of phosphoric acid at a molar ratio of the cation and phosphorus between 0.02:1 and 0.7:1, between 0.03:1 and 0.7:1, between 0.04:1 and 0.7:1, between 0.05:1 and 0.7:1, between 0.06:1 and 0.7:1, between 0.07:1 and 0.7:1, between 0.08:1 and 0.7:1, between 0.09:1 and 0.7:1, between 0.1:1 and 0.7:1, between 0:15 and 0.7:1, between 0.25:1 and 0.7:1, between 0.3:1 and 0.7:1, between 0.35:1 and 0.7:1, between 0.4:1 and 0.7:1, between 0.45:1 and 0.7:1, between 0.5:1 and 0.7:1, between 0.55:1 and 0.7:1, between 0.6:1 and 0.7:1, between 0.65:1 and 0.7:1. In some embodiments, the lyophilized pharmaceutical composition comprises a salt of phosphoric acid at a molar ratio of the cation and phosphorus between 0.02:1 and 0.65:1, between 0.03:1 and 0.65:1, between 0.04: 1 and 0.65:1, between 0.05:1 and 0.65:1, between 0.06:1 and 0.65:1, between 0.07:1 and 0.65:1, between 0.08:1 and 0.65:1, between 0.09:1 and 0.65:1, between 0.1:1 and 0.65:1, between 0:15 and 0.65:1, between 0.25:1 and 0.65:1, between 0.3:1 and 0.65:1, between 0.35:1 and 0.65:1, between 0.4:1 and 0.65:1, between 0.45:1 and 0.65:1, between 0.5:1 and 0.65:1, between 0.55:1 and 0.65:1, between 0.6:1 and 0.65:1. In some embodiments, the lyophilized pharmaceutical composition comprises a salt of phosphoric acid at a molar ratio of the cation and phosphorus between 0.02:1 and 0.6:1, between 0.03:1 and 0.6:1, between 0.04:1 and 0.6:1, between 0.05:1 and 0.6:1, between 0.06:1 and 0.6:1, between 0.07:1 and 0.6:1, between 0.08:1 and 0.6:1, between 0.09:1 and 0.6:1, between 0.1:1 and 0.6:1, between 0:15 and 0.6:1, between 0.25:1 and 0.6:1, between 0.3:1 and 0.6:1, between 0.35:1 and 0.6:1, between 0.4:1 and 0.6:1, between 0.45:1 and 0.6:1, between 0.5:1 and 0.6:1, between 0.55:1 and 0.6:1. In some embodiments, the lyophilized pharmaceutical composition comprises a salt of phosphoric acid at a molar ratio of the cation and phosphorus between 0.02:1 and 0.55:1, between 0.03:1 and 0.55:1, between 0.04:1 and 0.55:1, between 0.05:1 and 0.55:1, between 0.06:1 and 0.55:1, between 0.07:1 and 0.55:1, between 0.08:1 and 0.55:1, between 0.09:1 and 0.55:1, between 0.1:1 and 0.55:1, between 0:15 and 0.55:1, between 0.25:1 and 0.55:1, between 0.3:1 and 0.55:1, between 0.35:1 and 0.55:1, between 0.4:1 and 0.55:1, between 0.45:1 and 0.55:1, between 0.5:1 and 0.55:1. In some embodiments, the lyophilized pharmaceutical composition comprises a salt of phosphoric acid at a molar ratio of the cation and phosphorus between 0.02:1 and 0.5:1, between 0.03:1 and 0.5:1, between 0.04:1 and 0.5:1, between 0.05:1 and 0.5:1, between 0.06:1 and 0.5:1, between 0.07:1 and 0.5:1, between 0.08:1 and 0.5:1, between 0.09:1 and 0.5:1, between 0.1:1 and 0.5:1, between 0:15 and 0.5:1, between 0.25:1 and 0.5:1, between 0.3:1 and 0.5:1, between 0.35:1 and 0.5:1, between 0.4:1 and 0.5:1, between 0.45:1 and 0.5:1. In some embodiments, the lyophilized pharmaceutical composition comprises a salt of phosphoric acid at a molar ratio of the cation and phosphorus between 0.02:1 and 0.45:1, between 0.03:1 and 0.45:1, between 0.04:1 and 0.45:1, between 0.05:1 and 0.45:1, between 0.06:1 and 0.45:1, between 0.07:1 and 0.45:1, between 0.08:1 and 0.45:1, between 0.09:1 and 0.45:1, between 0.1:1 and 0.45:1, between 0:15 and 0.45:1, between 0.25:1 and 0.45:1, between 0.3:1 and 0.45:1, between 0.35:1 and 0.45:1, between 0.4:1 and 0.45:1. In some embodiments, the lyophilized pharmaceutical composition comprises a salt of phosphoric acid at a molar ratio of the cation and phosphorus between 0.02:1 and 0.4:1, between 0.03:1 and 0.4:1, between 0.04:1 and 0.4:1, between 0.05:1 and 0.4:1, between 0.06:1 and 0.4:1, between 0.07:1 and 0.4:1, between 0.08:1 and 0.4:1, between 0.09:1 and 0.4:1, between 0.1:1 and 0.4:1, between 0:15 and 0.4:1, between 0.25:1 and 0.4:1, between 0.3:1 and 0.4:1, between 0.35:1 and 0.4:1. In some embodiments, the lyophilized pharmaceutical composition comprises a salt of phosphoric acid at a molar ratio of the cation and phosphorus between 0.02:1 and 0.35:1, between 0.03:1 and 0.35:1, between 0.04:1 and 0.35:1, between 0.05:1 and 0.35:1, between 0.06:1 and 0.35:1, between 0.07:1 and 0.35:1, between 0.08:1 and 0.35:1, between 0.09:1 and 0.35:1, between 0.1:1 and 0.35:1, between 0:15 and 0.35:1, between 0.25:1 and 0.35:1, between 0.3:1 and 0.35:1. In some embodiments, the lyophilized pharmaceutical composition comprises a salt of phosphoric acid at a molar ratio of the cation and phosphorus between 0.02:1 and 0.3:1, between 0.03:1 and 0.3:1, between 0.04:1 and 0.3:1, between 0.05:1 and 0.3:1, between 0.06:1 and 0.3:1, between 0.07:1 and 0.3:1, between 0.08:1 and 0.3:1, between 0.09:1 and 0.3:1, between 0.1:1 and 0.3:1, between 0:15 and 0.3:1, between 0.25:1 and 0.3:1. In some embodiments, the lyophilized pharmaceutical composition comprises a salt of phosphoric acid at a molar ratio of the cation and phosphorus between 0.02:1 and 0.25:1, between 0.03:1 and 0.25:1, between 0.04: 1 and 0.25:1, between 0.05:1 and 0.25:1, between 0.06:1 and 0.25:1, between 0.07:1 and 0.25:1, between 0.08:1 and 0.25:1, between 0.09:1 and 0.25:1, between 0.1:1 and 0.25:1, between 0:15 and 0.25:1, between 0.2:1 and 0.25:1. In some embodiments, the lyophilized pharmaceutical composition comprises a salt of phosphoric acid at a molar ratio of the cation and phosphorus between 0.02:1 and 0.2:1, between 0.03:1 and 0.2:1, between 0.04:1 and 0.2:1, between 0.05:1 and 0.2: 1, between 0.06:1 and 0.2:1, between 0.07:1 and 0.2:1, between 0.08:1 and 0.2:1, between 0.09:1 and 0.2:1, between 0.1:1 and 0.2:1, between 0:15 and 0.2:1.

The measurement of sodium can be carried out with the standard curve method (nonlinear fitting) for flame spectrometry (Chinese Pharmacopoeia 2015 version, Volume IV, General Chapter 0407 General Chapter 3110). The sodium ion content is determined with respect to the luminous intensity according to the external standard method.

The measurement of potassium can be carried out with the standard curve method (nonlinear fitting) for flame spectrometry (Chinese Pharmacopoeia 2015 version, Volume IV, General Chapter 0407, General Chapter 3109). The potassium ion content is determined with respect to the luminous intensity according to the external standard method.

The measurement of ammonium can be carried out with titration method (Chinese Pharmacopoeia 2015 version, Volume IV, General Chapter 3104). Each ml of sulfuric acid (0.05 mol/L) volumetric solution is equivalent to 1.804 mg of NH₄⁺.

The measurement of phosphorous can be taken using molybdate assay. Phosphate ion reacts with ammonium molybdate in the acid solution to generate ammonium phosphomolybdate, which will turn into a blue substance called "molybdenum blue" in the presence of the reducing agent (the mixture of molybdenum trioxide and molybdenum hemipentoxide). Carry out the method for ultra - visible spectrophotometry (Chinese Pharmacopoeia 2015 version, Volume IV, General Chapter 0401, General Chapter 3103), the phosphorous content is determined with respect to the absorbance according to the standard curve method at the wavelength of 820 nm.

In some embodiments, when the lyophilized pharmaceutical composition is dissolved in water to form a aqueous solution having albuvirtide at a concentration of 10.0 mg/mL, the formed aqueous solution has a pH between 1.0 and 3.5, between 1.0 and 3.0, or between 1.0 and 2.5. In some embodiments, when the lyophilized pharmaceutical composition is dissolved in water to form a aqueous solution having albuvirtide at a concentration of 10.0 mg/mL, the formed aqueous solution has a pH between 1.5 and 4.5, between 1.5 and 4.0, between 1.5 and 3.5, between 1.5 and 3.0, or between 1.5 and 2.5. In some embodiments, when the lyophilized pharmaceutical composition is dissolved in water to form a aqueous solution having albuvirtide at a concentration of 10.0 mg/mL, the formed aqueous solution has a pH between 2.0 and 4.0, between 2.0 and 3.5, between 2.0 and 3.0, between 2.0 and 2.5.

The measurements of the pH can be taken at the temperature of 4 °C. The measurements of the pH can be taken at room temperature. The measurements of the pH can be taken at about 22 °C.

In some embodiments, the lyophilized pharmaceutical composition comprises a stabilizer comprising sodium dihydrogen phosphate and phosphoric acid, and the ratio of phosphoric acid and sodium dihydrogen phosphate is between 100:0 (molar) and 5:95 (molar), between 95:5 (molar) and 5:95 (molar), between 90:10 (molar) and 10:90 (molar), between 85:15 (molar) and 15:85 (molar), between 80:20 (molar) and 20:80 (molar), between 75:25 (molar) and 25:75 (molar), between 70:30 (molar) and 30:70 (molar), between 65:35 (molar) and 35:65 (molar), between 60:40 (molar) and 40:60 (molar), between 55:45 (molar) and 45:55 (molar), or at about 50:50 (molar).

This lyophilized pharmaceutical composition retains the stability of the albuvirtide, and prevents the albuvirtide intended for administration to human subjects from physical and chemical degradation during storage.

In some embodiments, the purity of albuvirtide in the lyophilized pharmaceutical compositions provided herein is decreased by at most 10%, at most 9%, at most 8%, at most 7%, at most 6%, at most 5%, at most 4%, at most 3%, at most 2%, at most 1%, at most 0.5%, at most 0.3% after 5-day storage at room temperature. In some embodiments, the purity of albuvirtide in the lyophilized pharmaceutical compositions provided herein is decreased by at most 10% after 5-day storage at room temperature. In some embodiments, the purity of albuvirtide in the lyophilized pharmaceutical compositions provided herein is decreased by at most 9% after 5-day storage at room temperature. In some embodiments, the purity of albuvirtide in the lyophilized pharmaceutical compositions provided herein is decreased by at most 8% after 5-day storage at room temperature. In some embodiments, the purity of albuvirtide in the lyophilized pharmaceutical compositions provided herein is decreased by at most 7% after 5-day storage at room temperature. In some embodiments, the purity of albuvirtide in the lyophilized pharmaceutical compositions provided herein is decreased by at most 6% after 5-day storage at room temperature. In some embodiments, the purity of albuvirtide in the lyophilized pharmaceutical compositions provided herein is decreased by at most 5% after 5-day storage at room temperature. In some embodiments, the purity of albuvirtide in the lyophilized pharmaceutical compositions provided herein is decreased by at most 4% after 5-day storage at room temperature. In some embodiments, the purity of albuvirtide in the lyophilized pharmaceutical compositions provided herein is decreased by at most 3% after 5-day storage at room temperature. In some embodiments, the purity of albuvirtide in the lyophilized pharmaceutical compositions provided herein is decreased by at most 2% after 5-day storage at room temperature. In some embodiments, the purity of albuvirtide in the lyophilized pharmaceutical compositions provided herein is decreased by at most 1% after 5-day storage at room temperature. In some embodiments, the purity of albuvirtide in the lyophilized pharmaceutical compositions provided herein is decreased by at most 0.5% after 5-day storage at room temperature. In some embodiments, the purity of albuvirtide in the lyophilized pharmaceutical compositions provided herein is decreased by at most 0.3% after 5-day storage at room temperature. In some embodiments, room temperature is between 22-26 °C. In some embodiments, room temperature is 25 °C.

In some embodiments, the purity of albuvirtide in the lyophilized pharmaceutical compositions provided herein is decreased by at most 10%, at most 9%, at most 8%, at most 7%, at most 6%, at most 5%, at most 4%, at most 3%, at most 2%, at most 1%, at most 0.5%, at most 0.3% after 30-day storage at refrigerated condition. In some embodiments, the purity of albuvirtide in the lyophilized pharmaceutical compositions provided herein is decreased by at most 10% after 30-day storage at refrigerated condition. In some embodiments, the purity of albuvirtide in the lyophilized pharmaceutical compositions provided herein is decreased by at most 9% after 30-day storage at refrigerated condition. In some embodiments, the purity of albuvirtide in the lyophilized pharmaceutical compositions provided herein is decreased by at most 8% after 30-day storage at refrigerated condition. In some embodiments, the purity of albuvirtide in the lyophilized pharmaceutical compositions provided herein is decreased by at most 7% after 30-day storage at refrigerated condition. In some embodiments, the purity of albuvirtide in the lyophilized pharmaceutical compositions provided herein is decreased by at most 6% after 30-day storage at refrigerated condition. In some embodiments, the purity of albuvirtide in the lyophilized pharmaceutical compositions provided herein is decreased by at most 5% after 30-day storage at refrigerated condition. In some embodiments, the purity of albuvirtide in the lyophilized pharmaceutical compositions provided herein is decreased by at most 4% after 30-day storage at refrigerated condition. In some embodiments, the purity of albuvirtide in the lyophilized pharmaceutical compositions provided herein is decreased by at most 3% after 30-day storage at refrigerated condition. In some embodiments, the purity of albuvirtide in the lyophilized pharmaceutical compositions provided herein is decreased by at most 2% after 30-day storage at refrigerated condition. In some embodiments, the purity of albuvirtide in the lyophilized pharmaceutical compositions provided herein is decreased by at most 1% after 30-day storage at refrigerated condition. In some embodiments, the purity of albuvirtide in the lyophilized pharmaceutical compositions provided herein is decreased by at most 0.5% after 30-day storage at refrigerated condition. In some embodiments, the purity of albuvirtide in the lyophilized pharmaceutical compositions provided herein is decreased by at most 0.3% after 30-day storage at refrigerated condition. In some embodiments, the lyophilized pharmaceutical compositions stored at refrigerated condition is stored at 0-8 °C. In some embodiments, the stable lyophilized compositions stored at refrigerated condition is stored at 4 °C. In some embodiments, the lyophilized pharmaceutical compositions stored at refrigerated condition is stored at 6 °C.

In some embodiments, the purity of albuvirtide in the lyophilized pharmaceutical compositions provided herein is decreased by at most 20%, at most 19%, at most 18%, at most 17%, at most 16%, at most 15%, at most 14%, at most 13%, at most 12%, at most 11%, at most 10%, at most 9%, at most 8%, at most 7%, at most 6%, or at most 5% after 30-day storage at room temperature. In some embodiments, the purity of albuvirtide in the lyophilized pharmaceutical compositions provided herein is decreased by at most 20% after 30-day storage at room temperature. In some embodiments, the purity of albuvirtide in the lyophilized pharmaceutical compositions provided herein is decreased by at most 19% after 30-day storage at room temperature. In some embodiments, the purity of albuvirtide in the lyophilized pharmaceutical compositions provided herein is decreased by at most 18% after 30-day storage at room temperature. In some embodiments, the purity of albuvirtide in the lyophilized pharmaceutical compositions provided herein is decreased by at most 17% after 30-day storage at room temperature. In some embodiments, the purity of albuvirtide in the lyophilized pharmaceutical compositions provided herein is decreased by at most 16% after 30-day storage at room temperature. In some embodiments, the purity of albuvirtide in the lyophilized pharmaceutical compositions provided herein is decreased by at most 15% after 30-day storage at room temperature. In some embodiments, the purity of albuvirtide in the lyophilized pharmaceutical compositions provided herein is decreased by at most 14% after 30-day storage at room temperature. In some embodiments, the purity of albuvirtide in the lyophilized pharmaceutical compositions provided herein is decreased by at most 13% after 30-day storage at room temperature. In some embodiments, the purity of albuvirtide in the lyophilized pharmaceutical compositions provided herein is decreased by at most 12% after 30-day storage at room temperature. In some embodiments, the purity of albuvirtide in the lyophilized pharmaceutical compositions provided herein is decreased by at most 11% after 30-day storage at room temperature. In some embodiments, the purity of albuvirtide in the lyophilized pharmaceutical compositions provided herein is decreased by at most 10% after 30-day storage at room temperature. In some embodiments, the purity of albuvirtide in the lyophilized pharmaceutical compositions provided herein is decreased by at most 9% after 30-day storage at room temperature. In some embodiments, the purity of albuvirtide in the lyophilized pharmaceutical compositions provided herein is decreased by at most 8% after 30-day storage at room temperature. In some embodiments, the purity of albuvirtide in the lyophilized pharmaceutical compositions provided herein is decreased by at most 7% after 30-day storage at room temperature. In some embodiments, the purity of albuvirtide in the lyophilized pharmaceutical compositions provided herein is decreased by at most 6% after 30-day storage at room temperature. In some embodiments, the purity of albuvirtide in the lyophilized pharmaceutical compositions provided herein is decreased by at most 5% after 30-day storage at room temperature. In some embodiments, room temperature is between 22-26 °C. In some embodiments, room temperature is 25 °C.

In some embodiments, the purity of albuvirtide in the lyophilized pharmaceutical compositions provided herein is decreased by at most 5%, at most 4%, at most 3%, at most 2%, at most 1%, at most 0.8%, at most 0.5%, at most 0.2%, or at most 0.1% after 3-month storage at frozen condition. In some embodiments, the purity of albuvirtide in the lyophilized pharmaceutical compositions provided herein is decreased by at most 5% after 3-month storage at frozen condition. In some embodiments, the purity of albuvirtide in the lyophilized pharmaceutical compositions provided herein is decreased by at most 4% after 3-month storage at frozen condition. In some embodiments, the purity of albuvirtide in the lyophilized pharmaceutical compositions provided herein is decreased by at most 3% after 3-month storage at frozen condition. In some embodiments, the purity of albuvirtide in the lyophilized pharmaceutical compositions provided herein is decreased by at most 2% after 3-month storage at frozen condition. In some embodiments, the purity of albuvirtide in the lyophilized pharmaceutical compositions provided herein is decreased by at most 1% after 3-month storage at frozen condition. In some embodiments, the purity of albuvirtide in the lyophilized pharmaceutical compositions provided herein is decreased by at most 0.8% after 3-month storage at frozen condition. In some embodiments, the purity of albuvirtide in the lyophilized pharmaceutical compositions provided herein is decreased by at most 0.5% after 3-month storage at frozen condition. In some embodiments, the purity of albuvirtide in the lyophilized pharmaceutical compositions provided herein is decreased by at most 0.2% after 3-month storage at frozen condition. In some embodiments, the purity of albuvirtide in the lyophilized pharmaceutical compositions provided herein is decreased by at most 0.1% after 3-month storage at frozen condition. In some embodiments, frozen condition is between -25 and -15 °C. In some embodiments, frozen condition is about -20 °C.

### C. Methods of Treatment (not encompassed by the wording of the claims)

Albuvirtide prevents HIV replication by inhibiting the fusion of the viral and cellular membranes. Albuvirtide has demonstrated in vitro activity against various HIV-1 strains and certain variants that are resistant to enfuvirtide (Fuzeon). In vivo, albuvirtide irreversibly conjugates with serum albumin, allowing for an extended peptide half-life. In some embodiments, the albuvirtide pharmaceutical formulations disclosed herein can be administered to a mammal in need of treatment, in accordance with known methods.

In some embodiments, provided herein are methods of inhibiting replication of HIV in a subject in need thereof by administering to the subject a therapeutically effective amounts of the albuvirtide pharmaceutical formulations disclosed herein. In some embodiments, provided herein are methods for treating or preventing HIV infection in a subject by administering to the subject a therapeutically effective amounts of the albuvirtide pharmaceutical formulations disclosed herein. In some embodiments, the subject has acquired immune deficiency syndromes (AIDS). In some embodiments, the subjects can have HIV infection. In some embodiments, the subjects can be at risk of HIV infection. In some embodiments, the subjects can be exposed to HIV. In some embodiments, the subjects can be at a high risk of being exposed to HIV.

Disclosed herein are methods for preventing HIV infection in a subject by administering to the subject a therapeutically effective amounts of the albuvirtide pharmaceutical formulations disclosed herein. The subject may be at a high risk of being exposed to HIV. The subject may have exposed to HIV within 24 hours. The subject may have been exposed to HIV within 48 hours. The subject may have been exposed to HIV within 72 hours.

Disclosed herein are methods for treating HIV infection in a subject by administering to the subject therapeutically effective amounts of the albuvirtide pharmaceutical formulations provided herein. The subjects may have HIV infection. The subject may have AIDS.

Disclosed herein are methods for treating or preventing AIDS in a subject by administering to the subject a therapeutically effective amounts of the albuvirtide pharmaceutical formulations provided herein.

These methods can include, but are not limited to intravenous administration as a bolus or by continuous infusion over a period of time, by intramuscular, intraperitoneal, intracerobrospinal, subcutaneous, intra-articular, intrasynovial, intrathecal, oral, topical, or inhalation routes. In some embodiments, the albuvirtide pharmaceutical formulations disclosed herein are administered by intramuscular administration. In some embodiments, the albuvirtide pharmaceutical formulations disclosed herein are administered by subcutaneous administration. In some embodiments, the albuvirtide pharmaceutical formulations disclosed herein are administered by intravenous administration. In some embodiments, the albuvirtide pharmaceutical formulations disclosed herein are administered by intraperitoneal administration. In some embodiments, the albuvirtide pharmaceutical formulations disclosed herein are administered by oral administration. In some embodiments, the albuvirtide pharmaceutical formulations disclosed herein are administered by topical administration. In some embodiments, the albuvirtide pharmaceutical formulations disclosed herein are administered by inhalation routes. In some embodiments, the albuvirtide pharmaceutical formulations disclosed herein are administered by intracerobrospinal administration.

The appropriate dosage of the albuvirtide will depend, for example, on the condition to be treated, the severity and course of the condition, whether the albuvirtide is administered for preventive or therapeutic purposes, previous therapy, the patient's clinical history and response to albuvirtide, and the discretion of the attending physician. Typically, the clinician will administer albuvirtide until a dosage is reached that achieves the desired effect. The progress of this therapy can be easily monitored by conventional assays. The subject being treated may be a human.

Methods disclosed herein comprise administering 100-2000 mg, 100-1800 mg, 100-1500 mg, 100-1200 mg, 100-1000 mg, 100-800 mg, 100-600 mg, or 100-400 mg albuvirtide to the subject in need thereof. Methods disclosed herein comprise administering 100-2000 mg albuvirtide to the subject in need thereof. Methods disclosed herein comprise administering 100-1800 mg albuvirtide to the subject in need thereof. Methods disclosed herein comprise administering 100-1600 mg albuvirtide to the subject in need thereof. Methods disclosed herein comprise administering 100-1400 mg albuvirtide to the subject in need thereof. Methods disclosed herein comprise administering 100-1200 mg albuvirtide to the subject in need thereof. Methods disclosed herein comprise administering 100-1000 mg albuvirtide to the subject in need thereof. Methods disclosed herein comprise administering 100-800 mg albuvirtide to the subject in need thereof. Methods disclosed herein comprise administering 100-600 mg albuvirtide to the subject in need thereof. Methods disclosed herein comprise administering 100-400 mg albuvirtide to the subject in need thereof. Methods disclosed herein comprise administering 200-400 mg albuvirtide to the subject in need thereof. Methods disclosed herein comprise administering 300-360 mg albuvirtide to the subject in need thereof.

Methods disclosed herein comprise administering 100 mg, 200 mg, 400 mg, 600 mg, 800 mg, 1000 mg, 1200 mg, 1400 mg, 1600 mg, 1800 mg, or 2000 mg albuvirtide to the subject in need thereof. Methods disclosed herein comprise administering 100 mg albuvirtide to the subject in need thereof. Methods disclosed herein comprise administering 200 mg albuvirtide to the subject in need thereof. Methods disclosed herein comprise administering 400 mg albuvirtide to the subject in need thereof. Methods disclosed herein comprise administering 600 mg albuvirtide to the subject in need thereof. Methods disclosed herein comprise administering 800 mg albuvirtide to the subject in need thereof. Methods disclosed herein comprise administering 1000 mg albuvirtide to the subject in need thereof. Methods disclosed herein comprise administering 1200 mg albuvirtide to the subject in need thereof. Methods disclosed herein comprise administering 1400 mg albuvirtide to the subject in need thereof. Methods disclosed herein comprise administering 1600 mg albuvirtide to the subject in need thereof. Methods disclosed herein comprise administering 1800 mg albuvirtide to the subject in need thereof. Methods disclosed herein comprise administering 2000 mg albuvirtide to the subject in need thereof.

Methods disclosed herein comprise administering 100 mg, 120 mg, 140 mg, 160 mg, 180 mg, 200 mg, 220 mg, 240 mg, 260 mg, 280 mg, 300 mg, 320 mg, 340 mg, 360 mg, 380 mg, 400 mg, 420 mg, 440 mg, 460 mg, 480 mg, 500 mg, 520 mg, 540 mg, 560 mg, 580 mg, 600 mg, 620 mg, or 640 mg albuvirtide to the subject in need thereof. Methods disclosed herein comprise administering 100 mg albuvirtide to the subject in need thereof. Methods disclosed herein comprise administering 120 mg albuvirtide to the subject in need thereof. Methods disclosed herein comprise administering 140 mg albuvirtide to the subject in need thereof. Methods disclosed herein comprise administering 160 mg albuvirtide to the subject in need thereof. Methods disclosed herein comprise administering 180 mg albuvirtide to the subject in need thereof. Methods disclosed herein comprise administering 200 mg albuvirtide to the subject in need Methods disclosed herein comprise administering 220 mg albuvirtide to the subject in need Methods disclosed herein comprise administering 240 mg albuvirtide to the subject in need thereof. Methods disclosed herein comprise administering 280 mg albuvirtide to the subject in need thereof. Methods disclosed herein comprise administering 320 mg albuvirtide to the subject in need thereof. Methods disclosed herein comprise administering 360 mg albuvirtide to the subject in need Methods disclosed herein comprise administering 400 mg albuvirtide to the subject in need Methods disclosed herein comprise administering 440 mg albuvirtide to the subject in need thereof. Methods disclosed herein comprise administering 480 mg albuvirtide to the subject in need thereof. Methods disclosed herein comprise administering 520 mg albuvirtide to the subject in need thereof. Methods disclosed herein comprise administering 560 mg albuvirtide to the subject in need thereof. Methods disclosed herein comprise administering 600 mg albuvirtide to the subject in need thereof. Methods disclosed herein comprise administering 640 mg albuvirtide to the subject in need thereof.

Methods disclosed herein comprise weekly administration of albuvirtide to the subject in need thereof. Methods disclosed herein comprise biweekly administration of albuvirtide to the subject in need Methods disclosed herein comprise monthly administration of albuvirtide to the subject in need thereof. Methods disclosed herein comprise bimonthly administration of albuvirtide to the subject in need thereof. Methods disclosed herein comprise quarterly administration of albuvirtide to the subject in need thereof.

Methods disclosed herein comprise administering the albuvirtide pharmaceutical formulations disclosed herein to a subject in need of include weekly administration of 320 mg albuvirtide. Methods disclosed herein comprise administering the albuvirtide pharmaceutical formulations to a subject in need of include biweekly administration of 320 mg albuvirtide.

The albuvirtide pharmaceutical formulations can be administered as the sole treatment. The albuvirtide pharmaceutical formulations can be administered in conjunction with other drugs or therapies useful in preventing or treating HIV infection. Two (or more) agents may be administered in combination when the two agents are administered simultaneously or are administered independently in a fashion such that the agents will act contemporaneously.

Methods disclosed herein include administering the albuvirtide pharmaceutical formulations disclosed herein in combination with one or more additional HIV treatment(s). The albuvirtide pharmaceutical formulations disclosed here may be administered prior to the additional HIV treatment. The albuvirtide pharmaceutical formulations disclosed here may be administered subsequent to the additional HIV The albuvirtide pharmaceutical formulations disclosed herein and the additional HIV treatment may be administered contemporaneously. The albuvirtide pharmaceutical formulations disclosed herein and the additional HIV treatment may be administered during same treatment period. The albuvirtide pharmaceutical formulations disclosed herein and the additional HIV treatment may be administered during overlapping treatment periods.

Methods disclosed herein include administering the albuvirtide pharmaceutical formulations disclosed herein in combination with one or more additional HIV treatment, the one or more additional HIV treatment can be any additional HIV treatment known in the art to be safe and efficacious for HIV treatment. The one or more additional HIV treatment may be any HIV treatment approved by U.S. Food and Drug Administration (FDA) or an equivalent regulatory agency in another jurisdiction. The one or more additional HIV treatment may be selected from the group consisting of: (a) abacavir (ZIAGEN); (b) enteric coated didanosine (VIDEX EC); (c) didanosine (VIDEX); (d) emtricitabine (EMTRIVA); (e) lamivudine (EPIVIR); (f) stavudine (ZERIT); (g) tenofovir alafenamide (VEMLIDY); (h) tenofovir disoproxil fumarate (VIREAD); (i) zidovudine (RETROVIR); (j) efavirenz (SUSTIVA); (k) etravirine (INTELENCE); (l) nevirapine (VIRAMUNE); (m) rilpivirine (EDURANT); (n) atazanavir (REYATAZ); (o) ATV/c (EVOTAZ); (p) darunavir (PREZISTA); (q) DRV/c (PREZCOBIX); (r) fosamprenavir (LEXIVA); (s) indinavir (CRIXIVAN); (t) lopinavir/ ritonavir (KALETRA); (u) nelfinavir (VIRACEPT); (v) ritonavir (NORVIR); (w) saquinavir (INVIRASE); (x) tipranavir (APTIVUS); (y) dolutegravir (TIVICAY); (z) raltegravir (ISENTRESS); (aa) enfuvirtide (FUZEON); (ab) maraviroc (SETZENTRY); (ac) ibalizumab (TROGARZO); and any combination thereof.

The one or more additional HIV treatment can be abacavir (ZIAGEN). The one or more additional HIV treatment can be enteric coated didanosine (VIDEX EC). The one or more additional HIV treatment can be didanosine (VIDEX). The one or more additional HIV treatment can be emtricitabine (EMTRIVA). The one or more additional HIV treatment can be lamivudine (EPIVIR). The one or more additional HIV treatment can be stavudine (ZERIT). The one or more additional HIV treatment can be tenofovir alafenamide (VEMLIDY). The one or more additional HIV treatment can be tenofovir disoproxil fumarate (VIREAD). The one or more additional HIV treatment can be zidovudine (RETROVIR). The one or more additional HIV treatment can be efavirenz (SUSTIVA). The one or more additional HIV treatment can be etravirine (INTELENCE). The one or more additional HIV treatment can be nevirapine (VIRAMUNE). The one or more additional HIV treatment can be rilpivirine (EDURANT) The one or more additional HIV treatment can be atazanavir (REYATAZ). The one or more additional HIV treatment can be ATV/c (EVOTAZ). The one or more additional HIV treatment can be darunavir (PREZISTA). The one or more additional HIV treatment can be DRV/c (PREZCOBIX). The one or more additional HIV treatment can be fosamprenavir (LEXIVA). The one or more additional HIV treatment can be indinavir (CRIXIVAN). The one or more additional HIV treatment can be lopinavir/ ritonavir (KALETRA). The one or more additional HIV treatment can be nelfinavir (VIRACEPT). The one or more additional HIV treatment can be ritonavir (NORVIR). The one or more additional HIV treatment can be saquinavir (INVIRASE). The one or more additional HIV treatment can be tipranavir (APTIVUS). The one or more additional HIV treatment can be dolutegravir (TIVICAY). The one or more additional HIV treatment can be raltegravir (ISENTRESS). The one or more additional HIV treatment can be enfuvirtide (FUZEON). The one or more additional HIV treatment can be maraviroc (SETZENTRY). The one or more additional HIV treatment can be ibalizumab (TROGARZO)

Methods disclosed herein include administering the albuvirtide pharmaceutical formulations disclosed herein in combination with one or more additional HIV treatment. The one or more additional HIV treatment can be selected from the group consisting of: (a) ABC/ZDV/3TC (TRIZIVIR); (b) ABC/3TC (EPZICOM); (c) ABC/3TC/DTG (TRIUMEQ); (d) FTC/EFV/TDF (ATRIPLA); (e) FTC/RPV/TDF (COMPLERA); (f) FTC/TAF (DESCOVY); (g) FTC/EVG/c/TAF (GENVOYA); (h) FTC/RPV/TAF (ODEFSEY); (i) FTC/EVG/c/TDF (STRIBILD); (j) FTC/TDF (TRUVADA); (k) 3TC/AZT (COMBIVIR); (l) BIC/FTC/TAF (BIKTARVY); (m) DTG/RPV (JULUCA); and any combination thereof.

Methods disclosed herein include administering the albuvirtide pharmaceutical formulations disclosed herein in combination with one or more additional HIV treatment. The one or more additional HIV treatment can be ABC/ZDV/3TC (TRIZIVIR). The one or more additional HIV treatment can be ABC/3TC (EPZICOM). The one or more additional HIV treatment can be ABC/3TC/DTG (TRIUMEQ). The one or more additional HIV treatment can be FTC/EFV/TDF (A TRIPLA). The one or more additional HIV treatment can be FTC/RPV/TDF (COMPLERA). The one or more additional HIV treatment can be FTC/TAF (DESCOVY). The one or more additional HIV treatment can be FTC/EVG/c/TAF (GENVOYA). The one or more additional HIV treatment can be FTC/RPV/TAF (ODEFSEY). The one or more additional HIV treatment can be FTC/EVG/c/TDF (STRIBILD). The one or more additional HIV treatment can be FTC/TDF (TRUVADA). The one or more additional HIV treatment can be 3TC/AZT (COMBIVIR). The one or more additional HIV treatment can be BIC/FTC/TAF (BIKTARVY). The one or more additional HIV treatment can be DTG/RPV (JULUCA).

As a person of ordinary skill in the art would understand, different permutations and combinations of the additional HIV treatment disclosed herein can be used in combinations with the albuvirtide pharmaceutical formulations disclosed herein. The choice of the appropriate combination therapies will be at the discretion of the attending physician.

The subject being treated may be a human.

### D. Methods of Manufacture

Albuvirtide can be prepared by standard solid-phase synthesis using Rinkam resin and 9-fluorenylmethoxy carbonyl-protected aminoacid. The side chain of the 13th lysine residue was protected by allyloxycabonyl (Aloc), which allow selective deprotection and addition of the linker molecule of [2-(2-amino)ethoxyl]ethoxy acetic acid and 3-maleimidopropionic acid. The cleaved peptide can be dissolved in TFA and acetonitrile in water and purified by reverse-phase HPLC. The purity and molecular weight were verified by LC-MS.

Provided herein are also methods of preparing liquid compositions of albuvirtide disclosed herein, which can be lyophilized to prepare the stable lyophilized compositions disclosed herein. Specifically, the cleaved peptides can be dissolved in an acidic buffers to achieve desired stability. As such, in some embodiments, provided herein are also methods of preparing stable lyophilized compositions of albuvirtide from a first composition comprising (a) albuvirtide and (b) a buffer used for cleaving albuvirtide from resin (the "cleaving buffer"). In some embodiments, the cleaving buffer can be TFA. In some embodiments, the cleaving buffer can be formic acid. In some embodiments, provided herein are also methods of preparing stable lyophilized compositions of albuvirtide from a first composition comprising (a) albuvirtide and (b) TFA or formic acid. In some embodiments, the first composition is chromatographic eluent. In some embodiments, the first composition further comprises an organic solvent.

In some embodiments, the methods of preparing stable lyophilized compositions of albuvirtide provided herein comprise: (i) replacing the TFA or formic acid in the first composition with an acidic buffer to make a second composition comprising albuvirtide and the acidic buffer, (ii) adjusting the albuvirtide concentration in the second composition to be between 0.1 mg/ml and 300 mg/ml, and (iii) lyophilizing the second composition to make the stable lyophilized albuvirtide composition. In some embodiments, the first composition comprises TFA. In some embodiments, the first composition comprises formic acid. In some embodiments, the acidic buffer comprises an acid selected from the group consisting of phosphoric acid, hydrochloric acid, acetic acid, citric acid, sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid.

The replacement of the cleaving buffer with the acidic buffer can be done using any methods known in the art. In some embodiments, the cleaving buffer in the first composition is replaced by the acidic buffer using gradient elution.

In some embodiments, the second compositions further comprise an organic solvent. The organic solvent can be acetonitrile, ethanol, methanol, isopropanol, or any combination thereof. In some embodiments, the organic solvent is acetonitrile. In some embodiments, the organic solvent is ethanol. In some embodiments, the organic solvent is methanol. In some embodiments, the organic solvent is isopropanol. In some embodiments, the second compositions provided herein are adjusted to comprise two organic solvents. In some embodiments, the second compositions provided herein are adjusted to comprise acetonitrile and ethanol. In some embodiments, the second compositions provided herein are adjusted to comprise acetonitrile and methanol. In some embodiments, the second compositions provided herein are adjusted to comprise acetonitrile and isopropanol. In some embodiments, the second compositions provided herein are adjusted to comprise ethanol and methanol. In some embodiments, the second compositions provided herein are adjusted to comprise ethanol and isopropanol. In some embodiments, the second compositions provided herein are adjusted to comprise methanol and isopropanol. The second compositions provided herein can comprise three organic solvents. In some embodiments, the second compositions provided herein are adjusted to comprise acetonitrile, ethanol, and methanol. In some embodiments, the second compositions provided herein are adjusted to comprise acetonitrile, ethanol, and isopropanol. In some embodiments, the second compositions provided herein are adjusted to comprise acetonitrile, methanol, and isopropanol. In some embodiments, the second compositions provided herein are adjusted to comprise ethanol, methanol, and isopropanol. In some embodiments, the second compositions provided herein are adjusted to comprise acetonitrile, ethanol, methanol, and isopropanol. In addition to those specifically exemplified above, the second compositions provided herein can also include other organic solvent known in the art, alone or in combination with those exemplified herein.

In some embodiments, the method provided herein further comprises removing the organic solvent from the second composition before step (ii). In some embodiments, the removal of the organic solvent can be done using any methods known in the art. In some embodiments, the organic solvent is removed by reduced-pressure evaporation, for example, using rotary evaporators.

The second compositions provided herein can have an organic solvent and water at different ratios. In some embodiments, the second compositions provided herein can have an organic solvent and water at a ratio between 5:95 and 95:5 (v:v), between 10:90 and 90:10 (v:v), between 15:85 and 85:15 (v:v), between 20:80 and 80:20 (v:v), between 20:80 and 45:55 (v:v),between 25:75 and 75:25 (v:v), between 30:70 and 70:30 (v:v), between 30:70 and 40:60 (v:v), between 35:65 and 65:35 (v:v), between 40:60 and 60:40 (v:v), between 45:55 and 55:45 (v:v), at about 35:65 (v:v), or at about 50:50 (v:v). In some embodiments, the second compositions provided herein can have an organic solvent and water at a ratio between 5:95 and 95:5 (v:v). In some embodiments, the second compositions provided herein can have an organic solvent and water at a ratio between 10:90 and 90:10 (v:v). In some embodiments, the second compositions provided herein can have an organic solvent and water at a ratio between 15:85 and 85:15 (v:v). In some embodiments, the second compositions provided herein can have an organic solvent and water at a ratio between 20:80 and 80:20 (v:v). In some embodiments, the second compositions provided herein can have an organic solvent and water at a ratio between 20:80 and 45:55 (v:v). In some embodiments, the second compositions provided herein can have an organic solvent and water at a ratio between 25:75 and 75:25 (v:v). In some embodiments, the second compositions provided herein can have an organic solvent and water at a ratio between 30:70 and 70:30 (v:v). In some embodiments, the second compositions provided herein can have an organic solvent and water at a ratio between 30:70 and 40:60 (v:v). In some embodiments, the second compositions provided herein can have an organic solvent and water at a ratio between 35:65 and 65:35 (v:v). In some embodiments, the second compositions provided herein can have an organic solvent and water at a ratio between 40:60 and 60:40 (v:v). In some embodiments, the second compositions provided herein can have an organic solvent and water at a ratio between 45:55 and 55:45 (v:v). In some embodiments, the second compositions provided herein can have an organic solvent and water at a ratio about 35:65 (v:v). In some embodiments, the second compositions provided herein can have an organic solvent and water at a ratio about 50:50 (v:v).

Methods of preparing stable lyophilized compositions of albuvirtide provided herein comprise (ii) adjusting the albuvirtide concentration in the second composition to be between 5 and 200 mg/mL, between 5 and 175 mg/mL, between 5 and 150 mg/mL, between 5 and 125 mg/mL, or between 5 and 100 mg/mL. In certain embodiments, the second compositions are adjusted to comprise albuvirtide at a concentration of between 10 and 200 mg/mL, between 10 and 175 mg/mL, between 10 and 150 mg/mL, between 10 and 120 mg/mL, or between 10 and 100 mg/mL. In certain embodiments, the second compositions are adjusted to comprise albuvirtide at a concentration of between 15 and 200 mg/mL, between 15 and 175 mg/mL, between 15 and 150 mg/mL, between 15 and 125 mg/mL, or between 15 and 100 mg/mL. In certain embodiments, the second compositions are adjusted to comprise albuvirtide at a concentration of between 20 and 200 mg/mL, between 20 and 175 mg/mL, between 20 and 150 mg/mL, between 20 and 125 mg/mL, or between 20 and 100 mg/mL.

In certain embodiments, the second compositions are adjusted to comprise albuvirtide at a concentration of between 5 and 75 mg/mL, between 10 and 75 mg/mL, between 15 and 75 mg/mL, between 20 and 75 mg/mL, between 5 and 50 mg/mL, between 10 and 50 mg/mL, between 15 and 50 mg/mL, between 20 and 50 mg/mL, between 5 and 45 mg/mL, between 10 and 45 mg/mL, between 15 and 45 mg/mL, between 20 and 45 mg/mL, between 5 and 40 mg/mL, between 10 and 40 mg/mL, between 15 and 40 mg/mL, between 20 and 40 mg/mL, between 5 and 35 mg/mL, between 10 and 35 mg/mL, between 15 and 35 mg/mL, between 20 and 35 mg/mL, between 5 and 30 mg/mL, between 10 and 30 mg/mL, between 15 and 30 mg/mL, or between 20 and 30 mg/mL.

The second compositions provided herein are adjusted to comprise albuvirtide at a concentration of between 5 and 200 mg/mL. In some embodiments, the second compositions provided herein are adjusted to comprise albuvirtide at a concentration of between 5 and 100 mg/mL. In some embodiments, the second compositions provided herein are adjusted to comprise albuvirtide at a concentration of between 5 and 75 mg/mL. In some embodiments, the second compositions provided herein are adjusted to comprise albuvirtide at a concentration of between 5 and 50 mg/mL. In some embodiments, the second compositions provided herein are adjusted to comprise albuvirtide at a concentration of between 5 and 45 mg/mL. In some embodiments, the second compositions provided herein are adjusted to comprise albuvirtide at a concentration of between 5 and 40 mg/mL. In some embodiments, the second compositions provided herein are adjusted to comprise albuvirtide at a concentration of between 5 and 35 mg/mL. In some embodiments, the second compositions provided herein are adjusted to comprise albuvirtide at a concentration of between 5 and 30 mg/mL. In some embodiments, the second compositions provided herein are adjusted to comprise albuvirtide at a concentration of between 5 and 25 mg/mL.

In some embodiments, the second compositions provided herein are adjusted to comprise albuvirtide at a concentration of between 15 and 200 mg/mL. In some embodiments, the second compositions provided herein are adjusted to comprise albuvirtide at a concentration of between 15 and 100 mg/mL. In some embodiments, the second compositions provided herein are adjusted to comprise albuvirtide at a concentration of between 15 and 75 mg/mL. In some embodiments, the second compositions provided herein are adjusted to comprise albuvirtide at a concentration of between 15 and 50 mg/mL. In some embodiments, the second compositions provided herein are adjusted to comprise albuvirtide at a concentration of between 15 and 45 mg/mL. In some embodiments, the second compositions provided herein are adjusted to comprise albuvirtide at a concentration of between 15 and 40 mg/mL. In some embodiments, the second compositions provided herein are adjusted to comprise albuvirtide at a concentration of between 15 and 35 mg/mL. In some embodiments, the second compositions provided herein are adjusted to comprise albuvirtide at a concentration of between 15 and 30 mg/mL. In some embodiments, the second compositions provided herein are adjusted to comprise albuvirtide at a concentration of between 15 and 25 mg/mL.

In certain embodiments, the second compositions provided herein are adjusted to comprise albuvirtide at a concentration of 10 mg/mL, 15 mg/mL, 20 mg/mL, 25 mg/mL, 30 mg/mL, 35 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, 100 mg/mL, 110 mg/mL, 120 mg/mL, 130 mg/mL, 140 mg/mL, 150 mg/mL, 160 mg/mL, 170 mg/mL, 180 mg/mL, or 190 mg/ mL.

In some embodiments, the second compositions provided herein are adjusted to comprise albuvirtide at a concentration of about 10 mg/mL. In some embodiments, the second compositions provided herein are adjusted to comprise albuvirtide at a concentration of about 15 mg/mL. In some embodiments, the second compositions provided herein are adjusted to comprise albuvirtide at a concentration of about 20 mg/mL. In some embodiments, the second compositions provided herein are adjusted to comprise albuvirtide at a concentration of about 25 mg/mL. In some embodiments, the second compositions provided herein are adjusted to comprise albuvirtide at a concentration of about 30 mg/mL. In some embodiments, the second compositions provided herein are adjusted to comprise albuvirtide at a concentration of about 35 mg/mL. In some embodiments, the second compositions provided herein are adjusted to comprise albuvirtide at a concentration of about 40 mg/mL. In some embodiments, the second compositions provided herein are adjusted to comprise albuvirtide at a concentration of about 45 mg/mL. In some embodiments, the second compositions provided herein are adjusted to comprise albuvirtide at a concentration of about 50 mg/mL. In some embodiments, the second compositions provided herein are adjusted to comprise albuvirtide at a concentration of about 75 mg/mL. In some embodiments, the second compositions provided herein are adjusted to comprise albuvirtide at a concentration of about 100 mg/mL. In some embodiments, the second compositions provided herein are adjusted to comprise albuvirtide at a concentration of about 125 mg/mL. In some embodiments, the second compositions provided herein are adjusted to comprise albuvirtide at a concentration of about 150 mg/mL. In some embodiments, the second compositions provided herein are adjusted to comprise albuvirtide at a concentration of about 175 mg/mL.

The methods provided herein include adjusting the pH of the second compositions. The second compositions provided herein are adjusted to have a pH between 1.0 and 3.5. In some embodiments, the second compositions provided herein are adjusted to have a pH between 1.0 and 3.0. In some embodiments, the second compositions provided herein are adjusted to have a pH between 1.0 and 2.5. In some embodiments, the second compositions provided herein are adjusted to have a pH between 1.5 and 3.5. In some embodiments, the second compositions provided herein are adjusted to have a pH between 1.5 and 3.0. In some embodiments, the second compositions provided herein are adjusted to have a pH between 1.5 and 2.5In some embodiments, the second compositions provided herein are adjusted to have a pH between 2.0 and 3.5. In some embodiments, the second compositions provided herein are adjusted to have a pH between 2.0 and 3.0. In some embodiments, the second compositions provided herein are adjusted to have a pH between 2.0 and 2.5.

In some embodiments, the second compositions provided herein are adjusted to have a pH of 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, or 3.4. In some embodiments, the second compositions provided herein are adjusted to have a pH of about 1.5. In some embodiments, the second compositions provided herein are adjusted to have a pH of about 2.0. In some embodiments, the second compositions provided herein are adjusted to have a pH of about 2.5. In some embodiments, the second compositions provided herein are adjusted to have a pH of about 3.0.

The measurements of the pH can be taken at the temperature of 4 °C. The measurements of the pH can be taken at room temperature. The measurements of the pH can be taken at about 22 °C.

The methods of preparing a stable lyophilized composition of albuvirtide from a first composition comprising (a) albuvirtide and (b) TFA or formic acid, comprise:
(i) replacing the TFA or formic acid in the first composition with an acidic buffer to make a second composition comprising albuvirtide and the acidic buffer, wherein the acidic buffer comprises an acid selected from the group consisting of phosphoric acid, hydrochloric acid, acetic acid, citric acid, and sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid, and
(ii) lyophilizing the second composition to make the stable lyophilized albuvirtide composition, wherein the second composition has a pH between 1.0 and 3.5, and the method further comprises adjusting the albuvirtide concentration in the second composition to be between 5 mg/ml and 200 mg/ml before lyophilization.

In some embodiments, the acidic buffer can comprise a phosphoric acid and a salt thereof. In some embodiments, the acidic buffer can comprise a hydrochloric acid and a salt thereof. In some embodiments, the acidic buffer can comprise an acetic acid and a salt thereof. In some embodiments, the acidic buffer can comprise a citric acid and a salt thereof. In some embodiments, the acidic buffer can comprise a sulfuric acid and a salt thereof. In some embodiments, the acidic buffer can comprise a tartaric acid and a salt thereof. In some embodiments, the acidic buffer can comprise a lactic acid and a salt thereof. In some embodiments, the acidic buffer can comprise a succinic acid and a salt thereof. In some embodiments, the acidic buffer can comprise an ascorbic acid and a salt thereof. In some embodiments, the acidic buffer can comprise an aspartic acid and a salt thereof. In some embodiments, the acidic buffer can comprise a glutamic acid and a salt thereof. In some embodiments, the acidic buffer can comprise a propanoic acid and a salt thereof. In some embodiments, the acidic buffer can comprise a propanedioic acid and a salt thereof. In some embodiments, the acidic buffer can comprise a butyric acid and a salt thereof. In some embodiments, the acidic buffer can comprise a maleic acid and a salt thereof. In some embodiments, the acidic buffer can comprise a fumaric acid and a salt thereof. In some embodiments, the acidic buffer can comprise a malic acid and a salt thereof. In some embodiments, the acidic buffer can comprise an oxalic acid and a salt thereof.

In some embodiments, the salt form of the acid comprises a sodium salt of the acid. In some embodiments, the salt form of the acid comprises a potassium salt of the acid. In some embodiments, the salt form of the acid comprises an ammonium salt of the acid. In some embodiments, the salt form of the acid comprises any combination of a sodium salt , a potassium salt, and an ammonium salt of the acid.

In some embodiments, the acidic buffer used in the methods disclosed herein can comprise a phosphoric acid and sodium phosphate. In some embodiments, the acidic buffer can comprise a hydrochloric acid and sodium chloride. In some embodiments, the acidic buffer can comprise an acetic acid and sodium acetate. In some embodiments, the acidic buffer can comprise a citric acid and sodium citrate. In some embodiments, the acidic buffer can comprise a sulfuric acid and sodium sulfate. In some embodiments, the acidic buffer can comprise a tartaric acid and sodium tartrate. In some embodiments, the acidic buffer can comprise a lactic acid and sodium lactate. In some embodiments, the acidic buffer can comprise a succinic acid and sodium succinate. In some embodiments, the acidic buffer can comprise an ascorbic acid and sodium ascorbate. In some embodiments, the acidic buffer can comprise an aspartic acid and sodium aspartate. In some embodiments, the acidic buffer can comprise a glutamic acid and sodium glutamate. In some embodiments, the acidic buffer can comprise a propanoic acid and sodium propanoate. In some embodiments, the acidic buffer can comprise a propanedioic acid and sodium propanedioate. In some embodiments, the acidic buffer can comprise a butyric acid and sodium butyrate. In some embodiments, the acidic buffer can comprise a maleic acid and sodium maleate. In some embodiments, the acidic buffer can comprise a fumaric acid and sodium fumarate. In some embodiments, the acidic buffer can comprise a malic acid and sodium malate. In some embodiments, the acidic buffer can comprise an oxalic acid and sodium oxalate.

In some embodiments, the acidic buffer used in the methods disclosed herein can comprise a phosphoric acid and potassium phosphate. In some embodiments, the acidic buffer can comprise a hydrochloric acid and potassium chloride. In some embodiments, the acidic buffer can comprise an acetic acid and potassium acetate. In some embodiments, the acidic buffer can comprise a citric acid and potassium citrate. In some embodiments, the acidic buffer can comprise a sulfuric acid and potassium sulfate. In some embodiments, the acidic buffer can comprise a tartaric acid and potassium tartrate. In some embodiments, the acidic buffer can comprise a lactic acid and potassium lactate. In some embodiments, the acidic buffer can comprise a succinic acid and potassium succinate. In some embodiments, the acidic buffer can comprise an ascorbic acid and potassium ascorbate. In some embodiments, the acidic buffer can comprise an aspartic acid and potassium aspartate. In some embodiments, the acidic buffer can comprise a glutamic acid and potassium glutamate. In some embodiments, the acidic buffer can comprise a propanoic acid and potassium propanoate. In some embodiments, the acidic buffer can comprise a propanedioic acid and potassium propanedioate. In some embodiments, the acidic buffer can comprise a butyric acid and potassium butyrate. In some embodiments, the acidic buffer can comprise a maleic acid and potassium maleate. In some embodiments, the acidic buffer can comprise a fumaric acid and potassium fumarate. In some embodiments, the acidic buffer can comprise a malic acid and potassium malate. In some embodiments, the acidic buffer can comprise an oxalic acid and potassium oxalate.

In some embodiments, the acidic buffer used in the methods disclosed herein can comprise a phosphoric acid and ammonium phosphate. In some embodiments, the acidic buffer can comprise a hydrochloric acid and ammonium chloride. In some embodiments, the acidic buffer can comprise an acetic acid and ammonium acetate. In some embodiments, the acidic buffer can comprise a citric acid and ammonium citrate. In some embodiments, the acidic buffer can comprise a sulfuric acid and ammonium sulfate. In some embodiments, the acidic buffer can comprise a tartaric acid and ammonium tartrate. In some embodiments, the acidic buffer can comprise a lactic acid and ammonium lactate. In some embodiments, the acidic buffer can comprise a succinic acid and ammonium succinate. In some embodiments, the acidic buffer can comprise an ascorbic acid and ammonium ascorbate. In some embodiments, the acidic buffer can comprise an aspartic acid and ammonium aspartate. In some embodiments, the acidic buffer can comprise a glutamic acid and ammonium glutamate. In some embodiments, the acidic buffer can comprise a propanoic acid and ammonium propanoate. In some embodiments, the acidic buffer can comprise a propanedioic acid and ammonium propanedioate. In some embodiments, the acidic buffer can comprise a butyric acid and ammonium butyrate. In some embodiments, the acidic buffer can comprise a maleic acid and ammonium maleate. In some embodiments, the acidic buffer can comprise a fumaric acid and ammonium fumarate. In some embodiments, the acidic buffer can comprise a malic acid and ammonium malate. In some embodiments, the acidic buffer can comprise an oxalic acid and ammonium oxalate.

In some embodiments, the second compositions comprise albuvirtide and two acidic buffers, wherein the acidic buffers are selected form the group consisting of a phosphoric acid, hydrochloric acid, acetic acid, citric acid, sulfuric acid, tartaric acid, lactic acid, succinic acid, ascorbic acid, aspartic acid, glutamic acid, propanoic acid, propanedioic acid, butyric acid, maleic acid, fumaric acid, malic acid, oxalic acid, and salts thereof. In some embodiments, the liquid second compositions provided herein are adjusted to comprise albuvirtide and three acidic buffers, wherein the acidic buffers are selected form the group consisting of a phosphoric acid, hydrochloric acid, acetic acid, citric acid, sulfuric acid, tartaric acid, lactic acid, succinic acid, ascorbic acid, aspartic acid, glutamic acid, propanoic acid, propanedioic acid, butyric acid, maleic acid, fumaric acid, malic acid, oxalic acid, and salts thereof. In some embodiments, the liquid second compositions provided herein are adjusted to comprise albuvirtide and four acidic buffers, wherein the acidic buffers are selected form the group consisting of a phosphoric acid, hydrochloric acid, acetic acid, citric acid, sulfuric acid, tartaric acid, lactic acid, succinic acid, ascorbic acid, aspartic acid, glutamic acid, propanoic acid, propanedioic acid, butyric acid, maleic acid, fumaric acid, malic acid, oxalic acid, and salts thereof.

In some embodiments, the second compositions provided herein can comprise albuvirtide and an acidic buffer, wherein the acidic buffer has a concentration between 5 and 200 mM. The buffer concentration refers to the total concertation of the acid and the salt thereof. For example, if the buffer of a second composition consists of 5 mM phosphoric acid and 5 mM sodium phosphate, its buffer concentration is 10 mM.

In some embodiments, the second compositions provided herein can comprise an acidic buffer at a concentration between 5 and 175 mM. In some embodiments, the second compositions provided herein can comprise an acidic buffer at a concentration between 5 and 150 mM. In some embodiments, the second compositions provided herein can comprise an acidic buffer at a concentration between 5 and 125 mM. In some embodiments, the second compositions provided herein can comprise an acidic buffer at a concentration between 5 and 100 mM. In some embodiments, the second compositions provided herein can comprise an acidic buffer at a concentration between 10 and 200 mM. In some embodiments, the second compositions provided herein can comprise an acidic buffer at a concentration between 10 and 175 mM. In some embodiments, the second compositions provided herein can comprise an acidic buffer at a concentration between 10 and 150 mM. In some embodiments, the second compositions provided herein can comprise an acidic buffer at a concentration between 10 and 125 mM. In some embodiments, the second compositions provided herein can comprise an acidic buffer at a concentration between 10 and 100 mM.

In some embodiments, the second compositions provided herein can comprise an acidic buffer at a concentration between 5 and 75 mM. In some embodiments, the second compositions provided herein can comprise an acidic buffer at a concentration between 5 and 50 mM. In some embodiments, the second compositions provided herein can comprise an acidic buffer at a concentration between 5 and 45 mM. In some embodiments, the second compositions provided herein can comprise an acidic buffer at a concentration between 5 and 40 mM. some embodiments, the second compositions provided herein can comprise an acidic buffer at a concentration between 5 and 35 mM. In some embodiments, the second compositions provided herein can comprise an acidic buffer at a concentration between 5 and 30 mM. In some embodiments, the second compositions provided herein can comprise an acidic buffer at a concentration between 5 and 25 mM. In some embodiments, the second compositions provided herein can comprise an acidic buffer at a concentration between 5 and 20 mM.

In some embodiments, the second compositions provided herein can comprise an acidic buffer at a concentration between 10 and 75 mM. In some embodiments, the second compositions provided herein can comprise an acidic buffer at a concentration between 10 and 50 mM. In some embodiments, the second compositions provided herein can comprise an acidic buffer at a concentration between 10 and 45 mM. In some embodiments, the second compositions provided herein can comprise an acidic buffer at a concentration between 10 and 40 mM. some embodiments, the second compositions provided herein can comprise an acidic buffer at a concentration between 10 and 35 mM. In some embodiments, the second compositions provided herein can comprise an acidic buffer at a concentration between 10 and 30 mM. In some embodiments, the second compositions provided herein can comprise an acidic buffer at a concentration between 10 and 25 mM. In some embodiments, the second compositions provided herein can comprise an acidic buffer at a concentration between 10 and 20 mM.

In some embodiments, the second compositions provided herein can comprise an acidic buffer at a concentration between 15 and 75 mM. In some embodiments, the second compositions provided herein can comprise an acidic buffer at a concentration between 15 and 50 mM. In some embodiments, the second compositions provided herein can comprise an acidic buffer at a concentration between 15 and 45 mM. In some embodiments, the second compositions provided herein can comprise an acidic buffer at a concentration between 15 and 40 mM. some embodiments, the second compositions provided herein can comprise an acidic buffer at a concentration between 15 and 35 mM. In some embodiments, the second compositions provided herein can comprise an acidic buffer at a concentration between 15 and 30 mM. In some embodiments, the second compositions provided herein can comprise an acidic buffer at a concentration between 15 and 25 mM. In some embodiments, the second compositions provided herein can comprise an acidic buffer at a concentration between 15 and 20 mM.

In some embodiments, the second compositions provided herein can comprise an acidic buffer at a concentration of about 5 mM, 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 100 mM, 110 mM, 120 mM, 130 mM, 140 mM, 150 mM, 160 mM, 170 mM, 180 mM, 190 mM, 195 mM, or 200 mM. In some embodiments, the second compositions provided herein can comprise an acidic buffer at a concentration of about 5 mM. In some embodiments, the second compositions provided herein can comprise an acidic buffer at a concentration of about 10 mM. In some embodiments, the second compositions provided herein can comprise an acidic buffer at a concentration of about 20 mM. In some embodiments, the second compositions provided herein can comprise an acidic buffer at a concentration of about 30 mM. In some embodiments, the second compositions provided herein can comprise an acidic buffer at a concentration of about 40 mM. In some embodiments, the second compositions provided herein can comprise an acidic buffer at a concentration of about 50 mM. In some embodiments, the second compositions provided herein can comprise an acidic buffer at a concentration of about 75 mM. In some embodiments, the second compositions provided herein can comprise an acidic buffer at a concentration of about 100 mM. In some embodiments, the second compositions provided herein can comprise an acidic buffer at a concentration of about 125 mM. In some embodiments, the second compositions provided herein can comprise an acidic buffer at a concentration of about 150 mM. In some embodiments, the second compositions provided herein can comprise an acidic buffer at a concentration of about 175 mM. In some embodiments, the second compositions provided herein can comprise an acidic buffer at a concentration of about 200 mM.

In some embodiments, the second compositions provided herein are adjusted to comprise an acidic buffer having the acid and the salt form of the acid at different ratios. In some embodiments, the acidic buffer can have the acid and salt form of the acid at a ratio between 100:0 (molar) and 5:95 (molar), namely, of 100 mM acidic buffer, there are 100 mM acid and 0 mM salt thereof, 5 mM acid and 95 mM salt thereof, or anything in between. In some embodiments, the acidic buffer can have the acid and salt form of the acid at a ratio between 95:5 (molar) and 5:95 (molar), between 90:10 (molar) and 10:90 (molar), between 85:15 (molar) and 15:85 (molar), between 80:20 (molar) and 20:80 (molar), between 75:25 (molar) and 25:75 (molar), between 70:30 (molar) and 30:70 (molar), between 65:35 (molar) and 35:65 (molar), between 60:40 (molar) and 40:60 (molar), between 55:45 (molar) and 45:55 (molar), or at about 50:50 (molar). In some embodiments, the second compositions provided herein can have acid and salt form of the acid at a ratio between 100:0 (molar) and 5:95 (molar). In some embodiments, the second compositions provided herein can have the acid and salt form of the acid at a ratio between 95:5 (molar) and 5:95 (molar). In some embodiments, the second compositions provided herein can have the acid and salt form of the acid at a ratio between 90:10 (molar) and 10:90 (molar). In some embodiments, the second compositions provided herein can have the acid and salt form of the acid at a ratio between 85:15 (molar) and 15:85 (molar). In some embodiments, the second compositions provided herein can have the acid and salt form of the acid at a ratio between 80:20 (molar) and 20:80 (molar). In some embodiments, the second compositions provided herein can have the acid and salt form of the acid at a ratio between 75:25 (molar) and 25:75 (molar). In some embodiments, the second compositions provided herein can have the acid and salt form of the acid at a ratio between 70:30 (molar) and 30:70 (molar). In some embodiments, the second compositions provided herein can have the acid and salt form of the acid at a ratio between 65:35 (molar) and 35:65 (molar). In some embodiments, the second compositions provided herein can have the acid and salt form of the acid at a ratio between 60:40 (molar) and 40:60 (molar). In some embodiments, the second compositions provided herein can have the acid and salt form of the acid at a ratio between 55:45 (molar) and 45:55 (molar).

In some embodiments, the second compositions provided herein can have the acid and salt form of the acid at a ratio of about 90:10 (molar). In some embodiments, the second compositions provided herein can have the acid and salt form of the acid at a ratio of about 80:20 (molar). In some embodiments, the second compositions provided herein can have the acid and salt form of the acid at a ratio of about 70:30 (molar). In some embodiments, the second compositions provided herein can have the acid and salt form of the acid at a ratio of about 60:40 (molar). In some embodiments, the second compositions provided herein can have the acid and salt form of the acid at a ratio of about 50:50 (molar).

The methods of preparing a stable lyophilized composition of albuvirtide from a first composition comprising (a) albuvirtide and (b) TFA or formic acid, comprise:
(i) replacing the TFA or formic acid in the first composition with an acidic buffer to make a second composition comprising albuvirtide and the acidic buffer, wherein the acidic buffer comprises an acid selected from the group consisting of phosphoric acid, hydrochloric acid, acetic acid, citric acid, and sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid, and
(ii) lyophilizing the second composition to make the stable lyophilized albuvirtide composition, wherein the second composition has a pH between 1.0 and 3.5, and the method further comprises adjusting the albuvirtide concentration in the second composition to be between 5 mg/ml and 200 mg/ml before lyophilization.

In some embodiments, the second compositions comprise albuvirtide and a phosphate buffer, wherein the second compositions have a pH between 1.0 and 3.5. The phosphate buffer can comprise a phosphoric acid and a salt thereof. In some embodiments, the phosphate buffer can comprise a phosphoric acid and sodium phosphate. In some embodiments, the phosphate buffer can comprise a phosphoric acid and sodium dihydrogen phosphate. In some embodiments, the phosphate buffer can comprise a phosphoric acid and potassium phosphate. In some embodiments, the phosphate buffer can comprise a phosphoric acid and potassium dihydrogen phosphate. In some embodiments, the phosphate buffer can comprise a phosphoric acid and ammonium phosphate. In some embodiments, the phosphate buffer can comprise a phosphoric acid and ammonium dihydrogen phosphate. In some embodiments, the phosphate buffer can comprise a phosphoric acid and any combination of the salts above.

In some embodiments, the second compositions provided herein can comprise albuvirtide and a phosphate buffer, wherein the phosphate buffer has a concentration between 5 and 200 mM. The buffer concentration refers to the total concertation of the phosphoric acid and the salt thereof. For example, if the buffer of a second composition consists of 5 mM phosphoric acid and 5 mM sodium dihydrogen phosphate, its buffer concentration is 10 mM.

In some embodiments, the second compositions provided herein can comprise a phosphate buffer at a concentration between 5 and 175 mM. In some embodiments, the second compositions provided herein can comprise a phosphate buffer at a concentration between 5 and 150 mM. In some embodiments, the second compositions provided herein can comprise a phosphate buffer at a concentration between 5 and 125 mM. In some embodiments, the second compositions provided herein can comprise a phosphate buffer at a concentration between 5 and 100 mM. In some embodiments, the second compositions provided herein can comprise a phosphate buffer at a concentration between 10 and 200 mM. In some embodiments, the second compositions provided herein can comprise a phosphate buffer at a concentration between 10 and 175 mM. In some embodiments, the second compositions provided herein can comprise a phosphate buffer at a concentration between 10 and 150 mM. In some embodiments, the second compositions provided herein can comprise a phosphate buffer at a concentration between 10 and 125 mM. In some embodiments, the second compositions provided herein can comprise a phosphate buffer at a concentration between 10 and 100 mM.

In some embodiments, the second compositions provided herein can comprise a phosphate buffer at a concentration between 5 and 75 mM. In some embodiments, the second compositions provided herein can comprise a phosphate buffer at a concentration between 5 and 50 mM. In some embodiments, the second compositions provided herein can comprise a phosphate buffer at a concentration between 5 and 45 mM. In some embodiments, the second compositions provided herein can comprise a phosphate buffer at a concentration between 5 and 40 mM. some embodiments, the second compositions provided herein can comprise a phosphate buffer at a concentration between 5 and 35 mM. In some embodiments, the second compositions provided herein can comprise a phosphate buffer at a concentration between 5 and 30 mM. In some embodiments, the second compositions provided herein can comprise a phosphate buffer at a concentration between 5 and 25 mM. In some embodiments, the second compositions provided herein can comprise a phosphate buffer at a concentration between 5 and 20 mM.

In some embodiments, the second compositions provided herein can comprise a phosphate buffer at a concentration between 10 and 75 mM. In some embodiments, the second compositions provided herein can comprise a phosphate buffer at a concentration between 10 and 50 mM. In some embodiments, the second compositions provided herein can comprise a phosphate buffer at a concentration between 10 and 45 mM. In some embodiments, the second compositions provided herein can comprise a phosphate buffer at a concentration between 10 and 40 mM. some embodiments, the second compositions provided herein can comprise a phosphate buffer at a concentration between 10 and 35 mM. In some embodiments, the second compositions provided herein can comprise a phosphate buffer at a concentration between 10 and 30 mM. In some embodiments, the second compositions provided herein can comprise a phosphate buffer at a concentration between 10 and 25 mM. In some embodiments, the second compositions provided herein can comprise a phosphate buffer at a concentration between 10 and 20 mM.

In some embodiments, the second compositions provided herein can comprise a phosphate buffer at a concentration between 15 and 75 mM. In some embodiments, the second compositions provided herein can comprise a phosphate buffer at a concentration between 15 and 50 mM. In some embodiments, the second compositions provided herein can comprise a phosphate buffer at a concentration between 15 and 45 mM. In some embodiments, the second compositions provided herein can comprise a phosphate buffer at a concentration between 15 and 40 mM. some embodiments, the second compositions provided herein can comprise a phosphate buffer at a concentration between 15 and 35 mM. In some embodiments, the second compositions provided herein can comprise a phosphate buffer at a concentration between 15 and 30 mM. In some embodiments, the second compositions provided herein can comprise a phosphate buffer at a concentration between 15 and 25 mM. In some embodiments, the second compositions provided herein can comprise a phosphate buffer at a concentration between 15 and 20 mM.

In some embodiments, the second compositions provided herein can comprise a phosphate buffer at a concentration of about 5 mM, 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 100 mM, 110 mM, 120 mM, 130 mM, 140 mM, 150 mM, 160 mM, 170 mM, 180 mM, 190 mM, or 195 mM. In some embodiments, the second compositions provided herein can comprise a phosphate buffer at a concentration of about 5 mM. In some embodiments, the second compositions provided herein can comprise a phosphate buffer at a concentration of about 10 mM. In some embodiments, the second compositions provided herein can comprise a phosphate buffer at a concentration of about 20 mM. In some embodiments, the second compositions provided herein can comprise a phosphate buffer at a concentration of about 30 mM. In some embodiments, the second compositions provided herein can comprise a phosphate buffer at a concentration of about 40 mM. In some embodiments, the second compositions provided herein can comprise a phosphate buffer at a concentration of about 50 mM. In some embodiments, the second compositions provided herein can comprise a phosphate buffer at a concentration of about 75 mM. In some embodiments, the second compositions provided herein can comprise a phosphate buffer at a concentration of about 100 mM. In some embodiments, the second compositions provided herein can comprise a phosphate buffer at a concentration of about 125 mM. In some embodiments, the second compositions provided herein can comprise a phosphate buffer at a concentration of about 150 mM. In some embodiments, the second compositions provided herein can comprise a phosphate buffer at a concentration of about 175 mM.

In some embodiments, the second compositions provided herein are adjusted to comprise a phosphate buffer having the phosphoric acid and the salt form of the phosphoric acid at different ratios. In some embodiments, the salt form of the phosphoric acid is sodium phosphate. In some embodiments, the salt form of the phosphoric acid is sodium dihydrogen phosphate. In some embodiments, the salt form of the phosphoric acid is potassium phosphate. In some embodiments, the salt form of the phosphoric acid is potassium dihydrogen phosphate. In some embodiments, the salt form of the phosphoric acid is ammonium phosphate. In some embodiments, the salt form of the phosphoric acid is ammonium dihydrogen phosphate. In some embodiments, the salt form of the phosphoric acid comprises any combination of the salts above.

In some embodiments, the phosphate buffer in the second compositions can have the phosphoric acid and salt form of the phosphoric acid at a ratio between 100:0 (molar) and 5:95 (molar), between 95:5 (molar) and 5:95 (molar), between 90:10 (molar) and 10:90 (molar), between 85:15 (molar) and 15:85 (molar), between 80:20 (molar) and 20:80 (molar), between 75:25 (molar) and 25:75 (molar), between 70:30 (molar) and 30:70 (molar), between 65:35 (molar) and 35:65 (molar), between 60:40 (molar) and 40:60 (molar), between 55:45 (molar) and 45:55 (molar), or at about 50:50 (molar). In some embodiments, the second compositions provided herein can have the phosphoric acid and salt form of the phosphoric acid at a ratio between 100:0 (molar) and 5:95 (molar). In some embodiments, the second compositions provided herein can have the phosphoric acid and salt form of the phosphoric acid at a ratio between 95:5 (molar) and 5:95 (molar). In some embodiments, the second compositions provided herein can have the phosphoric acid and salt form of the phosphoric acid at a ratio between 90:10 (molar) and 10:90 (molar). In some embodiments, the second compositions provided herein can have the phosphoric acid and salt form of the phosphoric acid at a ratio between 85:15 (molar) and 15:85 (molar). In some embodiments, the second compositions provided herein can have the phosphoric acid and salt form of the phosphoric acid at a ratio between 80:20 (molar) and 20:80 (molar). In some embodiments, the second compositions provided herein can have the phosphoric acid and salt form of the phosphoric acid at a ratio between 75:25 (molar) and 25:75 (molar). In some embodiments, the second compositions provided herein can have the phosphoric acid and salt form of the phosphoric acid at a ratio between 70:30 (molar) and 30:70 (molar). In some embodiments, the second compositions provided herein can have the phosphoric acid and salt form of the phosphoric acid at a ratio between 65:35 (molar) and 35:65 (molar). In some embodiments, the second compositions provided herein can have the phosphoric acid and salt form of the phosphoric acid at a ratio between 60:40 (molar) and 40:60 (molar). In some embodiments, the second compositions provided herein can have the phosphoric acid and salt form of the phosphoric acid at a ratio between 55:45 (molar) and 45:55 (molar).

In some embodiments, the second compositions provided herein can have the phosphoric acid and salt form of the phosphoric acid at a ratio of about 90:10 (molar). In some embodiments, the second compositions provided herein can have the phosphoric acid and salt form of the phosphoric acid at a ratio of about 80:20 (molar). In some embodiments, the second compositions provided herein can have the phosphoric acid and salt form of the phosphoric acid at a ratio of about 70:30 (molar). In some embodiments, the second compositions provided herein can have the phosphoric acid and salt form of the phosphoric acid at a ratio of about 60:40 (molar). In some embodiments, the second compositions provided herein can have the phosphoric acid and salt form of the phosphoric acid at a ratio of about 50:50 (molar).

In some embodiments, the methods provided herein include replacing the TFA or formic acid in the first compositions with a phosphate buffer that comprises a phosphoric acid and a salt thereof to make a second composition. In some embodiments, the second compositions provided herein further comprise an organic solvent. The organic solvent can be acetonitrile, ethanol, methanol, isopropanol, or any combination thereof. In some embodiments, the organic solvent is acetonitrile. In some embodiments, the organic solvent is ethanol. In some embodiments, the organic solvent is methanol. In some embodiments, the organic solvent is isopropanol. In some embodiments, the second compositions provided herein are adjusted to comprise two organic solvents. In some embodiments, the second compositions provided herein are adjusted to comprise acetonitrile and ethanol. In some embodiments, the second compositions provided herein are adjusted to comprise acetonitrile and methanol. In some embodiments, the second compositions provided herein are adjusted to comprise acetonitrile and isopropanol. In some embodiments, the second compositions provided herein are adjusted to comprise ethanol and methanol. In some embodiments, the second compositions provided herein are adjusted to comprise ethanol and isopropanol. In some embodiments, the second compositions provided herein are adjusted to comprise methanol and isopropanol. The second compositions provided herein can comprise three organic solvents. In some embodiments, the second compositions provided herein are adjusted to comprise acetonitrile, ethanol, and methanol. In some embodiments, the second compositions provided herein are adjusted to comprise acetonitrile, ethanol, and isopropanol. In some embodiments, the second compositions provided herein are adjusted to comprise acetonitrile, methanol, and isopropanol. In some embodiments, the second compositions provided herein are adjusted to comprise ethanol, methanol, and isopropanol. In some embodiments, the second compositions provided herein are adjusted to comprise acetonitrile, ethanol, methanol, and isopropanol. In addition to those specifically exemplified above, the second compositions provided herein can also include other organic solvent known in the art, alone or in combination with those exemplified herein.

In some embodiments, method provided herein further comprises removing the organic solvent from the second composition before step (i). In some embodiments, the organic solvent is removed by reduced-pressure evaporation. In some embodiments, the organic solvent is removed using rotary evaporator. The second compositions provided herein can have an organic solvent and water at different ratios. In some embodiments, the second compositions provided herein can have an organic solvent and water at a ratio between 5:95 (v:v) and 95:5 (v:v), between 10:90 (v:v) and 90:10 (v:v), between 15:85 (v:v) and 85:15 (v:v), between 20:80 (v:v) and 80:20 (v:v), between 20:80 (v:v) and 45:55 (v:v), between 25:75 (v:v) and 75:25 (v:v), between 30:70 (v:v) and 70:30 (v:v), between 30:70 (v:v) and 40:60 (v:v), between 35:65 (v:v) and 65:35 (v:v), between 40:60 (v:v) and 60:40 (v:v), between 45:55 (v:v) and 55:45 (v:v), at about 35:65, or at about 50:50 (v:v). In some embodiments, the second compositions provided herein can have an organic solvent and water at a ratio between 5:95 (v:v) and 95:5 (v:v). In some embodiments, the second compositions provided herein can have an organic solvent and water at a ratio between 10:90 (v:v) and 90:10 (v:v). In some embodiments, the second compositions provided herein can have an organic solvent and water at a ratio between 15:85 (v:v) and 85:15 (v:v). In some embodiments, the second compositions provided herein can have an organic solvent and water at a ratio between 20:80 (v:v) and 80:20 (v:v). In some embodiments, the second compositions provided herein can have an organic solvent and water at a ratio between 20:80 (v:v) and 45:55 (v:v).In some embodiments, the second compositions provided herein can have an organic solvent and water at a ratio between 25:75 (v:v) and 75:25 (v:v). In some embodiments, the second compositions provided herein can have an organic solvent and water at a ratio between 30:70 (v:v) and 70:30 (v:v). In some embodiments, the second compositions provided herein can have an organic solvent and water at a ratio between 30:70 (v:v) and 40:60 (v:v). In some embodiments, the second compositions provided herein can have an organic solvent and water at a ratio between 35:65 (v:v) and 65:35 (v:v). In some embodiments, the second compositions provided herein can have an organic solvent and water at a ratio between 40:60 (v:v) and 60:40 (v:v). In some embodiments, the second compositions provided herein can have an organic solvent and water at a ratio between 45:55 (v:v) and 55:45 (v:v). In some embodiments, the second compositions provided herein can have an organic solvent and water at a ratio about 35:65 (v:v). In some embodiments, the second compositions provided herein can have an organic solvent and water at a ratio about 50:50 (v:v).

In some embodiments, the methods provided herein include replacing the TFA or formic acid in the first compositions with a phosphate buffer that comprises a phosphoric acid and a salt thereof to make a second composition. The second compositions provided herein can be adjusted to have a pH between 1.0 and 3.5. In some embodiments, the second compositions provided herein can be adjusted to have a pH between 1.0 and 3.0. In some embodiments, the second compositions provided herein can be adjusted to have a pH between 1.0 and 2.5. In some embodiments, the second compositions provided herein can be adjusted to have a pH between 1.5 and 3.5. In some embodiments, the second compositions provided herein can be adjusted to have a pH between 1.5 and 3.0. In some embodiments, the second compositions provided herein can be adjusted to have a pH between 1.5 and 2.5. In some embodiments, the second compositions provided herein can be adjusted to have a pH between 2.0 and 3.5. In some embodiments, the second compositions provided herein can be adjusted to have a pH between 2.0 and 3.0. In some embodiments, the second compositions provided herein can be adjusted to have a pH between 2.0 and 2.5.

In some embodiments, the second compositions provided herein can be adjusted to have a pH of about 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5. In some embodiments, the second compositions provided herein can be adjusted to have a pH of about 1.0. In some embodiments, the second compositions provided herein can be adjusted to have a pH of about 1.5. In some embodiments, the second compositions provided herein can be adjusted to have a pH of about 2.0. In some embodiments, the second compositions provided herein can be adjusted to have a pH of about 2.5. In some embodiments, the second compositions provided herein can be adjusted to have a pH of about 3.0. In some embodiments, the second compositions provided herein can be adjusted to have a pH of about 3.5.

The measurements of the pH can be taken at the temperature of 4 °C. The measurements of the pH can be taken at room temperature. The measurements of the pH can be taken at about 22 °C.

Provided herein are methods of preparing a stable lyophilized composition of albuvirtide from a first composition comprising (a) albuvirtide and (b) TFA or formic acid, comprising: (i) replacing the TFA or formic acid in the first composition with an acidic buffer to make a second composition comprising albuvirtide and the acidic buffer, wherein the acidic buffer comprises an acid selected from the group consisting of phosphoric acid, hydrochloric acid, acetic acid, citric acid, sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid, (ii) adjusting the albuvirtide concentration in the second composition to be between 5 mg/ml and 200 mg/ml and the pH of the second composition to be between 1.0 and 3.5, and (iii) lyophilizing the second composition to make the stable lyophilized albuvirtide composition. In some embodiments, the second composition further comprise acetonitrile.

In some embodiments, provided herein are methods of preparing a stable lyophilized composition of albuvirtide from a first composition comprising (a) albuvirtide and (b) TFA or formic acid, comprising: (i) replacing the TFA or formic acid in the first composition with a phosphate buffer to make a second composition comprising albuvirtide and the phosphate buffer, (ii) adjusting the albuvirtide concentration in the second composition to be between 5 mg/ml and 200 mg/ml and the pH of the second composition to be between 1.0 and 3.5 and (iii) lyophilizing the second composition to make the stable lyophilized albuvirtide composition.

In some embodiments, the second composition further comprise acetonitrile.

In some embodiments, the phosphate buffer is present at a concentration between 10 mM and 200 mM.

In some embodiments, the phosphate buffer comprises a phosphoric acid and sodium dihydrogen phosphate at a ratio between 80:20 to 20:80.

In some embodiments, provided herein are methods of preparing a stable lyophilized composition of albuvirtide from a first composition comprising (a) albuvirtide and (b) TFA or formic acid, comprising: (i) replacing the TFA or formic acid in the first composition with a phosphate buffer to make a second composition comprising albuvirtide and the phosphate buffer, (ii) adjusting the albuvirtide concentration in the second composition to be between 10 mg/ml and 50 mg/ml and the pH of the second composition to be between 1.0 and 3.5, and (iii) lyophilizing the second composition to make the stable lyophilized albuvirtide composition.

In some embodiments, the second composition further comprise acetonitrile.

In some embodiments, the phosphate buffer is present at a concentration between 10 mM and 50 mM.

In some embodiments, the phosphate buffer comprises a phosphoric acid and sodium dihydrogen phosphate at a ratio between 60:40 to 40:60.

In some embodiments, provided herein are methods of preparing a stable lyophilized composition of albuvirtide from a first composition comprising (a) albuvirtide and (b) TFA or formic acid, comprising: (i) replacing the TFA or formic acid in the first composition with a phosphate buffer to make a second composition comprising albuvirtide and the phosphate buffer, (ii) adjusting the albuvirtide concentration in the second composition to be between 15 mg/ml and 35 mg/ml and the pH of the second composition to be between 1.0 and 3.5, and (iii) lyophilizing the second composition to make the stable lyophilized albuvirtide composition.

In some embodiments, the second composition further comprise acetonitrile.

In some embodiments, the phosphate buffer is present at a concentration between 15 mM and 35 mM.

In some embodiments, the phosphate buffer comprises a phosphoric acid and sodium dihydrogen phosphate at a ratio between 60:40 to 40:60.

In some embodiments, provided herein are methods of preparing a stable lyophilized composition of albuvirtide from a first composition comprising (a) albuvirtide and (b) TFA or formic acid, comprising: (i) replacing the TFA or formic acid in the first composition with a phosphate buffer to make a second composition comprising albuvirtide and the phosphate buffer, (ii) adjusting the albuvirtide concentration in the second composition to be about 20 mg/ml and the pH of the second composition to be between 1.5 and 3.0, and (iii) lyophilizing the second composition to make the stable lyophilized albuvirtide composition.

In some embodiments, the second composition further comprise acetonitrile.

In some embodiments, the phosphate buffer is present at a concentration of about 20 mM.

In some embodiments, the phosphate buffer comprises a phosphoric acid and sodium dihydrogen phosphate at a ratio of about 60:40.

Different combinations and permutations of organic solvents, acids, and salt forms thereof, in addition to those expressly exemplified herein, are also contemplated herein.

Stable lyophilized compositions prepared according to the methods described herein are also contemplated herein.

In some embodiments, methods provided herein further include dissolving stable lyophilized compositions provided herein in water-for-injection and sterilizing the dissolved solutions to prepare liquid pharmaceutical compositions. In some embodiments, methods provided herein comprise septic filtration. In some embodiments, the methods provided herein further comprise aliquoting the liquid pharmaceutical compositions described herein. In some embodiments, each aliquot comprises 20 mg albuvirtide. In some embodiments, each aliquot comprises 50 mg albuvirtide. In some embodiments, each aliquot comprises 100 mg albuvirtide. In some embodiments, each aliquot comprises 150 mg albuvirtide. In some embodiments, each aliquot comprises 200 mg albuvirtide. In some embodiments, each aliquot comprises 300 mg albuvirtide. In some embodiments, each aliquot comprises 400 mg albuvirtide. In some embodiments, each aliquot comprises 500 mg albuvirtide. In some embodiments, each aliquot contains a single dose of albuvirtide.

In some embodiments, methods provided herein further comprise lyophilizing the liquid pharmaceutical compositions of albuvirtide provided herein to prepare lyophilized pharmaceutical compositions. In some embodiments, methods provided herein comprise lyophilizing the aliquoted liquid pharmaceutical compositions of albuvirtide to prepare lyophilized pharmaceutical compositions in aliquots. In some embodiments, each aliquot comprises 20 mg albuvirtide. In some embodiments, each aliquot comprises 50 mg albuvirtide. In some embodiments, each aliquot comprises 100 mg albuvirtide. In some embodiments, each aliquot comprises 150 mg albuvirtide. In some embodiments, each aliquot comprises 200 mg albuvirtide. In some embodiments, each aliquot comprises 300 mg albuvirtide. In some embodiments, each aliquot comprises 400 mg albuvirtide. In some embodiments, each aliquot comprises 500 mg albuvirtide. In some embodiments, each aliquot contains a single dose of albuvirtide.

In some embodiments, methods provided herein comprise reconstituting the lyophilized pharmaceutical composition with a pharmaceutically acceptable carrier to make pharmaceutical formulations of albuvirtide that can be administered to a subject. In some embodiments, the pharmaceutically acceptable carrier comprises water-for-injection. In some embodiments, the pharmaceutically acceptable carrier comprises saline. In some embodiments, the pharmaceutically acceptable carrier comprises saline and sodium bicarbonate.

In some embodiments, the subject is a human. In some embodiments, the lyophilized pharmaceutical compositions can be reconstituted within a short time (e.g. less than ten minutes). In some embodiments, the lyophilized pharmaceutical compositions can be reconstituted to solutions containing about 10 mg/ml to about 80 mg/ml albuvirtide. In some embodiments, the pharmaceutical formulations of albuvirtide are ready for parenteral administration. In some embodiments, the pharmaceutical formulations of albuvirtide are ready for intravenous administration. In some embodiments, the pharmaceutical formulations of albuvirtide are ready for intramuscular administration. In some embodiments, the pharmaceutical formulations of albuvirtide are ready for intraperitoneal administration. In some embodiments, the pharmaceutical formulations of albuvirtide are ready for subcutaneous injection.

In some embodiments, provided herein are also articles of manufacture comprising the stable lyophilized compositions of albuvirtide prepared according to the methods disclosed herein. In some embodiments, an article of manufacture is provided which contains the stable lyophilized compositions described herein and instructions for its use. The article of manufacture can include a container. Suitable containers include, for example, bottles, vials (e.g., dual chamber vials), syringes (such as dual chamber syringes), autoinjector pen containing a syringe, and test tubes. The container can be formed from a variety of materials such as glass, plastic or polycarbonate. The container holds the compositions described herein and the label on, or associated with, the container can indicate directions for use. For example, the label can indicate that the composition is useful or intended for subcutaneous administration. The container holding the composition can be a multi-use vial, which allows for repeat administrations (e.g., from 2-6 administrations) of the aqueous compositions. The article of manufacture can further comprise a second container. The article of manufacture can further include other materials desirable from a commercial and user standpoint, including other buffers, diluents, filters, needles, syringes, and package inserts with instructions for use.

The methods of preparing a stable lyophilized composition of albuvirtide from a first composition comprising (a) albuvirtide and (b) TFA or formic acid, comprise:
(i) replacing the TFA or formic acid in the first composition with an acidic buffer to make a second composition comprising albuvirtide and the acidic buffer, wherein the acidic buffer comprises an acid selected from the group consisting of phosphoric acid, hydrochloric acid, acetic acid, citric acid, and sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid, and
(ii) lyophilizing the second composition to make the stable lyophilized albuvirtide composition, wherein the second composition has a pH between 1.0 and 3.5, and the method further comprises adjusting the albuvirtide concentration in the second composition to be between 5 mg/ml and 200 mg/ml before lyophilization.

In some embodiments, the liquid compositions provided herein further comprise an organic solvent. The organic solvent can be acetonitrile, ethanol, methanol, isopropanol, or any combination thereof. In some embodiments, the organic solvent is acetonitrile. In some embodiments, the organic solvent is ethanol. In some embodiments, the organic solvent is methanol. In some embodiments, the organic solvent is isopropanol. In some embodiments, the liquid compositions provided herein comprises two organic solvents. In some embodiments, the liquid compositions provided herein comprises acetonitrile and ethanol. In some embodiments, the liquid compositions provided herein comprises acetonitrile and methanol. In some embodiments, the liquid compositions provided herein comprises acetonitrile and isopropanol. In some embodiments, the liquid compositions provided herein are comprises ethanol and methanol. In some embodiments, the liquid compositions provided comprises ethanol and isopropanol. In some embodiments, the liquid compositions provided herein comprises methanol and isopropanol. The liquid compositions provided herein can comprise three organic solvents. In some embodiments, the liquid compositions provided herein comprises acetonitrile, ethanol, and methanol. In some embodiments, the liquid compositions provided herein comprises acetonitrile, ethanol, and isopropanol. In some embodiments, the liquid compositions provided herein comprises acetonitrile, methanol, and isopropanol. In some embodiments, the liquid compositions provided herein comprises ethanol, methanol, and isopropanol. In some embodiments, the liquid compositions provided herein comprises acetonitrile, ethanol, methanol, and isopropanol. In addition to those specifically exemplified above, the liquid compositions provided herein can also include other organic solvent known in the art, alone or in combination with those exemplified herein.

In some embodiments, the liquid compositions provided herein can have an organic solvent and water at different ratios. In some embodiments, the liquid compositions provided herein can have an organic solvent and water at a ratio between 5:95 (v:v) and 95:5 (v:v), between 10:90 (v:v) and 90:10 (v:v), between 15:85 (v:v) and 85:15 (v:v), between 20:80 (v:v) and 80:20 (v:v), between 20:80 (v:v) and 45:55 (v:v), between 25:75 (v:v) and 75:25 (v:v), between 30:70 (v:v) and 70:30 (v:v), between 30:70 (v:v) and 40:60 (v:v), between 35:65 (v:v) and 65:35 (v:v), between 40:60 (v:v) and 60:40 (v:v), between 45:55 (v:v) and 55:45 (v:v), at about 35:65, or at about 50:50 (v:v). In some embodiments, the liquid compositions provided herein can have an organic solvent and water at a ratio between 5:95 (v:v) and 95:5 (v:v). In some embodiments, the liquid compositions provided herein can have an organic solvent and water at a ratio between 10:90 (v:v) and 90:10 (v:v). In some embodiments, the liquid compositions provided herein can have an organic solvent and water at a ratio between 15:85 (v:v) and 85:15 (v:v). In some embodiments, the liquid compositions provided herein can have an organic solvent and water at a ratio between 20:80 (v:v) and 80:20 (v:v). In some embodiments, the liquid compositions provided herein can have an organic solvent and water at a ratio between 20:80 (v:v) and 45:55 (v:v).In some embodiments, the liquid compositions provided herein can have an organic solvent and water at a ratio between 25:75 (v:v) and 75:25 (v:v). In some embodiments, the liquid compositions provided herein can have an organic solvent and water at a ratio between 30:70 (v:v) and 70:30 (v:v). In some embodiments, the liquid compositions provided herein can have an organic solvent and water at a ratio between 30:70 (v:v) and 40:60 (v:v). In some embodiments, the liquid compositions provided herein can have an organic solvent and water at a ratio between 35:65 (v:v) and 65:35 (v:v). In some embodiments, the liquid compositions provided herein can have an organic solvent and water at a ratio between 40:60 (v:v) and 60:40 (v:v). In some embodiments, the liquid compositions provided herein can have an organic solvent and water at a ratio between 45:55 (v:v) and 55:45 (v:v). In some embodiments, the liquid compositions provided herein can have an organic solvent and water at a ratio about 35:65 (v:v). In some embodiments, the liquid compositions provided herein can have an organic solvent and water at a ratio about 50:50 (v:v).

In some embodiments, the liquid compositions provided herein can comprise albuvirtide and an acidic buffer, wherein the amount of the acid buffer can be adjusted to a molar ratio of acidic buffer and albuvirtide is between 1: 10 and 200: 1, between 1:5 and 150:1, between 1:5 and 100: 1, between 1:2 and 100: 1, between 1:2 and 50:1, between 1:1 and 50:1, between 1:1 and 25:1, between 2: 1 and 25: 1, between 3:1 and 25:1, between 3: 1 and 10:1.

Methods of preparing stable lyophilized compositions of albuvirtide provided herein comprise adjusting the albuvirtide concentration in the liquid composition to be between 5 and 200 mg/mL, between 5 and 175 mg/mL, between 5 and 150 mg/mL, between 5 and 125 mg/mL, or between 5 and 100 mg/mL. In certain embodiments, the liquid compositions are adjusted to comprise albuvirtide at a concentration of between 10 and 200 mg/mL. between 10 and 175 mg/mL, between 10 and 150 mg/mL, between 10 and 120 mg/mL, or between 10 and 100 mg/mL. In certain embodiments, the liquid compositions are adjusted to comprise albuvirtide at a concentration of between 15 and 200 mg/mL, between 15 and 175 mg/mL, between 15 and 150 mg/mL, between 15 and 125 mg/mL, or between 15 and 100 mg/mL. In certain embodiments, the liquid compositions are adjusted to comprise albuvirtide at a concentration of between 20 and 300 mg/mL, between 20 and 250 mg/mL, between 20 and 200 mg/mL, between 20 and 175 mg/mL, between 20 and 150 mg/mL, between 20 and 125 mg/mL, or between 20 and 100 mg/mL.

In certain embodiments, the liquid compositions are adjusted to comprise albuvirtide at a concentration of between 5 and 75 mg/mL, between 10 and 75 mg/mL, between 15 and 75 mg/mL, between 20 and 75 mg/mL, between 5 and 50 mg/mL, between 10 and 50 mg/mL, between 15 and 50 mg/mL, between 20 and 50 mg/mL, between 5 and 45 mg/mL, between 10 and 45 mg/mL, between 15 and 45 mg/mL, between 20 and 45 mg/mL, between 5 and 40 mg/mL, between 10 and 40 mg/mL, between 15 and 40 mg/mL, between 20 and 40 mg/mL, between 5 and 35 mg/mL, between 10 and 35 mg/mL, between 15 and 35 mg/mL, between 20 and 35 mg/mL, between 5 and 30 mg/mL, between 10 and 30 mg/mL, between 15 and 30 mg/mL, or between 20 and 30 mg/mL.

The liquid compositions provided herein are adjusted to comprise albuvirtide at a concentration of between 5 and 200 mg/mL. In some embodiments, the liquid compositions provided herein are adjusted to comprise albuvirtide at a concentration of between 5 and 100 mg/mL. In some embodiments, the liquid compositions provided herein are adjusted to comprise albuvirtide at a concentration of between 5 and 75 mg/mL. In some embodiments, the liquid compositions provided herein are adjusted to comprise albuvirtide at a concentration of between 5 and 50 mg/mL. In some embodiments, the liquid compositions provided herein are adjusted to comprise albuvirtide at a concentration of between 5 and 45 mg/mL. In some embodiments, the liquid compositions provided herein are adjusted to comprise albuvirtide at a concentration of between 5 and 40 mg/mL. In some embodiments, the liquid compositions provided herein are adjusted to comprise albuvirtide at a concentration of between 5 and 35 mg/mL. In some embodiments, the liquid compositions provided herein are adjusted to comprise albuvirtide at a concentration of between 5 and 30 mg/mL. In some embodiments, the liquid compositions provided herein are adjusted to comprise albuvirtide at a concentration of between 5 and 25 mg/mL.

In some embodiments, the liquid compositions provided herein are adjusted to comprise albuvirtide at a concentration of between 15 and 200 mg/mL. In some embodiments, the liquid compositions provided herein are adjusted to comprise albuvirtide at a concentration of between 15 and 100 mg/mL. In some embodiments, the liquid compositions provided herein are adjusted to comprise albuvirtide at a concentration of between 15 and 75 mg/mL. In some embodiments, the liquid compositions provided herein are adjusted to comprise albuvirtide at a concentration of between 15 and 50 mg/mL. In some embodiments, the liquid compositions provided herein are adjusted to comprise albuvirtide at a concentration of between 15 and 45 mg/mL. In some embodiments, the liquid compositions provided herein are adjusted to comprise albuvirtide at a concentration of between 15 and 40 mg/mL. In some embodiments, the liquid compositions provided herein are adjusted to comprise albuvirtide at a concentration of between 15 and 35 mg/mL. In some embodiments, the liquid compositions provided herein are adjusted to comprise albuvirtide at a concentration of between 15 and 30 mg/mL. In some embodiments, the liquid compositions provided herein are adjusted to comprise albuvirtide at a concentration of between 15 and 25 mg/mL.

In certain embodiments, the liquid compositions provided herein are adjusted to comprise albuvirtide at a concentration of, 10 mg/mL, 15 mg/mL, 20 mg/mL, 25 mg/mL, 30 mg/mL, 35 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, 100 mg/mL, 110 mg/mL, 120 mg/mL, 130 mg/mL, 140 mg/mL, 150 mg/mL, 160 mg/mL, 170 mg/mL, 180 mg/mL, or 190 mg/ mL.

. In some embodiments, the liquid compositions provided herein are adjusted to comprise albuvirtide at a concentration of about 10 mg/mL. In some embodiments, the liquid compositions provided herein are adjusted to comprise albuvirtide at a concentration of about 15 mg/mL. In some embodiments, the liquid compositions provided herein are adjusted to comprise albuvirtide at a concentration of about 20 mg/mL. In some embodiments, the liquid compositions provided herein are adjusted to comprise albuvirtide at a concentration of about 25 mg/mL. In some embodiments, the liquid compositions provided herein are adjusted to comprise albuvirtide at a concentration of about 30 mg/mL. In some embodiments, the liquid compositions provided herein are adjusted to comprise albuvirtide at a concentration of about 35 mg/mL. In some embodiments, the liquid compositions provided herein are adjusted to comprise albuvirtide at a concentration of about 40 mg/mL. In some embodiments, the liquid compositions provided herein are adjusted to comprise albuvirtide at a concentration of about 45 mg/mL. In some embodiments, the liquid compositions provided herein are adjusted to comprise albuvirtide at a concentration of about 50 mg/mL. In some embodiments, the liquid compositions provided herein are adjusted to comprise albuvirtide at a concentration of about 75 mg/mL. In some embodiments, the liquid compositions provided herein are adjusted to comprise albuvirtide at a concentration of about 100 mg/mL. In some embodiments, the liquid compositions provided herein are adjusted to comprise albuvirtide at a concentration of about 125 mg/mL. In some embodiments, the liquid compositions provided herein are adjusted to comprise albuvirtide at a concentration of about 150 mg/mL. In some embodiments, the liquid compositions provided herein are adjusted to comprise albuvirtide at a concentration of about 175 mg/mL

In some embodiments, the liquid compositions provided herein are adjusted to comprise an acidic buffer has a concentration between 5 and 200 mM. The buffer concentration refers to the total concertation of the acid and the salt thereof. For example, if the buffer of a liquid composition consists of 5 mM phosphoric acid and 5 mM sodium phosphate, its buffer concentration is 10 mM.

In some embodiments, the liquid compositions provided herein are adjusted to comprise an acidic buffer at a concentration between 5 and 175 mM, between 5 and 150 mM, between 5 and 125 mM, between 5 and 100 mM, between 10 and 200 mM, between 10 and 175 mM, between 10 and 150 mM, between 10 and 125 mM, between 10 and 100 mM. In some embodiments, the liquid compositions provided herein are adjusted to comprise an acidic buffer at a concentration between 5 and 75 mM, between 5 and 50 mM, between 5 and 45 mM, between 5 and 40 mM, between 5 and 35 mM, 5 and 30 mM, between 5 and 25 mM, between 5 and 20 mM.In some embodiments, the liquid compositions provided herein are adjusted to comprise an acidic buffer at a concentration between 10 and 75 mM, between 10 and 50 mM, between 10 and 45 mM, between 10 and 40 mM, between 10 and 35 mM, between 10 and 30 mM, between 10 and 25 mM, between 10 and 20 mM. In some embodiments, the liquid compositions provided herein are adjusted to comprise an acidic buffer at a concentration between 15 and 75 mM, between 15 and 50 mM, between 15 and 45 mM, between 15 and 40 mM, between 15 and 35 mM, between 15 and 30 mM, between 15 and 25 mM, between 15 and 20 mM.

In some embodiments, the liquid compositions provided herein are adjusted to comprise an acidic buffer at a concentration of about 5 mM, 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 100 mM, 110 mM, 120 mM, 130 mM, 140 mM, 150 mM, 160 mM, 170 mM, 180 mM, 190 mM, 195 mM, or 200 mM.

The liquid compositions provided herein are adjusted to have a pH between 1.0 and 3.5. In some embodiments, the liquid compositions provided herein are adjusted to have a pH between 1.0 and 3.0. In some embodiments, the liquid compositions provided herein are adjusted to have a pH between 1.0 and 2.5In some embodiments, the liquid compositions provided herein are adjusted to have a pH between 1.5 and 3.5. In some embodiments, the liquid compositions provided herein are adjusted to have a pH between 1.5 and 3.0. In some embodiments, the liquid compositions provided herein are adjusted to have a pH between 1.5 and 2.5. In some embodiments, the liquid compositions provided herein are adjusted to have a pH between 2.0 and 3.5. In some embodiments, the liquid compositions provided herein are adjusted to have a pH between 2.0 and 3.0. In some embodiments, the liquid compositions provided herein are adjusted to have a pH between 2.0 and 2.5.

In some embodiments, the liquid compositions provided herein are adjusted to have a pH of 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, or 3.4. In some embodiments, the liquid compositions provided herein are adjusted to have a pH of about 1.5. In some embodiments, the liquid compositions provided herein are adjusted to have a pH of about 2.0. In some embodiments, the liquid compositions provided herein are adjusted to have a pH of about 2.5. In some embodiments, the liquid compositions provided herein are adjusted to have a pH of about 3.0.

The measurements of the pH can be taken at the temperature of 4 °C. The measurements of the pH can be taken at room temperature. The measurements of the pH can be taken at about 22 °C.

In some embodiments, the acidic buffer in the liquid composition can comprise an acid selected from the group consisting of a phosphoric acid, hydrochloric acid, acetic acid, citric acid, sulfuric acid, tartaric acid, lactic acid, succinic acid, ascorbic acid, aspartic acid, glutamic acid, propanoic acid, propanedioic acid, butyric acid, maleic acid, fumaric acid, malic acid, oxalic acid, and salts thereof. In some embodiments, the acidic buffer can comprise a phosphoric acid and a salt thereof. In some embodiments, the acidic buffer can comprise a hydrochloric acid and a salt thereof. In some embodiments, the acidic buffer can comprise an acetic acid and a salt thereof. In some embodiments, the acidic buffer can comprise a citric acid and a salt thereof. In some embodiments, the acidic buffer can comprise a sulfuric acid and a salt thereof. In some embodiments, the acidic buffer can comprise a tartaric acid and a salt thereof. In some embodiments, the acidic buffer can comprise a lactic acid and a salt thereof. In some embodiments, the acidic buffer can comprise a succinic acid and a salt thereof. In some embodiments, the acidic buffer can comprise an ascorbic acid and a salt thereof. In some embodiments, the acidic buffer can comprise an aspartic acid and a salt thereof. In some embodiments, the acidic buffer can comprise a glutamic acid and a salt thereof. In some embodiments, the acidic buffer can comprise a propanoic acid and a salt thereof. In some embodiments, the acidic buffer can comprise a propanedioic acid and a salt thereof. In some embodiments, the acidic buffer can comprise a butyric acid and a salt thereof. In some embodiments, the acidic buffer can comprise a maleic acid and a salt thereof. In some embodiments, the acidic buffer can comprise a fumaric acid and a salt thereof. In some embodiments, the acidic buffer can comprise a malic acid and a salt thereof. In some embodiments, the acidic buffer can comprise an oxalic acid and a salt thereof.

In some embodiments, the salt form of the acid comprises a sodium salt of the acid. In some embodiments, the salt form of the acid comprises a potassium salt of the acid. In some embodiments, the salt form of the acid comprises an ammonium salt of the acid. In some embodiments, the salt form of the acid comprises any combination of a sodium salt , a potassium salt, and an ammonium salt of the acid.

In some embodiments, the acidic buffer used in the methods disclosed herein can comprise a phosphoric acid and sodium phosphate. In some embodiments, the acidic buffer can comprise a hydrochloric acid and sodium chloride. In some embodiments, the acidic buffer can comprise an acetic acid and sodium acetate. In some embodiments, the acidic buffer can comprise a citric acid and sodium citrate. In some embodiments, the acidic buffer can comprise a sulfuric acid and sodium sulfate. In some embodiments, the acidic buffer can comprise a tartaric acid and sodium tartrate. In some embodiments, the acidic buffer can comprise a lactic acid and sodium lactate. In some embodiments, the acidic buffer can comprise a succinic acid and sodium succinate. In some embodiments, the acidic buffer can comprise an ascorbic acid and sodium ascorbate. In some embodiments, the acidic buffer can comprise an aspartic acid and sodium aspartate. In some embodiments, the acidic buffer can comprise a glutamic acid and sodium glutamate. In some embodiments, the acidic buffer can comprise a propanoic acid and sodium propanoate. In some embodiments, the acidic buffer can comprise a propanedioic acid and sodium propanedioate. In some embodiments, the acidic buffer can comprise a butyric acid and sodium butyrate. In some embodiments, the acidic buffer can comprise a maleic acid and sodium maleate. In some embodiments, the acidic buffer can comprise a fumaric acid and sodium fumarate. In some embodiments, the acidic buffer can comprise a malic acid and sodium malate. In some embodiments, the acidic buffer can comprise an oxalic acid and sodium oxalate.

In some embodiments, the acidic buffer used in the methods disclosed herein can comprise a phosphoric acid and potassium phosphate. In some embodiments, the acidic buffer can comprise a hydrochloric acid and potassium chloride. In some embodiments, the acidic buffer can comprise an acetic acid and potassium acetate. In some embodiments, the acidic buffer can comprise a citric acid and potassium citrate. In some embodiments, the acidic buffer can comprise a sulfuric acid and potassium sulfate. In some embodiments, the acidic buffer can comprise a tartaric acid and potassium tartrate. In some embodiments, the acidic buffer can comprise a lactic acid and potassium lactate. In some embodiments, the acidic buffer can comprise a succinic acid and potassium succinate. In some embodiments, the acidic buffer can comprise an ascorbic acid and potassium ascorbate. In some embodiments, the acidic buffer can comprise an aspartic acid and potassium aspartate. In some embodiments, the acidic buffer can comprise a glutamic acid and potassium glutamate. In some embodiments, the acidic buffer can comprise a propanoic acid and potassium propanoate. In some embodiments, the acidic buffer can comprise a propanedioic acid and potassium propanedioate. In some embodiments, the acidic buffer can comprise a butyric acid and potassium butyrate. In some embodiments, the acidic buffer can comprise a maleic acid and potassium maleate. In some embodiments, the acidic buffer can comprise a fumaric acid and potassium fumarate. In some embodiments, the acidic buffer can comprise a malic acid and potassium malate. In some embodiments, the acidic buffer can comprise an oxalic acid and potassium oxalate.

In some embodiments, the acidic buffer used in the methods disclosed herein can comprise a phosphoric acid and ammonium phosphate. In some embodiments, the acidic buffer can comprise a hydrochloric acid and ammonium chloride. In some embodiments, the acidic buffer can comprise an acetic acid and ammonium acetate. In some embodiments, the acidic buffer can comprise a citric acid and ammonium citrate. In some embodiments, the acidic buffer can comprise a sulfuric acid and ammonium sulfate. In some embodiments, the acidic buffer can comprise a tartaric acid and ammonium tartrate. In some embodiments, the acidic buffer can comprise a lactic acid and ammonium lactate. In some embodiments, the acidic buffer can comprise a succinic acid and ammonium succinate. In some embodiments, the acidic buffer can comprise an ascorbic acid and ammonium ascorbate. In some embodiments, the acidic buffer can comprise an aspartic acid and ammonium aspartate. In some embodiments, the acidic buffer can comprise a glutamic acid and ammonium glutamate. In some embodiments, the acidic buffer can comprise a propanoic acid and ammonium propanoate. In some embodiments, the acidic buffer can comprise a propanedioic acid and ammonium propanedioate. In some embodiments, the acidic buffer can comprise a butyric acid and ammonium butyrate. In some embodiments, the acidic buffer can comprise a maleic acid and ammonium maleate. In some embodiments, the acidic buffer can comprise a fumaric acid and ammonium fumarate. In some embodiments, the acidic buffer can comprise a malic acid and ammonium malate. In some embodiments, the acidic buffer can comprise an oxalic acid and ammonium oxalate.

In some embodiments, the liquid compositions comprise albuvirtide and two acidic buffers, wherein the acidic buffers are selected form the group consisting of a phosphoric acid, hydrochloric acid, acetic acid, citric acid, sulfuric acid, tartaric acid, lactic acid, succinic acid, ascorbic acid, aspartic acid, glutamic acid, propanoic acid, propanedioic acid, butyric acid, maleic acid, fumaric acid, malic acid, oxalic acid, and salts thereof. In some embodiments, the liquid compositions provided herein are adjusted to comprise albuvirtide and three acidic buffers, wherein the acidic buffers are selected form the group consisting of a phosphoric acid, hydrochloric acid, acetic acid, citric acid, sulfuric acid, tartaric acid, lactic acid, succinic acid, ascorbic acid, aspartic acid, glutamic acid, propanoic acid, propanedioic acid, butyric acid, maleic acid, fumaric acid, malic acid, oxalic acid, and salts thereof. In some embodiments, the liquid compositions provided herein are adjusted to comprise albuvirtide and four acidic buffers, wherein the acidic buffers are selected form the group consisting of a phosphoric acid, hydrochloric acid, acetic acid, citric acid, sulfuric acid, tartaric acid, lactic acid, succinic acid, ascorbic acid, aspartic acid, glutamic acid, propanoic acid, propanedioic acid, butyric acid, maleic acid, fumaric acid, malic acid, oxalic acid, and salts thereof.

In some embodiments, the liquid compositions provided herein are adjusted to comprise an acidic buffer having the acid and the salt form of the acid at different ratios. In some embodiments, the acidic buffer can have the acid and salt form of the acid at a ratio between 100:0 (molar) and 5:95 (molar), namely, of 100 mM acidic buffer, there are 100 mM acid and 0 mM salt thereof, 5 mM acid and 95 mM salt thereof, or anything in between. In some embodiments, the acidic buffer can have the acid and salt form of the acid at a ratio between 95:5 (molar) and 5:95 (molar), between 90:10 (molar) and 10:90 (molar), between 85:15 (molar) and 15:85 (molar), between 80:20 (molar) and 20:80 (molar), between 75:25 (molar) and 25:75 (molar), between 70:30 (molar) and 30:70 (molar), between 65:35 (molar) and 35:65 (molar), between 60:40 (molar) and 40:60 (molar), between 55:45 (molar) and 45:55 (molar), or at about 50:50 (molar). In some embodiments, the liquid compositions provided herein can have acid and salt form of the acid at a ratio between 100:0 (molar) and 5:95 (molar). In some embodiments, the liquid compositions provided herein can have the acid and salt form of the acid at a ratio between 95:5 (molar) and 5:95 (molar). In some embodiments, the liquid compositions provided herein can have the acid and salt form of the acid at a ratio between 90:10 (molar) and 10:90 (molar). In some embodiments, the liquid compositions provided herein can have the acid and salt form of the acid at a ratio between 85:15 (molar) and 15:85 (molar). In some embodiments, the liquid compositions provided herein can have the acid and salt form of the acid at a ratio between 80:20 (molar) and 20:80 (molar). In some embodiments, the liquid compositions provided herein can have the acid and salt form of the acid at a ratio between 75:25 (molar) and 25:75 (molar). In some embodiments, the liquid compositions provided herein can have the acid and salt form of the acid at a ratio between 70:30 (molar) and 30:70 (molar). In some embodiments, the liquid compositions provided herein can have the acid and salt form of the acid at a ratio between 65:35 (molar) and 35:65 (molar). In some embodiments, the liquidcompositions provided herein can have the acid and salt form of the acid at a ratio between 60:40 (molar) and 40:60 (molar). In some embodiments, the liquid compositions provided herein can have the acid and salt form of the acid at a ratio between 55:45 (molar) and 45:55 (molar).

In some embodiments, the liquid compositions provided herein can have the acid and salt form of the acid at a ratio of about 90:10 (molar). In some embodiments, the liquid compositions provided herein can have the acid and salt form of the acid at a ratio of about 80:20 (molar). In some embodiments, the liquid compositions provided herein can have the acid and salt form of the acid at a ratio of about 70:30 (molar). In some embodiments, the liquid compositions provided herein can have the acid and salt form of the acid at a ratio of about 60:40 (molar). In some embodiments, the liquid compositions provided herein can have the acid and salt form of the acid at a ratio of about 50:50 (molar).

In some embodiments, the methods of preparing stable lyophilized compositions of albuvirtide from a liquid composition comprising (a) albuvirtide and (b) an acidic buffer comprising:
(i)adjusting the amount of albuvirtide and the acidic buffer in the liquid composition; and (ii) lyophilizing the liquid composition to make the stable lyophilized albuvirtide composition, wherein the acidic buffer is a phosphate buffer.

In some embodiments, the liquid compositions comprise albuvirtide and a phosphate buffer, wherein the liquid compositions have a pH between 1.0 and 3.5. The phosphate buffer can comprise a phosphoric acid and a salt thereof. In some embodiments, the phosphate buffer can comprise a phosphoric acid and sodium phosphate. In some embodiments, the phosphate buffer can comprise a phosphoric acid and sodium dihydrogen phosphate. In some embodiments, the phosphate buffer can comprise a phosphoric acid and potassium phosphate. In some embodiments, the phosphate buffer can comprise a phosphoric acid and potassium dihydrogen phosphate. In some embodiments, the phosphate buffer can comprise a phosphoric acid and ammonium phosphate. In some embodiments, the phosphate buffer can comprise a phosphoric acid and ammonium dihydrogen phosphate.

In some embodiments, the liquid compositions provided herein can comprise albuvirtide and a phosphate buffer, wherein the phosphate buffer has a concentration between 5 and 200 mM. The buffer concentration refers to the total concertation of the phosphoric acid and the salt thereof. For example, if the buffer of a liquid composition consists of 5 mM phosphoric acid and 5 mM sodium dihydrogen phosphate, its buffer concentration is 10 mM.

In some embodiments, the liquid compositions provided herein can comprise a phosphate buffer at a concentration between 5 and 175 mM. In some embodiments, the liquid compositions provided herein can comprise a phosphate buffer at a concentration between 5 and 150 mM. In some embodiments, the liquid compositions provided herein can comprise a phosphate buffer at a concentration between 5 and 125 mM. In some embodiments, the liquid compositions provided herein can comprise a phosphate buffer at a concentration between 5 and 100 mM. In some embodiments, the liquid compositions provided herein can comprise a phosphate buffer at a concentration between 10 and 200 mM. In some embodiments, the liquid compositions provided herein can comprise a phosphate buffer at a concentration between 10 and 175 mM. In some embodiments, the liquid compositions provided herein can comprise a phosphate buffer at a concentration between 10 and 150 mM. In some embodiments, the liquid compositions provided herein can comprise a phosphate buffer at a concentration between 10 and 125 mM. In some embodiments, the liquid compositions provided herein can comprise a phosphate buffer at a concentration between 10 and 100 mM.

In some embodiments, the liquid compositions provided herein can comprise a phosphate buffer at a concentration between 5 and 75 mM. In some embodiments, the liquid compositions provided herein can comprise a phosphate buffer at a concentration between 5 and 50 mM. In some embodiments, the liquid compositions provided herein can comprise a phosphate buffer at a concentration between 5 and 45 mM. In some embodiments, the liquid compositions provided herein can comprise a phosphate buffer at a concentration between 5 and 40 mM. some embodiments, the liquid compositions provided herein can comprise a phosphate buffer at a concentration between 5 and 35 mM. In some embodiments, the liquid compositions provided herein can comprise a phosphate buffer at a concentration between 5 and 30 mM. In some embodiments, the liquid compositions provided herein can comprise a phosphate buffer at a concentration between 5 and 25 mM. In some embodiments, the liquid compositions provided herein can comprise a phosphate buffer at a concentration between 5 and 20 mM.

In some embodiments, the liquid compositions provided herein can comprise a phosphate buffer at a concentration between 10 and 75 mM. In some embodiments, the liquid compositions provided herein can comprise a phosphate buffer at a concentration between 10 and 50 mM. In some embodiments, the liquid compositions provided herein can comprise a phosphate buffer at a concentration between 10 and 45 mM. In some embodiments, the liquid compositions provided herein can comprise a phosphate buffer at a concentration between 10 and 40 mM. some embodiments, the liquid compositions provided herein can comprise a phosphate buffer at a concentration between 10 and 35 mM. In some embodiments, the liquid compositions provided herein can comprise a phosphate buffer at a concentration between 10 and 30 mM. In some embodiments, the liquid compositions provided herein can comprise a phosphate buffer at a concentration between 10 and 25 mM. In some embodiments, the liquid compositions provided herein can comprise a phosphate buffer at a concentration between 10 and 20 mM.

In some embodiments, the liquid compositions provided herein can comprise a phosphate buffer at a concentration between 15 and 75 mM. In some embodiments, the liquid compositions provided herein can comprise a phosphate buffer at a concentration between 15 and 50 mM. In some embodiments, the liquid compositions provided herein can comprise a phosphate buffer at a concentration between 15 and 45 mM. In some embodiments, the liquid compositions provided herein can comprise a phosphate buffer at a concentration between 15 and 40 mM. some embodiments, the liquid compositions provided herein can comprise a phosphate buffer at a concentration between 15 and 35 mM. In some embodiments, the liquid compositions provided herein can comprise a phosphate buffer at a concentration between 15 and 30 mM. In some embodiments, the liquid compositions provided herein can comprise a phosphate buffer at a concentration between 15 and 25 mM. In some embodiments, the liquid compositions provided herein can comprise a phosphate buffer at a concentration between 15 and 20 mM.

In some embodiments, the liquid compositions provided herein can comprise a phosphate buffer at a concentration of about 5 mM, 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 100 mM, 110 mM, 120 mM, 130 mM, 140 mM, 150 mM, 160 mM, 170 mM, 180 mM, 190 mM, or 195 mM. In some embodiments, the liquid compositions provided herein can comprise a phosphate buffer at a concentration of about 5 mM. In some embodiments, the liquid compositions provided herein can comprise a phosphate buffer at a concentration of about 10 mM. In some embodiments, the liquid compositions provided herein can comprise a phosphate buffer at a concentration of about 20 mM. In some embodiments, the liquid compositions provided herein can comprise a phosphate buffer at a concentration of about 30 mM. In some embodiments, the liquid compositions provided herein can comprise a phosphate buffer at a concentration of about 40 mM. In some embodiments, the liquid compositions provided herein can comprise a phosphate buffer at a concentration of about 50 mM. In some embodiments, the liquid compositions provided herein can comprise a phosphate buffer at a concentration of about 75 mM. In some embodiments, the liquid compositions provided herein can comprise a phosphate buffer at a concentration of about 100 mM. In some embodiments, the liquid compositions provided herein can comprise a phosphate buffer at a concentration of about 125 mM. In some embodiments, the liquid compositions provided herein can comprise a phosphate buffer at a concentration of about 150 mM. In some embodiments, the liquid compositions provided herein can comprise a phosphate buffer at a concentration of about 175 mM. In some embodiments, the liquid compositions provided herein can comprise a phosphate buffer at a concentration of about 200 mM.

In some embodiments, the liquid compositions provided herein are adjusted to comprise a phosphate buffer having the phosphoric acid and the salt form of the phosphoric acid at different ratios. In some embodiments, the salt form of the phosphoric acid is sodium phosphate. In some embodiments, the salt form of the phosphoric acid is sodium dihydrogen phosphate. In some embodiments, the salt form of the phosphoric acid is potassium phosphate. In some embodiments, the salt form of the phosphoric acid is potassium dihydrogen phosphate. In some embodiments, the salt form of the phosphoric acid is ammonium phosphate. In some embodiments, the salt form of the phosphoric acid is ammonium dihydrogen phosphate. In some embodiments, the salt form of the phosphoric acid comprises any combination of the salts above.

In some embodiments, the phosphate buffer in the liquid compositions can have the phosphoric acid and salt form of the phosphoric acid at a ratio between 100:0 (molar) and 5:95 (molar), between 95:5 (molar) and 5:95 (molar), between 90:10 (molar) and 10:90 (molar), between 85:15 (molar) and 15:85 (molar), between 80:20 (molar) and 20:80 (molar), between 75:25 (molar) and 25:75 (molar), between 70:30 (molar) and 30:70 (molar), between 65:35 (molar) and 35:65 (molar), between 60:40 (molar) and 40:60 (molar), between 55:45 (molar) and 45:55 (molar), or at about 50:50 (molar). In some embodiments, the liquid compositions provided herein can have the phosphoric acid and salt form of the phosphoric acid at a ratio between 100:0 (molar) and 5:95 (molar). In some embodiments, the liquid compositions provided herein can have the phosphoric acid and salt form of the phosphoric acid at a ratio between 95:5 (molar) and 5:95 (molar). In some embodiments, the liquid compositions provided herein can have the phosphoric acid and salt form of the phosphoric acid at a ratio between 90:10 (molar) and 10:90 (molar). In some embodiments, the liquid compositions provided herein can have the phosphoric acid and salt form of the phosphoric acid at a ratio between 85:15 (molar) and 15:85 (molar). In some embodiments, the liquid compositions provided herein can have the phosphoric acid and salt form of the phosphoric acid at a ratio between 80:20 (molar) and 20:80 (molar). In some embodiments, the liquid compositions provided herein can have the phosphoric acid and salt form of the phosphoric acid at a ratio between 75:25 (molar) and 25:75 (molar). In some embodiments, the liquid compositions provided herein can have the phosphoric acid and salt form of the phosphoric acid at a ratio between 70:30 (molar) and 30:70 (molar). In some embodiments, the liquid compositions provided herein can have the phosphoric acid and salt form of the phosphoric acid at a ratio between 65:35 (molar) and 35:65 (molar). In some embodiments, the liquid compositions provided herein can have the phosphoric acid and salt form of the phosphoric acid at a ratio between 60:40 (molar) and 40:60 (molar). In some embodiments, the liquid compositions provided herein can have the phosphoric acid and salt form of the phosphoric acid at a ratio between 55:45 (molar) and 45:55 (molar).

In some embodiments, the liquid compositions provided herein can have the phosphoric acid and salt form of the phosphoric acid at a ratio of about 90:10 (molar). In some embodiments, the liquid compositions provided herein can have the phosphoric acid and salt form of the phosphoric acid at a ratio of about 80:20 (molar). In some embodiments, the liquid compositions provided herein can have the phosphoric acid and salt form of the phosphoric acid at a ratio of about 70:30 (molar). In some embodiments, the liquid compositions provided herein can have the phosphoric acid and salt form of the phosphoric acid at a ratio of about 60:40 (molar). In some embodiments, the liquid compositions provided herein can have the phosphoric acid and salt form of the phosphoric acid at a ratio of about 50:50 (molar).

The methods of preparing a stable lyophilized composition of albuvirtide from a first composition comprising (a) albuvirtide and (b) TFA or formic acid, comprise:
(i) replacing the TFA or formic acid in the first composition with an acidic buffer to make a second composition comprising albuvirtide and the acidic buffer, wherein the acidic buffer comprises an acid selected from the group consisting of phosphoric acid, hydrochloric acid, acetic acid, citric acid, and sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid, and
(ii) lyophilizing the second composition to make the stable lyophilized albuvirtide composition, wherein the second composition has a pH between 1.0 and 3.5, and the method further comprises adjusting the albuvirtide concentration in the second composition to be between 5 mg/ml and 200 mg/ml before lyophilization.

In some embodiments, the phosphate buffer is present at a concentration between 10 mM and 200 mM.

In some embodiments, the phosphate buffer comprises a phosphoric acid and sodium dihydrogen phosphate at a ratio between 80:20 to 20:80.

In some embodiments, the methods of preparing stable lyophilized compositions of albuvirtide from a liquid composition comprising (a) albuvirtide and (b) an acidic buffer comprising: (i) adjusting the amount of albuvirtide and the acidic buffer in the liquid composition so that albuvirtide has a concentration between 10 mg/ml and 50 mg/ml and the acidic buffer has a concentration between 10 mM and 50 mM, wherein the acidic buffer comprises an acid selected from the group consisting of phosphoric acid, hydrochloric acid, acetic acid, citric acid, sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid; and (ii) lyophilizing the liquid composition to make the stable lyophilized albuvirtide composition.

In some embodiments, the phosphate buffer comprises a phosphoric acid and sodium dihydrogen phosphate at a ratio between 60:40 to 40:60.

In some embodiments, the methods of preparing stable lyophilized compositions of albuvirtide from a liquid composition comprising (a) albuvirtide and (b) an acidic buffer comprising: (i) adjusting the amount of albuvirtide and the acidic buffer in the liquid composition so that albuvirtide has a concentration between 10 mg/ml and 50 mg/ml and the acidic buffer has a concentration between 15 mM and 35 mM, wherein the acidic buffer comprises an acid selected from the group consisting of phosphoric acid, hydrochloric acid, acetic acid, citric acid, sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid; and (ii) lyophilizing the liquid composition to make the stable lyophilized albuvirtide composition.

In some embodiments, the phosphate buffer comprises a phosphoric acid and sodium dihydrogen phosphate at a ratio between 60:40 to 40:60.

In some embodiments, the methods of preparing stable lyophilized compositions of albuvirtide from a liquid composition comprising (a) albuvirtide and (b) an acidic buffer comprising: (i) adjusting the amount of albuvirtide and the acidic buffer in the liquid composition so that albuvirtide has a concentration between 15 mg/ml and 25 mg/ml and the acidic buffer has a concentration of about 20 mM, wherein the acidic buffer comprises an acid selected from the group consisting of phosphoric acid, hydrochloric acid, acetic acid, citric acid, sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid; and (ii) lyophilizing the liquid composition to make the stable lyophilized albuvirtide composition.

In some embodiments, the phosphate buffer comprises a phosphoric acid and sodium dihydrogen phosphate at a ratio of about 60:40.

Different combinations and permutations of organic solvents, acids, and salt forms thereof, in addition to those expressly exemplified herein, are also contemplated herein.

Stable lyophilized compositions prepared according to the methods described herein are also contemplated herein.

In some embodiments, methods provided herein further include dissolving stable lyophilized compositions provided herein in water-for-injection and sterilizing the dissolved solutions to prepare liquid pharmaceutical compositions. In some embodiments, methods provided herein comprise septic filtration. In some embodiments, the methods provided herein further comprise aliquoting the liquid pharmaceutical compositions described herein. In some embodiments, each aliquot comprises 20 mg albuvirtide. In some embodiments, each aliquot comprises 50 mg albuvirtide. In some embodiments, each aliquot comprises 100 mg albuvirtide. In some embodiments, each aliquot comprises 150 mg albuvirtide. In some embodiments, each aliquot comprises 200 mg albuvirtide. In some embodiments, each aliquot comprises 300 mg albuvirtide. In some embodiments, each aliquot comprises 400 mg albuvirtide. In some embodiments, each aliquot comprises 500 mg albuvirtide. In some embodiments, each aliquot contains a single dose of albuvirtide.

In some embodiments, methods provided herein further comprise lyophilizing the liquid pharmaceutical compositions of albuvirtide provided herein to prepare lyophilized pharmaceutical compositions. In some embodiments, methods provided herein comprise lyophilizing the aliquoted liquid pharmaceutical compositions of albuvirtide to prepare lyophilized pharmaceutical compositions in aliquots. In some embodiments, each aliquot comprises 20 mg albuvirtide. In some embodiments, each aliquot comprises 50 mg albuvirtide. In some embodiments, each aliquot comprises 100 mg albuvirtide. In some embodiments, each aliquot comprises 150 mg albuvirtide. In some embodiments, each aliquot comprises 200 mg albuvirtide. In some embodiments, each aliquot comprises 300 mg albuvirtide. In some embodiments, each aliquot comprises 400 mg albuvirtide. In some embodiments, each aliquot comprises 500 mg albuvirtide. In some embodiments, each aliquot contains a single dose of albuvirtide.

In some embodiments, methods provided herein comprise reconstituting the lyophilized pharmaceutical composition with a pharmaceutically acceptable carrier to make pharmaceutical formulations of albuvirtide that can be administered to a subject. In some embodiments, the pharmaceutically acceptable carrier comprises water-for-injection. In some embodiments, the pharmaceutically acceptable carrier comprises saline. In some embodiments, the pharmaceutically acceptable carrier comprises saline and sodium bicarbonate.

### EXAMPLES

The following is a description of various methods and materials used in the studies, and are put forth so as to provide those of ordinary skill in the art with exemplary description of how to make and use the present invention, and are not intended to limit the scope of what the inventors regard as their invention nor are they intended to represent that the experiments below were performed and are all of the experiments that may be performed. Efforts have been made to ensure accuracy with respect to numbers used (e.g., amounts, temperature, etc.), but some experimental errors and deviations should be accounted for.

Unless indicated otherwise, parts are parts by weight, molecular weight is weight average molecular weight, temperature is in degrees Celsius (°C), and pressure is at or near atmospheric. Standard abbreviations are used, including the following: mg = milligram; g = gram; µl or µL = microliter; ml or mL = milliliter; 1 or L = liter; µM = micromolar; mM = millimolar; M = molar; kDa = kilodalton; v/v = Volume/Volume; TFA = trifluoroacetic acid; PB = phosphate buffer; ACN = acetonitrile; HAc = Acetic Acid; HPLC = high performance liquid chromatography; LC-MS = liquid chromatography-mass spectrometry; LYO = lyophilization; ABT = Albuvirtide; N/D = not determined

### Example 1 - Synthesis of Albuvirtide

Albuvirtide was prepared by standard solid-phase synthesis using Rink amide resin and 9-fluorenylmethoxy carbonyl-protected amino acid. The side chain of the 13th lysine residue was protected by allyloxycabonyl (Aloc), which allow selective deprotection and addition of the linker molecule of [2-(2-amino)ethoxyl]ethoxy acetic acid and 3-maleimidopropionic acid.

The cleaved peptide was dissolved in 0.1% TFA in 30% ACN in water (v/v) and purified by reverse-phase HPLC at a purity greater than 90%. The purity and molecular weight were verified by LC-MS.

### Example 2 -Preparation of Albuvirtide Compositions having HAc

The purified peptide in Example 1 in TFA (Albuvirtide-TFA) underwent a salt exchange step which replaced TFA (CF₃CO²⁻) buffer with acetate (CH₃COO⁻) buffer as provided below:
Hardware - Agilent 1100 series HPLC
Mobile phase A - 1% HAc in water (pH 2.6-2.8)
Mobile phase B - HAc in 50% ACN in water (v/v)
Column - Daiso-C18-10µm 30*250mm, 50*250mm
Flow rate - 30 mL/min for 30*250mm column, 50 mL/min for 50*250mm column
Temperature - room temperature
Detection wavelength - 214 nm

The column was firstly washed with 30%B for 30 minutes to achieve salt balance and then eluted with 100%B. The eluent shown major product peak at A₂₁₄ was collected as a liquid composition comprising albuvirtide and HAc (albuvirtide-HAc).

### Example 3 - Stability Test of Compositions having Albuvirtide and Different Buffer systems

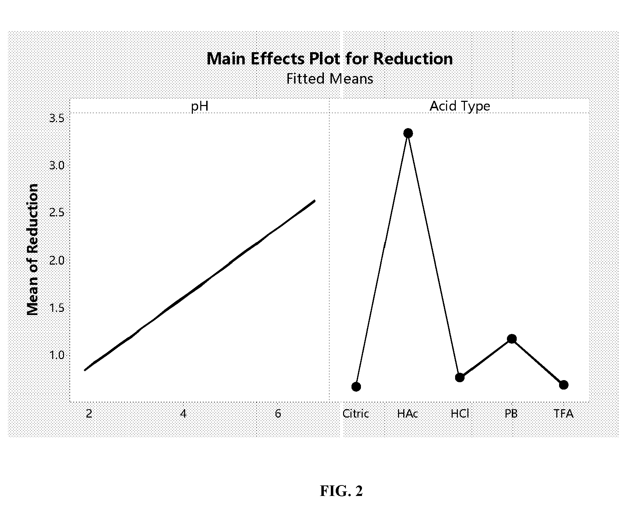

Liquid compositions having albuvirtide in different buffers and combinations of acidic buffers were prepared by replacing TFA in Example 2 with different buffers or buffer combinations. These liquid compositions were then lyophilized and their stability tested using HPLC. Part of the organic solvent could be removed using reduced-pressure evaporation. Results were summarized below and listed in Table 1. The mean reduction in purity in compositions having different pH or buffers is also shown in FIG. 2. As shown, factors including buffer components, ratio and concentration and pH significantly affected the stability. Compositions with different buffers also showed different stability, with citric,TFA, PB and HCl providing better stability than acetic acid.

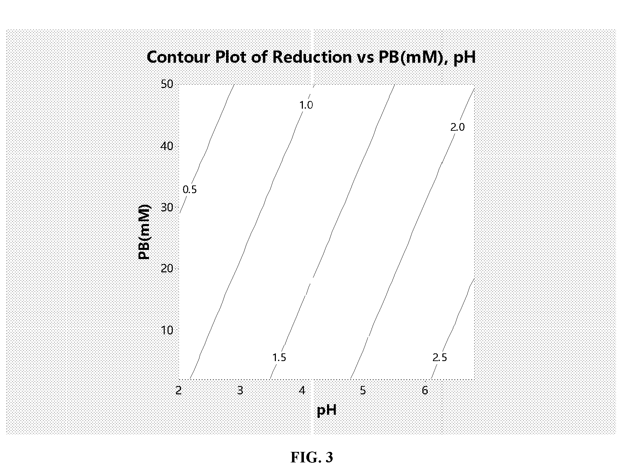

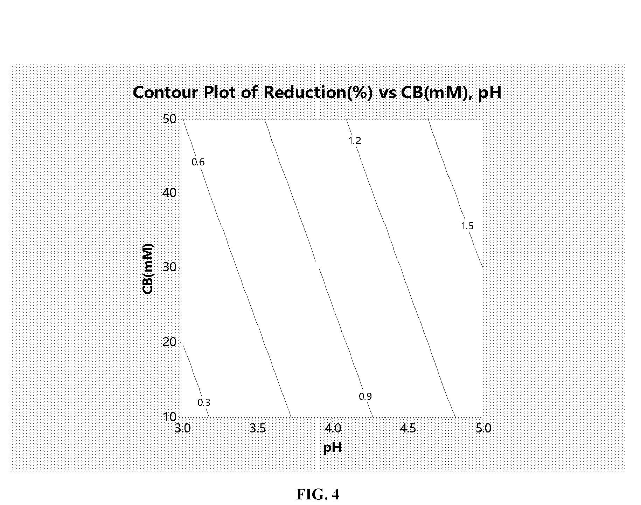

Contour plots of mean reduction in purity of compositions having phosphate buffer, or citrate buffer are also shown in FIG. 3 (phosphate buffer) and FIG. 4 (citrate buffer), respectively.
1. The purity of albuvirtide in 0.1% TFA was maintained at >98.0% during lyophilization; but the lyophilized composition had 9.5% TFA.
2. Inclusion of 10 mM, 20 mM, or 50 mM PB (pH 2.0), 10 mM or 20 PB (pH 2.5), or 20 mM PB (pH 3.0) in the liquid composition of albuvirtide in 0.1% TFA also achieved high stability. The purity of albuvirtide was maintained at >98.0% during lyophilization.
3. Inclusion of 10mM, 50mM Citric/Na (pH 3.0) in the liquid composition of albuvirtide in 0.1% TFA achieved high stability. The purity of albuvirtide was maintained at >98.0% during lyophilization.
4. Inclusion of HCl in the liquid composition of albuvirtide in 0.1% TFA to adjust its pH to 2.0 or 2.5 also achieved high stability. The purity of albuvirtide was maintained at >98.0% during lyophilization.
5. The concentration of albuvirtide affected its stability.

**Table 1: Stability Test of Albuvirtide**

| **Acidic Buffer** | **pH** | **ABT, mg/mL** | **Purity, %** | | |
|---|---|---|---|---|---|
| | | | **Before LYO** | **After LYO** | **Reduction** |
| 0.1% TFA | N/D | 10 | 98.9 | 98.2 | 0.7 |
| 0.1% TFA | N/D | 4 | 98.9 | 98.7 | 0.2 |
| 1% HAc | N/D | 10 | 98.6 | 94.6 | 4.0 |
| 1% HAc | N/D | 4 | 98.6 | 95.0 | 3.6 |
| 1% HAc + 2mM PB | 2.0 | 4 | 98.6 | 96.8 | 1.8 |
| 1% HAc + 10mM PB | 2.0 | 4 | 98.6 | 98.4 | 0.2 |
| 1% HAc + | 2.0 | 4 | 98.6 | 98.5 | 0.1 |
| 20mM PB | | | | | |
| 1% HAc + 50mM PB | 2.0 | 4 | 98.6 | 98.3 | 0.3 |
| 1% HAc + 2mM PB | 2.5 | 4 | 98.6 | 96.6 | 2.0 |
| 1% HAc + 10mM PB | 2.5 | 4 | 98.6 | 98.2 | 0.4 |
| 1% HAc + 20mM PB | 2.5 | 4 | 98.6 | 98.4 | 0.2 |
| 1% HAc + 2mM PB | 3.0 | 4 | 98.6 | 96.3 | 2.3 |
| 1% HAc + 10mM PB | 3.0 | 4 | 98.6 | 97.2 | 1.4 |
| 1% HAc + 20mM PB | 3.0 | 4 | 98.6 | 98.0 | 0.6 |
| 1% HAc + 10mM PB | 5.5 | 4 | 98.6 | 97.0 | 1.6 |
| 1% HAc + 10mM PB | 6.8 | 4 | 98.6 | 96.5 | 2.1 |
| 1% HAc + 50mM PB | 6.8 | 4 | 98.6 | 95.8 | 2.8 |
| 1% HAc + 10mM Citric/Na | 3.0 | 4 | 98.6 | 98.0 | 0.6 |
| 1% HAc + 50mM Citric/Na | 3.0 | 4 | 98.6 | 98.4 | 0.2 |
| 1% HAc + 10mM Citric/Na | 5.0 | 4 | 98.6 | 97.7 | 0.9 |
| 1% HAc + 50mM Citric/Na | 5.0 | 4 | 98.6 | 96.5 | 2.1 |
| 1% HAc + | 2.0 | 4 | 98.6 | 98.3 | 0.3 |
| HCl | | | | | |
| 1% HAc + HCl | 2.5 | 4 | 98.6 | 98.1 | 0.5 |
| 1% HAc + 1% HAc | N/D | 4 | 98.6 | 95.5 | 3.1 |
| 0.5% HAc | N/D | 4 | 98.6 | 95.8 | 2.8 |

### Example 4 - Preparation of Albuvirtide-PB

The liquid composition having albuvirtide in Example 1, in which TFA was the acidic buffer (Albuvirtide-TFA), went through a salt exchange step by eluting with a phosphate buffer (PB, H₃PO₄:NaH₂PO₄ = 100:0 - 5:95). The salt exchange step was performed by HPLC as provided below:
Hardware - Agilent 1100 series HPLC
Mobile phase A - PB in water
Mobile phase B - PB in 50% ACN in water (v/v)
Column - Daiso-C18-10µm 30*250mm, 50*250mm
Flow rate - 30 mL/min for 30*250mm column, 50 mL/min for 50*250mm column
Temperature - room temperature
Detection wavelength - 214 nM

The eluent was firstly washed with 30%B for 30 minutes to achieve salt balance and then eluted with 100%B. The eluent shown major product peak at A₂₁₄ was collected as a liquid composition comprising albuvirtide and PB (Albuvirtide-PB).

### Example 5 - Stability of Albuvirtide-PB

The liquid compositions having albuvirtide and PB were then lyophilized and their stability were tested. Part of the organic solvent could be removed using reduced-pressure evaporation. Stability of Albuvirtide-PB upon lyophilization, upon storage at 25 °C, 6 °C and -20 °C were tested using HPLC. The lyophilized powder of Albuvirtide-PB were kept at 25 °C, 6 °C and -20 °C for certain periods of time while their purities were monitored during such periods by HPLC. The results are shown in Table 2.

**Table 2: Stability of albuvirtide-PB during lyophilization ("LYO")**

| **PB, mM** | **ABT, mg/mL** | **pH** | **Purity, %** | | **Isomer 1, %** | |
|---|---|---|---|---|---|---|
| | | | **Before LYO** | **After LYO** | **Before LYO** | **After LYO** |
| 20 | 5 | 2.7 | 98.64 | 98.48 | 0.32 | 0.45 |
| 10 | 5 | 2.6 | 98.64 | 98.60 | 0.32 | 0.41 |
| 10 | 5 | 3.2 | 98.64 | 98.68 | 0.32 | 0.3 |
| 10 | 23 | 3.7 | 99.38 | 99.00 | 0.45 | 0.50 |
| 10 | 14 | 3.4 | 99.09 | 98.88 | 0.57 | 0.52 |
| 5 | 11.5 | 3.7 | 99.38 | 98.88 | 0.45 | 0.49 |

| **PB, mM** | **ABT, mg/mL** | **pH** | **Isomer 2, %** | | **Dimer 1, %** | |
|---|---|---|---|---|---|---|
| | | | **Before LYO** | **After LYO** | **Before LYO** | **After LYO** |
| 20 | 5 | 2.7 | 1.04 | 1.07 | N/D | N/D |
| 10 | 5 | 2.6 | 1.04 | 0.99 | N/D | N/D |
| 10 | 5 | 3.2 | 1.04 | 1.02 | N/D | N/D |
| 10 | 23 | 3.7 | 0.17 | 0.21 | 0 | 0.29 |
| 10 | 14 | 3.4 | 0.34 | 0.29 | 0 | 0.23 |
| 5 | 11.5 | 3.7 | 0.17 | 0.24 | 0 | 0.26 |

| **PB, mM** | **ABT, mg/mL** | **pH** | **Unknown impurity, %** | | **Total Impurities, %** | |
|---|---|---|---|---|---|---|
| | | | **Before LYO** | **After LYO** | **Before LYO** | **After LYO** |
| 20 | 5 | 2.7 | N/D | N/D | 1.36 | 1.52 |
| 10 | 5 | 2.6 | N/D | N/D | 1.36 | 1.4 |
| 10 | 5 | 3.2 | N/D | N/D | 1.36 | 1.32 |
| 10 | 23 | 3.7 | 0 | 0 | 0.62 | 1.00 |
| 10 | 14 | 3.4 | 0 | 0.08 | 0.91 | 1.12 |
| 5 | 11.5 | 3.7 | 0 | 0.13 | 0.62 | 1.12 |

As shown, the albuvirtide in the liquid compositions of albuvirtide-PB was stable (barely any change in purity or dimer formation) during lyophilization.

**Table 3: Stability of albuvirtide when stored at 25 °C (5 mg/mL Albuvirtide)**

| **Acidic Buffer** | **pH** | **Purity at day, %** | | | | **Isomer 1 at day, %** | | | |
|---|---|---|---|---|---|---|---|---|---|
| | | **0** | **4** | **8** | **30** | **0** | **4** | **8** | **30** |
| 10mM PB | 2.6 | 98.60 | 95.03 | 92.90 | 69.12 | 0.41 | 0.74 | 0.70 | 3.01 |
| 10mM PB | 3.2 | 98.68 | 98.61 | 97.81 | 91.23 | 0.30 | 0.20 | 0.40 | 1.39 |
| HAc | N/D | 94.79 | 81.66 | 71.61 | 49.88 | 0.91 | 1.34 | 2.08 | 3.01 |
| 0.1% TFA | N/D | 98.63 | 98.57 | 97.70 | 92.98 | 0.43 | 0.49 | 0.84 | 1.69 |

| **Acidic Buffer** | **pH** | **Isomer 2 at day, %** | | | | **Dimer 1 at day, %** | | | |
|---|---|---|---|---|---|---|---|---|---|
| | | **0** | **4** | **8** | **30** | **0** | **4** | **8** | **30** |
| 10mM PB | 2.6 | 0.99 | 4.23 | 4.28 | 29.29 | 0 | 0 | 2.12 | 1.58 |
| 10mM PB | 3.2 | 1.02 | 1.19 | 1.57 | 5.01 | 0 | 0 | 0.22 | 1.58 |
| HAc | N/D | 0.94 | 0.58 | 0.94 | 1.45 | 2.1 | 10.64 | 15.82 | 23.84 |
| 0.1% TFA | N/D | 0.94 | 0.94 | 1.46 | 2.85 | 0 | 0 | 0 | 1.22 |

| **Acidic Buffer** | **pH** | **Dimer 2 at day, %** | | | | **Total Impurities at day, %** | | | |
|---|---|---|---|---|---|---|---|---|---|
| | | **0** | **4** | **8** | **30** | **0** | **4** | **8** | **30** |
| 10mM PB | 2.6 | 0 | 0 | 0 | 0 | 1.40 | 4.97 | 7.10 | 30.88 |
| 10mM PB | 3.2 | 0 | 0 | 0 | 0.79 | 1.32 | 1.39 | 2.19 | 8.77 |
| HAc | N/D | 1.26 | 5.78 | 9.55 | 21.82 | 5.21 | 18.34 | 28.39 | 50.12 |
| 0.1% TFA | N/D | 0 | 0 | 0 | 1.26 | 1.37 | 1.43 | 2.30 | 7.02 |

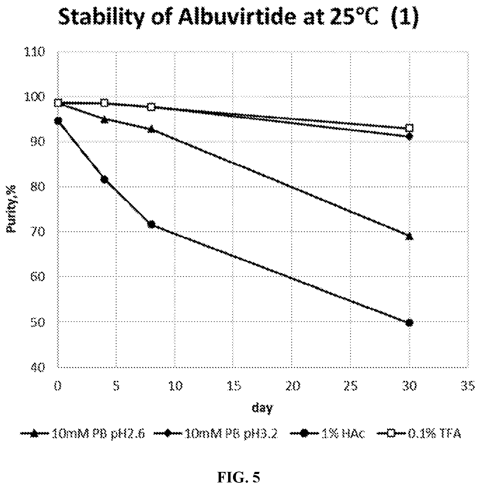

As shown in Table 3 and FIG. 5, the stability of albuvirtide at 25 °C in lyophilized compositions prepared from Albuvirtide-PB or Albuvirtide-TFA was significantly better than in those prepared from Albuvirtide-HAc.

**Table 4: Stability of albuvirtide-PB when stored at 25 °C**

| **PB** | **pH** | **ABT, mg/mL** | **Purity at day, %** | | | | **Isomer 1 at day, %** | | | |
|---|---|---|---|---|---|---|---|---|---|---|
| | | | **0** | **5** | **11** | **30** | **0** | **5** | **11** | **30** |
| 10mM | 3.35 | 5 | 99.00 | 98.45 | 97.17 | 91.66 | 0.71 | 0.92 | 1.00 | 1.54 |
| 10mM | 3.56 | 10 | 98.54 | 97.36 | 93.28 | 89.63 | 0.75 | 0.91 | 1.03 | 1.10 |
| 10mM | 3.71 | 15 | 98.00 | 95.68 | 93.82 | 87.70 | 0.74 | 0.89 | 0.98 | 1.23 |
| 20mM | 3.30 | 15 | 98.73 | 98.06 | 97.52 | 88.98 | 0.60 | 0.90 | 0.82 | 0.87 |
| 10mM | 3.80 | 20 | 97.98 | 94.59 | 91.06 | 79.44 | 0.63 | 1.04 | 1.14 | 1.47 |
| 20mM | 3.35 | 20 | 98.66 | 97.62 | 96.97 | 90.85 | 0.58 | 0.88 | 0.76 | 0.85 |

| **PB** | **pH** | **ABT, mg/mL** | **Isomer 2 at day, %** | | | | **Dimer 1 at day, %** | | | |
|---|---|---|---|---|---|---|---|---|---|---|
| | | | **0** | **5** | **11** | **30** | **0** | **5** | **11** | **30** |
| 10mM | 3.35 | 5 | 0.19 | 0.37 | 0.51 | 3.99 | 0.10 | 0.07 | 0.28 | 0.80 |
| 10mM | 3.56 | 10 | 0.24 | 0.35 | 0.63 | 3.29 | 0.11 | 0.38 | 1.17 | 1.58 |
| 10mM | 3.71 | 15 | 0.26 | 0.28 | 0.46 | 2.26 | 0.18 | 0.82 | 1.14 | 2.11 |
| 20mM | 3.30 | 15 | 0.25 | 0.40 | 0.60 | 7.06 | 0.06 | 0.16 | 0.22 | 0.61 |
| 10mM | 3.80 | 20 | 0.26 | 0.27 | 0.53 | 3.19 | 0.25 | 1.09 | 1.77 | 3.60 |
| 20mM | 3.35 | 20 | 0.26 | 0.45 | 0.49 | 4.47 | 0.08 | 0.28 | 0.42 | 1.18 |

| **PB** | **pH** | **ABT, mg/mL** | **Dimer 2 at day, %** | | | | **Dimer 3 at day, %** | | | |
|---|---|---|---|---|---|---|---|---|---|---|
| | | | **0** | **5** | **11** | **30** | **0** | **5** | **11** | **30** |
| 10mM | 3.35 | 5 | 0 | 0.19 | 0.67 | 1.65 | 0 | 0 | 0.10 | 0.23 |
| 10mM | 3.56 | 10 | 0.27 | 0.80 | 2.39 | 3.25 | 0.09 | 0.20 | 0.70 | 0.62 |
| 10mM | 3.71 | 15 | 0.39 | 1.61 | 2.22 | 3.85 | 0.43 | 0.53 | 0.80 | 1.24 |
| 20mM | 3.30 | 15 | 0.18 | 0.41 | 0.56 | 1.49 | 0.05 | 0.07 | 0.10 | 0.17 |
| 10mM | 3.80 | 20 | 0.51 | 2.04 | 3.45 | 6.53 | 0.25 | 0.77 | 1.14 | 2.26 |
| 20mM | 3.35 | 20 | 0.20 | 0.65 | 0.98 | 1.98 | 0.10 | 0.12 | 0.21 | 0.36 |

| **PB** | **pH** | **ABT, mg/mL** | **Total Impurities at day, %** | | | | | | | |
|---|---|---|---|---|---|---|---|---|---|---|
| | | | **0** | **5** | **11** | **30** | | | | |
| 10mM | 3.35 | 5 | 1.00 | 1.55 | 2.83 | 8.34 | | | | |
| 10mM | 3.56 | 10 | 1.46 | 2.64 | 6.33 | 10.37 | | | | |
| 10mM | 3.71 | 15 | 2.00 | 4.32 | 5.90 | 12.30 | | | | |
| 20mM | 3.30 | 15 | 1.27 | 1.94 | 2.30 | 11.02 | | | | |
| 10mM | 3.80 | 20 | 2.02 | 5.41 | 8.56 | 20.56 | | | | |
| 20mM | 3.35 | 20 | 1.34 | 2.38 | 2.86 | 9.15 | | | | |

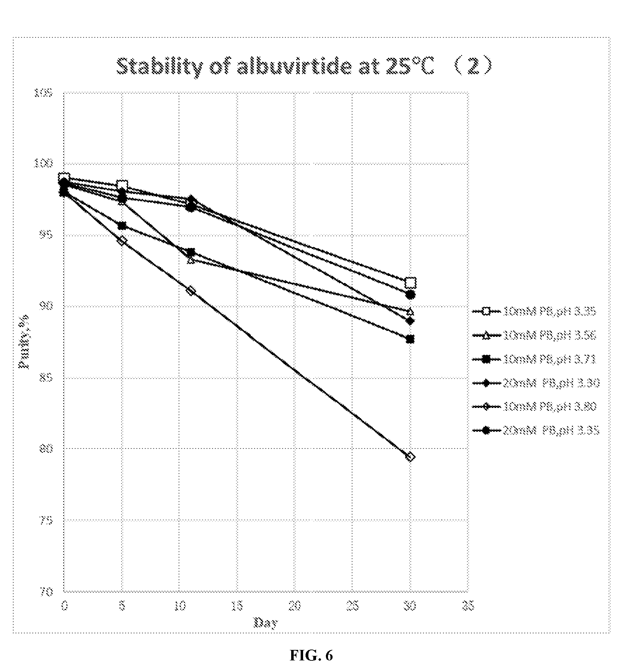

As shown in Table 4 and FIG. 6, high stability of albuvirtide at 25 °C was achieved in lyophilized compositions prepared from Albuvirtide-PB, even when albuvirtide was present at high concentrations (10 mM or 20 mM). Less than 10% total impurities were identified in each tested composition at day 11.

**Table 5: Stability of albuvirtide-PB when stored at 6 °C**

| **PB** | **pH** | **ABT, mg/mL** | **Purity, %** | | **Isomer 1, %** | |
|---|---|---|---|---|---|---|
| | | | **0 day** | **1 month** | **0 day** | **1 month** |
| 10mM | 3.35 | 5 | 99.00 | 98.01 | 0.71 | 0.81 |
| 10mM | 3.56 | 10 | 98.54 | 97.45 | 0.75 | 0.83 |
| 10mM | 3.71 | 15 | 98.00 | 96.67 | 0.74 | 0.91 |
| 20mM | 3.30 | 15 | 98.73 | 97.22 | 0.60 | 0.84 |
| 10mM | 3.80 | 20 | 97.98 | 94.82 | 0.63 | 1.04 |
| 20mM | 3.35 | 20 | 98.66 | 97.76 | 0.58 | 0.85 |

| **PB** | **pH** | **ABT, mg/mL** | **Isomer 2 at day, %** | | **Dimer 1 at day, %** | |
|---|---|---|---|---|---|---|
| | | | **0 day** | **1 month** | **0 day** | **1 month** |
| 10mM | 3.35 | 5 | 0.19 | 0.77 | 0.10 | 0.06 |
| 10mM | 3.56 | 10 | 0.24 | 0.66 | 0.11 | 0.24 |
| 10mM | 3.71 | 15 | 0.26 | 0.71 | 0.18 | 0.47 |
| 20mM | 3.30 | 15 | 0.25 | 0.83 | 0.06 | 0.18 |
| 10mM | 3.80 | 20 | 0.26 | 0.78 | 0.25 | 0.77 |
| 20mM | 3.35 | 20 | 0.26 | 0.66 | 0.08 | 0.18 |

| **PB** | **pH** | **ABT, mg/mL** | **Dimer 2 at day, %** | | **Total Impurities at day, %** | |
|---|---|---|---|---|---|---|
| | | | **0 day** | **1 month** | **0 day** | **1 month** |
| 10mM | 3.35 | 5 | 0 | 0.20 | 1.00 | 1.99 |
| 10mM | 3.56 | 10 | 0.27 | 0.54 | 1.46 | 2.55 |
| 10mM | 3.71 | 15 | 0.39 | 0.82 | 2.00 | 3.33 |
| 20mM | 3.30 | 15 | 0.18 | 0.29 | 1.27 | 2.78 |
| 10mM | 3.80 | 20 | 0.51 | 1.33 | 2.02 | 5.18 |
| 20mM | 3.35 | 20 | 0.20 | 0.43 | 1.34 | 2.24 |

| **PB** | **pH** | **ABT, mg/mL** | **Dimer 3 at day, %** | | | |
|---|---|---|---|---|---|---|
| | | | **0 day** | **1 month** | | |
| 10mM | 3.35 | 5 | 0 | 0 | | |
| 10mM | 3.56 | 10 | 0.09 | 0.12 | | |
| 10mM | 3.71 | 15 | 0.43 | 0.26 | | |
| 20mM | 3.30 | 15 | 0.05 | 0.04 | | |
| 10mM | 3.80 | 20 | 0.25 | 0.47 | | |
| 20mM | 3.35 | 20 | 0.10 | 0.09 | | |

As shown, the change in purity of albuvirtide during storage for 1 month at 6 °C was equivalent to that during storage at 25 °C for 5 days.

**Table 6: Stability of albuvirtide-PB when stored at -20 °C**

| **PB** | **pH** | **ABT, mg/mL** | **Purity, %** | | |
|---|---|---|---|---|---|
| | | | **0** | **3 month** | **Reduction** |
| 10mM | 2.6 | 2.5 | 98.60 | 97.80 | 0.80 |
| 10mM | 3.2 | 5 | 98.68 | 98.02 | 0.66 |
| 10mM | 3.7 | 23 | 99.00 | 98.54 | 0.46 |

As shown, the purity of albuvirtide was barely changed during storage for 3 month at -20 °C.

**Table 7: Stability of albuvirtide-HAc and albuvirtide-PB**

| **Acidic Buffer** | **pH** | **ABT, mg/mL** | **Purity, %** | | |
|---|---|---|---|---|---|
| | | | **Before LYO** | **After LYO** | **Reduction** |
| HAc | NA | NA | 98.8 | 94.4 | 4.4 |
| HAc | NA | NA | 98.8 | 95.0 | 3.8 |
| HAc | NA | NA | 97.3 | 94.2 | 3.1 |
| HAc | 3.07 | 9.89 | 98.64 | 94.79 | 3.85 |
| HAc | NA | NA | 98.6 | 93.8 | 4.8 |
| HAc | NA | NA | 98.5 | 94.3 | 4.2 |
| HAc | NA | NA | 98.6 | 94.7 | 3.9 |
| 10mM PB | 3.2 | 5 | 98.64 | 98.68 | -0.04 |
| 10mM PB | 3.7 | 23 | 99.38 | 99.00 | 0.38 |
| 10mM PB | 3.35 | 5 | 99.04 | 99.00 | 0.04 |
| 10mM PB | 3.56 | 10 | 99.22 | 98.54 | 0.68 |
| 20mM PB | 3.30 | 15 | 99.22 | 98.73 | 0.49 |
| 10mM PB | 3.35 | 20 | 99.22 | 98.66 | 0.56 |

As shown, the stability of albuvirtide was significantly better in lyophilized compositions of Albuvirtide-PB than that in lyophilized compositions of Albuvirtide-HAc.

**Table 8: Stability of albuvirtide-PB**

| **ABT, mg/mL** | **pH** | **PB, mM** | **Purity, %** | | |
|---|---|---|---|---|---|
| | | | **0 Day** | **10 Day** | **reduced** |
| 5 | 3.2 | 10 | 99.00 | 97.17 | 1.83 |
| 5 | 3.06 | 20 | 95.56 | 81.20 | 14.36 |
| 5 | 2.92 | 30 | 94.61 | 64.2 | 30.41 |
| 5 | 2.86 | 50 | 95.99 | 56.50 | 39.49 |
| 7 | N/D | 10 | 99.00 | 97.48 | 1.52 |
| 7 | 3.03 | 20 | 95.58 | 85.00 | 10.58 |
| 7 | 2.99 | 30 | 95.21 | 64.00 | 31.21 |
| 10 | 3.56 | 10 | 98.54 | 93.28 | 5.26 |
| 10 | 3.2 | 20 | 97.57 | 94.19 | 3.38 |
| 10 | 3.00 | 30 | 96.37 | 86.6 | 9.77 |
| 10 | 2.89 | 50 | 96.17 | 79.10 | 17.07 |
| 12 | 3.33 | 10 | 96.51 | 95.10 | 1.41 |
| 12 | 3.10 | 20 | 96.92 | 90.90 | 6.02 |
| 12 | 3.02 | 30 | 96.68 | 86.30 | 10.38 |
| 12 | 2.90 | 50 | 96.43 | 80.10 | 16.33 |
| 15 | 3.71 | 10 | 98.00 | 93.82 | 4.18 |
| 15 | 3.30 | 20 | 98.73 | 97.52 | 1.21 |
| 15 | 3.00 | 30 | 96.78 | 92.40 | 4.38 |
| 20 | 3.7 | 10 | 99.00 | 94.52 | 4.48 |
| 20 | 3.35 | 20 | 98.66 | 96.97 | 1.69 |
| 20 | 3.02 | 30 | 96.66 | 95.80 | 0.86 |
| 20 | 2.92 | 50 | 96.70 | 92.40 | 4.30 |

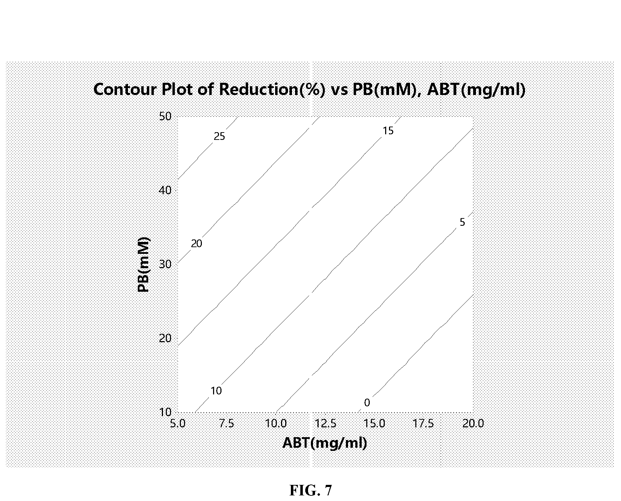

Stability of lyophilized compositions of Albuvirtide-PB having different pH, different concentrations of albuvirtide or phosphate buffer, and different compositions of phosphate buffer at 25 °C is shown above. Contour plots of mean reduction in purity of compositions having albuvirtide and phosphate buffer at various concentrations are also shown in FIG. 7.

As shown in Table 9 below, the purity of albuvirtide was barely changed during storage for 5 days at 25 °C.

**Table 9: Stability of albuvirtide-PB when stored at 25 °C**

| **PB** | **pH** | **ABT, mg/mL** | **Total Impurities, %** | |
|---|---|---|---|---|
| | | | **Day 0** | **Day 5** |
| 20mM | 2.7 | 19.4 | 4.1 | 4.4 |

As shown in Table 10 below, the purity of albuvirtide was barely changed during storage for 2 months at 6°C.

**Table 10: Stability of albuvirtide-PB when stored at 6 °C**

| **PB** | **pH** | **ABT, mg/mL** | **Total Impurities, %** | |
|---|---|---|---|---|
| | | | **Day 0** | **2 month** |
| 20mM | 2.6 | 20.0 | 3.9 | 4.4 |
| 20mM | 2.7 | 18.9 | 5.1 | 5.3 |

As shown in Table 11 below, the purity of albuvirtide was barely changed during storage for 1 month at 25°C.

**Table 11: Stability of albuvirtide-PB when stored at 25 °C**

| **PB** | **pH** | **ABT, mg/mL** | **Total Impurities, %** | |
|---|---|---|---|---|
| | | | **Day 0** | **1 month** |
| 20mM | 2.6 | 20.0 | 3.9 | 8.5 |
| 20mM | 2.7 | 18.9 | 5.1 | 9.5 |

### Example 6 - Preparation of Albuvirtide Compositions having HCl

The liquid composition having albuvirtide in Example 1, in which TFA was the acidic buffer (Albuvirtide-TFA), went through a salt exchange step by eluting with an acid buffer having hydrochloric acid (HCl). The salt exchange step was performed by HPLC as described above.

### Example 7- Stability of Albuvirtide-HCl

The liquid compositions having albuvirtide and HCl were then lyophilized and their stability were tested. Part of the organic solvent could be removed using reduced-pressure evaporation. Stabilities of Albuvirtide-HCl upon lyophilization, upon storage at 25 °C and 6 °C were measured using HPLC. The lyophilized powder of Albuvirtide-HCl were kept at 25 °C and 6 °C for certain periods of time while their purities were monitored during the above period by HPLC. The results are shown in Table 12.

**Table 12: Stability of Albuvirtide-HCl during lyophilization**

| **HCl, mM** | **pH** | **ABT, mg/mL** | **Purity, %** | | **Isomer 1, %** | |
|---|---|---|---|---|---|---|
| | | | **Before LYO** | **After LYO** | **Before LYO** | **After LYO** |
| 5 | 3.05 | 12 | 99.13 | 98.59 | 0.41 | 0.34 |
| 5 | 3.05 | 12 | 98.53 | 98.66 | 0.38 | 0.35 |
| 5 | 2.30 | 9.3 | 98.94 | 98.42 | 0.23 | 0.21 |
| 5 | 2.22 | 6.4 | 99.03 | 98.97 | 0.19 | 0.17 |

| **HCl, mM** | **pH** | **ABT, mg/mL** | **Isomer 2, %** | | **Dimer 1, %** | |
|---|---|---|---|---|---|---|
| | | | **Before LYO** | **After LYO** | **Before LYO** | **After LYO** |
| 5 | 3.05 | 12 | 0.20 | 0.31 | 0 | 0 |
| 5 | 3.05 | 12 | 0.30 | 0.23 | 0 | 0 |
| 5 | 2.30 | 9.3 | 0.18 | 0.20 | 0 | 0 |
| 5 | 2.22 | 6.4 | 0.24 | 0.18 | 0 | 0 |

| **HCl, mM** | **pH** | **ABT, mg/mL** | **Dimer 2, %** | | **Dimer 3, %** | |
|---|---|---|---|---|---|---|
| | | | **Before LYO** | **After LYO** | **Before LYO** | **After LYO** |
| 5 | 3.05 | 12 | 0 | 0 | 0 | 0 |
| 5 | 3.05 | 12 | 0 | 0 | 0 | 0 |
| 5 | 2.30 | 9.3 | 0 | 0 | 0.04 | 0 |
| 5 | 2.22 | 6.4 | 0 | 0 | 0.03 | 0 |

| **HCl, mM** | **pH** | **ABT, mg/mL** | **Total Impurities, %** | | | |
|---|---|---|---|---|---|---|
| | | | **Before LYO** | **Before LYO** | | |
| 5 | 3.05 | 12 | 0.87 | 1.40 | | |
| 5 | 3.05 | 12 | 1.47 | 1.34 | | |
| 5 | 2.30 | 9.3 | 1.06 | 1.58 | | |
| 5 | 2.22 | 6.4 | 0.97 | 1.03 | | |

As shown, albuvirtide was highly stable in lyophilized compositions of Albuvirtide-HCl.

**Table 13: Stability of albuvirtide-HCl when stored at 25 °C (5mM HCl, pH=3.05, 12 mg/mL Albuvirtide)**

| | **Purity, %** | **Isomer 1, %** | **Isomer 2, %** | **Dimer 1, %** | **Dimer 2, %** | **Dimer 3, %** | **Total Impurities, %** |
|---|---|---|---|---|---|---|---|
| **0 day** | 98.59 | 0.34 | 0.31 | 0 | 0 | 0 | 1.40 |
| **5 day** | 98.21 | 0.40 | 0.47 | 0 | 0.04 | 0 | 1.79 |
| **11 day** | 98.16 | 0 | 0.73 | 0 | 0 | 0 | 1.84 |
| **30 day** | 93.61 | 0.65 | 1.26 | 0.83 | 1.51 | 0.39 | 6.39 |

**Table 14: Stability of albuvirtide-HCl when stored at 6 °C (5mM HCl, pH=3.05, 12 mg/mL Albuvirtide)**

| | **Purity, %** | **Isomer 1, %** | **Isomer 2, %** | **Dimer 1, %** | **Dimer 2, %** | **Dimer 3, %** | **Total Impurities, %** |
|---|---|---|---|---|---|---|---|
| **0 day** | 98.59 | 0.34 | 0.31 | 0 | 0 | 0 | 1.40 |
| **30 day** | 97.82 | 0.45 | 0.76 | 0.03 | 0.08 | 0.02 | 2.18 |

As shown, albuvirtide was highly stable in lyophilized composition of Albuvirtide-HCl as well.

### Example 8- Effect of pH on the Albuvirtide Stability

The effect of pH in the pre-lyophilized liquid compositions on stability of albuvirtide was measured by monitoring the purity of albuvirtide in lyophilized compositions during storage. Results are summarized below in Table 15.

**Table 15: Effect of pH on albuvirtide stability**

| **Acidic Buffer** | **pH** | **ABT, mg/mL** | **Purity, %** | | | | **Solubility** |
|---|---|---|---|---|---|---|---|
| | | | **Before LYO** | **After LYO (0 Day)** | **10 Day At 25 °C** | **30 Day At 25 °C** | |
| TFA | 2.0 | 10.1 | 98.90 | 98.63 | 97.70 | 92.98 | partial |
| TFA | 2.0 | 5.9 | 98.74 | 98.36 | / | / | partial |
| HAc | 3.07 | 9.89 | 98.60 | 94.60 | 71.61 | 49.88 | cloudy |
| HCl | 3.05 | 12 | 98.53 | 98.59 | 98.16 | 93.61 | partial |
| HCl | 2.30 | 9.3 | 98.94 | 98.42 | / | / | complete |
| HCl | 2.22 | 6.4 | 99.03 | 98.97 | / | / | complete |
| PB | 2.6 | 2.5 | 98.64 | 98.60 | 92.90 | 69.12 | complete |
| PB | 3.2 | 5 | 98.64 | 98.68 | 97.81 | 91.23 | complete |
| PB | 3.7 | 23 | 99.38 | 99.00 | 94.52 | 90.43 | slightly cloudy |
| PB | N/D | 7.16 | 99.80 | 99.00 | 97.48 | 88.80 | complete |
| PB | 3.2 | 11.74 | 96.54 | 97.57 | 94.19 | 73.4 | complete |
| PB | 3.35 | 5.0 | 99.22 | 99.00 | 97.17 | 91.66 | / |
| PB | 3.56 | 10 | 99.22 | 98.54 | 93.28 | 89.63 | complete |
| PB | 3.71 | 15 | 99.22 | 98.00 | 93.82 | 87.70 | slightly cloudy |
| PB | 3.30 | 15 | 99.22 | 98.73 | 97.52 | 88.98 | complete |
| PB | 3.80 | 20.5 | 99.22 | 97.98 | 91.06 | 79.44 | cloudy |
| PB | 3.35 | 20 | 99.22 | 98.66 | 96.97 | 90.95 | complete |

As shown, liquid compositions of albuvirtide having a suitable buffer and pH between 2.0-3.7 were most stable during lyophilization and storage of the lyophilized compositions.

## Claims

1. A liquid composition comprising albuvirtide at a concentration between 5 mg/ml and 200 mg/ml and an acidic buffer at a concentration between 5 and 200 mM, wherein the composition has a pH between 1.0 and 3.5.

2. The liquid composition of claim 1, further comprising water and an organic solvent, wherein the organic solvent is acetonitrile, methanol, ethanol, isopropanol, or any combination thereof, and wherein the organic solvent and water are at a volume ratio between 5:95 and 95:5.

3. The liquid composition of claim 1 or claim 2, wherein the acidic buffer comprises an acid selected from the group consisting of phosphoric acid, hydrochloric acid, acetic acid, citric acid, formic acid, trifluoroacetic acid (TFA) , sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid; and a salt form of the acid.

4. The liquid composition of claim 3, wherein the acid is phosphoric acid and the salt form of the acid comprises a sodium salt of the acid, a potassium salt of the acid, an ammonium salt of the acid, or any combination thereof

5. The liquid composition of claim 3 or claim 4, wherein the acidic buffer comprises the acid and the salt form of the acid at a molar ratio between 100:0 to 5:95, and wherein the salt form of phosphoric acid is sodium phosphate, or sodium dihydrogen phosphate.

6. A stable lyophilized composition of albuvirtide prepared by lyophilizing the liquid composition of any one of claims 1 to 5.

7. A method of preparing a stable lyophilized composition of albuvirtide from a first composition comprising (a) albuvirtide and (b) TFA or formic acid, comprising:
(i) replacing the TFA or formic acid in the first composition with an acidic buffer to make a second composition comprising albuvirtide and the acidic buffer, wherein the acidic buffer comprises an acid selected from the group consisting of phosphoric acid, hydrochloric acid, acetic acid, citric acid, and sulfuric acid, tartaric acid, lactic acid, succinic acid, ascorbic acid, aspartic acid, glutamic acid, propanoic acid, propanedioic acid, butyric acid, maleic acid, fumaric acid, malic acid, and oxalic acid, and
(ii) lyophilizing the second composition to make the stable lyophilized albuvirtide composition, wherein the second composition has a pH between 1.0 and 3.5, and the method further comprises adjusting the albuvirtide concentration in the second composition to be between 5 mg/ml and 200 mg/ml before lyophilization.

## Patentansprüche

1. Flüssige Zusammensetzung, umfassend Albuvirtid in einer Konzentration zwischen 5 mg/ml und 200 mg/ml und einen sauren Puffer in einer Konzentration zwischen 5 und 200 mM, wobei die Zusammensetzung einen pH zwischen 1,0 und 3,5 aufweist.

2. Flüssige Zusammensetzung nach Anspruch 1, ferner umfassend Wasser und ein organisches Lösungsmittel, wobei das organische Lösungsmittel Acetonitril, Methanol, Ethanol, Isopropanol oder eine beliebige Kombination davon ist und wobei das organische Lösungsmittel und Wasser in einem Volumenverhältnis zwischen 5:95 und 95:5 vorliegen.

3. Flüssige Zusammensetzung nach Anspruch 1 oder Anspruch 2, wobei der saure Puffer eine Säure umfasst, die ausgewählt ist aus der Gruppe bestehend aus Phosphorsäure, Salzsäure, Essigsäure, Zitronensäure, Ameisensäure, Trifluoressigsäure (TFA), Schwefelsäure, Weinsäure, Milchsäure, Bernsteinsäure, Ascorbinsäure, Asparaginsäure, Glutaminsäure, Propansäure, Propandisäure, Buttersäure, Maleinsäure, Fumarsäure, Malinsäure und Oxalsäure; und eine Salzform der Säure.

4. Flüssige Zusammensetzung nach Anspruch 3, wobei die Säure Phosphorsäure ist und die Salzform der Säure ein Natriumsalz der Säure, ein Kaliumsalz der Säure, ein Ammoniumsalz der Säure oder eine beliebige Kombination davon umfasst.

5. Flüssige Zusammensetzung nach Anspruch 3 oder Anspruch 4, wobei der saure Puffer die Säure und die Salzform der Säure in einem Molverhältnis zwischen 100:0 bis 5:95 umfasst und wobei es sich bei der Salzform von Phosphorsäure um Natriumphosphat oder Natriumdihydrogenphosphat handelt.

6. Stabile lyophilisierte Zusammensetzung von Albuvirtid, zubereitet durch Lyophilisieren der flüssigen Zusammensetzung nach einem der Ansprüche 1 bis 5.

7. Verfahren zur Zubereitung einer stabilen lyophilisierten Zusammensetzung von Albuvirtid aus einer ersten Zusammensetzung, umfassend (a) Albuvirtid und (b) TFA oder Ameisensäure, umfassend:
(i) Ersetzen der TFA oder Ameisensäure in der ersten Zusammensetzung mit einem sauren Puffer, um eine zweite Zusammensetzung herzustellen, die Albuvirtid und den sauren Puffer umfasst, wobei der saure Puffer eine Säure umfasst, die ausgewählt ist aus der Gruppe bestehend aus Phosphorsäure, Salzsäure, Essigsäure, Zitronensäure und Schwefelsäure, Weinsäure, Milchsäure, Bernsteinsäure, Ascorbinsäure, Asparaginsäure, Glutaminsäure, Propansäure, Propandisäure, Buttersäure, Maleinsäure, Fumarsäure, Malinsäure und Oxalsäure und
(ii) Lyophilisieren der zweiten Zusammensetzung, um die stabile lyophilisierte Albuvirtid-Zusammensetzung herzustellen, wobei die zweite Zusammensetzung einen pH zwischen 1,0 und 3,5 aufweist und das Verfahren ferner das Einstellen der Albuvirtid-Konzentration in der zweiten Zusammensetzung auf zwischen 5 mg/ml und 200 mg/ml vor der Lyophilisierung umfasst.

## Revendications

1. Composition liquide comprenant de l'albuvirtide à une concentration comprise entre 5 mg/ml et 200 mg/ml et un tampon acide à une concentration comprise entre 5 et 200 mM, ladite composition présentant un pH compris entre 1,0 et 3,5.

2. Composition liquide de la revendication 1, comprenant en outre de l'eau et un solvant organique, dans laquelle le solvant organique est l'acétonitrile, le méthanol, l'éthanol, l'isopropanol, ou toute combinaison de ceux-ci, et dans laquelle le solvant organique et l'eau sont dans un rapport volumique compris entre 5:95 et 95:5.

3. Composition liquide de la revendication 1 ou la revendication 2, dans laquelle le tampon acide comprend un acide choisi dans le groupe constitué de l'acide phosphorique, de l'acide chlorhydrique, de l'acide acétique, de l'acide citrique, de l'acide formique, de l'acide trifluoroacétique (TFA), de l'acide sulfurique, de l'acide tartrique, de l'acide lactique, de l'acide succinique, de l'acide ascorbique, de l'acide aspartique, de l'acide glutamique, de l'acide propanoïque, de l'acide propanedioïque, de l'acide butyrique, de l'acide maléique, de l'acide fumarique, de l'acide malique et de l'acide oxalique ; et une forme sel de l'acide.

4. Composition liquide de la revendication 3, dans laquelle l'acide est l'acide phosphorique et la forme sel de l'acide comprend un sel de sodium de l'acide, un sel de potassium de l'acide, un sel d'ammonium de l'acide, ou toute combinaison de ceux-ci.

5. Composition liquide de la revendication 3 ou la revendication 4, dans laquelle le tampon acide comprend l'acide et la forme sel de l'acide dans un rapport molaire compris entre 100:0 et 5:95, et dans laquelle la forme sel de l'acide phosphorique est le phosphate de sodium ou le dihydrogénophosphate de sodium.

6. Composition lyophilisée stable d'albuvirtide préparée par lyophilisation de la composition liquide de l'une quelconque des revendications 1 à 5.

7. Procédé de préparation d'une composition lyophilisée stable d'albuvirtide à partir d'une première composition comprenant (a) de l'albuvirtide et (b) du TFA ou de l'acide formique, comprenant :
(i) le remplacement du TFA ou de l'acide formique dans la première composition par un tampon acide pour préparer une seconde composition comprenant l'albuvirtide et le tampon acide, ledit tampon acide comprenant un acide choisi dans le groupe constitué de l'acide phosphorique, de l'acide chlorhydrique, de l'acide acétique, de l'acide citrique, de l'acide sulfurique, de l'acide tartrique, de l'acide lactique, de l'acide succinique, de l'acide ascorbique, de l'acide aspartique, de l'acide glutamique, de l'acide propanoïque, de l'acide propanedioïque, de l'acide butyrique, de l'acide maléique, de l'acide fumarique, de l'acide malique et de l'acide oxalique, et
(ii) la lyophilisation de la seconde composition pour préparer la composition d'albuvirtide lyophilisée stable, ladite seconde composition présentant un pH compris entre 1,0 et 3,5, et ledit procédé comprenant en outre l'ajustement de la concentration d'albuvirtide dans la seconde composition pour qu'elle soit comprise entre 5 mg/ml et 200 mg/ml avant la lyophilisation.
